# EUROPEAN PATENT SPECIFICATION

(11) **EP 4 525 938 B1**
(45) Date of publication and mention of the grant of the patent: **11.03.2026**
(21) Application number: 23727492.3
(22) Date of filing: 13.05.2023
(51) Int. Cl.: A61K 51/10, A61P 35/00, C07K 16/30, A61K 31/4155, A61K 45/06

(54) **RADIOPHARMACEUTICAL COMPLEXES TARGETING PROSTATE-SPECIFIC MEMBRANE ANTIGEN AND ITS COMBINATIONS**
RADIOPHARMAZEUTISCHE KOMPLEXE ABZIELEND AUF PROSTATA-SPEZIFISCHES ANTIGEN UND DEREN KOMBINATIONEN
COMPLEXES RADIOPHARMACEUTIQUES CIBLÉS À L'ANTIGÈNE PROSTATIQUE ET LEURS COMBINAISONS

(30) Priority: 17.05.2022 EP 22173871; 05.10.2022 EP 22199703; 29.03.2023 EP 23164865
(43) Date of publication of application: 26.03.2025
(73) Proprietor: Bayer Aktiengesellschaft, 51373 Leverkusen (DE); Bayer AS, 0283 Oslo (NO)
(72) Inventor: SCHATZ, Christoph, 13465 Berlin (DE); ZITZMANN-KOLBE, Sabine, 13507 Berlin (DE); LARSEN, Aasmund, 0283 Oslo (NO); RYAN, Olav B., 0283 Oslo (NO); INDREVOLL, Bard, 0283 Oslo (NO); CUTHBERTSON, Alan, 0283 Oslo (NO); BJERKE, Roger Malerbakken, 0283 Oslo (NO); MOEN, Ingrid, 0283 Oslo (NO); HAENDLER, Bernard, 13465 Berlin (DE); HAGEMANN, Urs Beat, 16548 Glienicke/Nordbahn (DE)
(74) Representative: BIP Patents
(86) International application number: PCT/EP2023/062860
(87) International publication number: WO 2023/222557

(56) References cited:
- WO-A2-03/034903
- US-A1- 2020 157 087
- MCDEVITT M R ET AL: "TUMOR THERAPY WITH TARGETED ATOMIC NANOGENERATORS", SCIENCE, AMERICAN ASSOCIATION FOR THE ADVANCEMENT OF SCIENCE, US, vol. 294, 16 November 2001 (2001-11-16), pages 1537 - 1540, XP001088572, ISSN: 0036-8075, DOI: 10.1126/SCIENCE.1064126
- TAGAWA ST: "Phase I study of 225Ac-J591 for men with metastatic castration-resistant prostate cancer (mCRPC). | Journal of Clinical Oncology", ASCO ANNUAL MEETING I, vol. 39 (15 suppl), 20 May 2021 (2021-05-20), XP055972172

## Description

### FIELD OF THE INVENTION

The present invention relates to tissue-targeting compounds. In particular, the present invention relates to tissue-targeting compounds comprising a monoclonal antibody or an antigen-binding fragment thereof having binding affinity for the prostate-specific membrane antigen (PSMA). Further, the present invention relates to combinations, preferably pharmaceutical combinations comprising a tissue-targeting compound of formula (I) and a further pharmaceutical agent. Said tissue-targeting compounds and/or combinations are useful in diagnostics and/or therapy, preferably in therapy, more preferably in treating hyperproliferative diseases such as cancer.

Further, the present invention relates to a process for the preparation of said tissue-targeting compounds and to their use in diagnostics and/or in the treatment of disease, preferably in the treatment of disease, particularly against hyperproliferative diseases, in particular against cancer.

### BACKGROUND OF THE INVENTION

Specific cell killing can be essential for the successful treatment of a variety of diseases in mammalian subjects. Typical examples of this are the treatment of malignant diseases such as sarcomas and carcinomas. However, the selective elimination of certain cell types can also play a key role in the treatment of other diseases, especially hyperplastic and neoplastic diseases.

The most common methods of selective treatment are currently surgery and external beam irradiation. Targeted radionuclide therapy is, however, a promising and developing area with the potential to deliver highly cytotoxic radiation specifically to cell types associated with disease. The most common forms of radiopharmaceuticals currently authorized for use in humans employ beta-emitting and/or gamma-emitting radionuclides. There has, however, been some interest in the use of alpha-emitting radionuclides in therapy because of their potential for more potent cell killing.

The radiation range of typical alpha emitters in physiological surroundings is generally less than 100 micrometers, the equivalent of only a few cell diameters. This makes these sources well suited for the treatment of tumors, including micro-metastases, because they have the range to reach neighboring cells within a tumor but if they are well targeted then little of the radiated energy will pass beyond the target cells. Thus, not every cell need be targeted but damage to surrounding healthy tissue may be minimized (see Feinendegen et al., Radiat. Res. 148:195-201 (1997)). In contrast, a beta particle has a range of 1 mm or more in water (see Wilbur, Antibody Immunocon. Radiopharm. 4: 85-96 (1991)).

The energy of alpha-particle radiation is high in comparison with that carried by beta particles, gamma rays and X-rays, typically being 5-8 MeV, or 5 to 10 times that of a beta particle and 20 or more times the energy of a gamma ray. Thus, this deposition of a large amount of energy over a very short distance gives alpha-radiation an exceptionally high linear energy transfer (LET), high relative biological efficacy (RBE) and low oxygen enhancement ratio (OER) compared to gamma and beta radiation (see Hall, "Radiobiology for the radiologist", Fifth edition, Lippincott Williams & Wilkins, Philadelphia PA, USA, 2000). This explains the exceptional cytotoxicity of alpha emitting radionuclides and also imposes stringent demands on the biological targeting of such isotopes and upon the level of control and study of alpha emitting radionuclide distribution which is necessary in order to avoid unacceptable side effects.

So far, with regards to the application in radioimmunotherapy the main attention has been focused on 211At, 213Bi and 225Ac and these three nuclides have been explored in clinical immunotherapy trials. However, although targeted radiotherapy has been practiced for some time using macrocyclic complexes of radionuclides, the compounds currently in use, see for example Michael R. McDevitt et al, "Tumor Therapy with Targeted Atomic Nanogenerators", Science 2001; 294:1537-1570) or Scott T. Tagawa et al, "Phase I study of 225Ac-J591 for men with metastatic castration-resistant prostate cancer (mCRPC)", J Clin Oncol 2021; 39:5015, which typically employ the macrocyclic chelator DOTA (2,2',2",2‴-(1,4,7,10-Tetraazacyclododecane-1,4,7,10-tetrayl)tetraacetic acid) are lacking in stability, leading to the dissociation of the radionuclide from the chelating macrocycle, which results in a reduced selectivity and activity to the targeted tissue and an increased toxicity to the non-targeted tissue.

For example, it has been reported that a high chelator-to-antigen (CAR) of 10 has been used in a pre-clinical study using a one-step radiolabeling procedure of a DOTA antibody chelator conjugate (ACC) (William F. Maguire, Michael R. McDevitt, Peter M. Smith-Jones, David A. Scheinberg. "Efficient one-step radiolabeling of monoclonal antibodies to high specific activity with Actinium-225 for alpha-particle radioimmunotherapy of cancer", J. Nucl. Med. 2014; 55(9): 1492-1498). However, after 2h labeling at 37 °C, quantitative labeling was not achieved which subsequentially requires a purification step to remove non-labeled Ac-225. This is in contrast to the tissue-targeting compounds of the present invention, in which Ac-225 can be chelated quantitatively at room temperature after one hour leading to complexes with significantly lower CAR ratios (<1).

While the use of larger macrocycles has led to more stable complexation of especially radionuclides of larger size, there is a need for the development of targeting compounds with improved stability, selectivity and efficacy, especially in the field of hyperproliferative diseases such as cancer. For some types of cancer, especially prostate cancer, patients initially respond to therapy but eventually develop resistances (Swami U, McFarland TR, Nussenzveig R, Agarwal N. Advanced Prostate Cancer: Treatment Advances and Future Directions. Trends Cancer 2020;6(8):702-15 doi 10.1016/j.trecan.2020.04.010). Thus, there is a need for new agents capable of selectively targeting specific cells and cell types in malignant diseases which are highly effective and reduce, mitigate and/or avoid the prevalence of resistances. In particular, there is a need to provide new therapeutic agents for the treatment of cancer and especially prostate cancer, which is the most frequent cancer in men (Mattiuzzi C, Lippi G. Current Cancer Epidemiology. J Epidemiol Glob Health 2019;9(4):217-22 doi 10.2991/jegh.k.191008.001). The prostate-specific membrane antigen (PSMA) encoded by the FOLH1 (folate hydrolase 1) gene, is highly expressed in prostate cancer cells, making it a suitable target for radiotherapy.

It has surprisingly been found that the tissue-targeting compounds of formula (I) and the combinations comprising a tissue-targeting compound of formula (I) and a further pharmaceutical agent of the present invention show advantageous properties with regard to their stability, activity, efficacy and selectivity. In particular, the tissue-targeting compounds of formula (I) of the present invention show high values for the immunoreactive fraction (IRF) at low chelator-to-antigen (CAR) ratios and high monomeric purity, thus leading to a higher fraction of radionuclide-labelled compound having affinity for PSMA. Further, the tissue-targeting compounds of formula (I) of the present invention show a favorable biodistribution in vivo and low accumulation in the liver, which is a sign of reduced dissociation of the radionuclide from the chelating moiety and therefore of increased stability. In contrast to previously reported examples in the literature, the tissue-targeting compounds of formula (I) of the present invention can be labelled with the radionuclide under very mild conditions and at reduced temperatures (i.e., room temperature), thereby reducing antibody denaturation and the amount of non-PSMA binding fraction in the final product. Thus, the tissue-targeting compounds of formula (I) of the present invention show a better tumor to liver ratio and can therefore treat disease more effectively and selectively, reducing damage to non-targeted tissue while maintaining a high potency against cancer cells in the targeted tissue. In combination with one or more further pharmaceutical agents, a greater therapeutic efficacy can be achieved than when either compound is used alone.

In the field of radiopharmaceuticals, there is an increased need for the provision of radionuclide-containing compounds and/or combinations comprising radionuclide-containing compounds which are stable over sufficient periods of time that allow their delivery to the administering physician and the patient without suffering from radiopharmaceutical degradation, e.g., by decomplexation of the radionuclide from the chelator. Increasing the stability of the radiopharmaceutical in radionuclide-containing compounds and/or combinations comprising radionuclide-containing compounds is especially desirable from a logistical perspective, since this would allow for the reliable delivery of the radiopharmaceutical-containing drug product across countries and/or continents. It would be desirable to have radionuclide-containing compounds and/or combinations comprising radionuclide-containing compounds available which are stable for at least 48 hours, preferably for 96 hours and/or which have a monomer content of at least 85% for at least 48 hours, preferably a monomer content of at least 90% for at least 48 hours. The present invention solves these issues by providing tissue-targeting compounds of formula (I) and combinations comprising a tissue-targeting compound of formula (I) and a further pharmaceutical agent.

In particular, combinations comprising a tissue-targeting compound of formula (I) and a further pharmaceutical agent of the present invention will serve to:
(1) yield better efficacy in reducing the growth of a tumor or even eliminate the tumor as compared to administration of either agent alone,
(2) provide for the administration of lesser amounts of the administered targeted compounds and/or chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time until tumor progression, and/or
(8) yield efficacy and tolerability results at least as good as those of the agents used alone, compared to known instances where other cancer agent combinations produce inhibitory effects.

### SUMMARY OF THE INVENTION

The present invention relates to tissue-targeting compounds of formula (I) 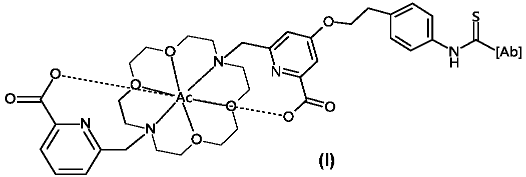 wherein [Ab] is a monoclonal antibody or an antigen-binding fragment thereof having binding affinity for the prostate-specific membrane antigen (PSMA).

### DEFINITIONS

The term "comprising" when used in the specification includes "consisting of".

If within the present text any item is referred to as "as mentioned herein", it means that it may be mentioned anywhere in the present text.

In the context of the present invention, "tissue targeting" is used herein to indicate that the substance in question (i.e. a tissue-targeting compound, a tissue-targeting actinium complex and/or a tissue-targeting moiety, particularly when in the form of a tissue-targeting complex as described herein), serves to localize itself (and particularly to localize any conjugated actinium complex) preferentially to at least one tissue site at which its presence (e.g. to deliver a radioactive decay) is desired. Thus, a tissue-targeting compound, complex, group or moiety serves to provide greater localization to at least one desired site in the body of a subject following administration to that subject in comparison with the concentration of an equivalent complex not having the targeting moiety. The targeting moiety in the present case will be preferably selected to bind specifically to cell-surface receptors associated with cancer cells or other receptors associated with the tumor microenvironment.

In the context of the present invention, the term "PSMA" refers to the prostate-specific membrane antigen encoded by the FOLH1 (folate hydrolase 1) gene (GenelD: 2346).

In the context of the present invention, the terms TLX592 and J592 can be used interchangeably and refer to the same monoclonal antibody, i.e., a monoclonal antibody having binding affinity for the antigen PSMA (anti-PSMA antibody) developed by Telix Pharmaceuticals which has been described in e.g., WO2021/000018.

TLX592 (or J592) comprises a heavy chain sequence according to SEQ ID NO. 19 and a light chain sequence according to SEQ ID NO. 20.

Further, TLX592 (or J592) comprises at least the three CDR heavy chain sequences according to SEQ ID NO. 21, SEQ ID NO. 22 and SEQ ID NO. 23 and the three CDR light chain sequences according to SEQ ID NO. 24, SEQ ID NO. 25 and SEQ ID NO. 26:
TLX592 CDR Heavy Chain Sequences
SEQ ID NO. 21: EYTIH
SEQ ID NO. 22: N INPNNGGTTY NQKFED
SEQ ID NO. 23: GW NFDY
TLX592 CDR Light Chain Sequences
SEQ ID NO. 24: KASQDVG TAVD
SEQ ID NO. 25: W ASTRHT
SEQ ID NO. 26: QQ YNSYPLT

The compounds of formula (I) of the present invention comprise a chelating moiety comprising a complexed actinium atom (Ac). In the context of the present invention, the term "Ac" means an ion of at least one alpha-emitting actinium isotope. Preferably, the alpha-emitting actinium isotope is 225Ac. Preferably, the ion of at the least one alpha-emitting actinium isotope is ²²⁵Ac having a triple positive charge, i.e., ²²⁵Ac³⁺. Thus, in the context of the present invention, the term "Ac" preferably, but not exclusively, means ²²⁵Ac³⁺.

In the compounds of formula (I) of the present invention, the actinium (Ac) atom is shown as being complexed to four oxygen and two nitrogen atoms of the macrocyclic ring as well as to two carboxylate groups. While this is the assumed complexation pattern for the actinium atom, this depiction includes all possible and conceivable cases in which one or more of the bonds is not present, e.g., where the actinium atom is not bound to all heteroatoms of the macrocyclic ring, or to the carboxylate groups, etc.

In the context of the present invention, the term IRF refers to the Immunoreactive Fraction i.e., the fraction of the labelled product (i.e., the compounds of formula (I) of the present invention) which is capable of binding to the target (i.e., PSMA). The Lindmo assay (Lindmo T., et al. (1984) "Determination of the immunoreactive fraction of radiolabeled monoclonal antibodies by linear extrapolation to binding at infinite antigen excess." J. Immunol. Methods. 72, 77-89) is the most commonly used method for assessing the immunoreactive fraction.

In the context of the present invention, the term CAR refers to the Chelator-to-Antigen Ratio, which is a measure of the specific activity of the radiolabeled compound (e.g., the compounds of formula (I) of the present invention).

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. Unless otherwise indicated, a particular polypeptide sequence also implicitly encompasses conservatively modified variants thereof.

Amino acids may be referred to herein by their commonly known three letter symbols or by the one-letter symbols recommended by the IUPAC-IUB Biochemical Nomenclature Commission. Nucleotides, likewise, may be referred to by their commonly accepted single-letter codes.

The term "antibody", as used herein, is intended to refer to immunoglobulin molecules including, but not limited to, full-length antibodies and monovalent antibodies. "Full-length antibodies" are preferably comprised of four polypeptide chains, two heavy (H) chains and two light (L) chains which are typically inter-connected by disulfide bonds. Each heavy chain is comprised of a heavy chain variable region (abbreviated herein as VH) and a heavy chain constant region. The heavy chain constant region can comprise e.g., three domains CH1, CH2 and CH3. Each light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4. "Monovalent antibodies" as used herein are preferably comprised of three polypeptide chains, two heavy (H) chains and one light (L) chain which are typically inter-connected by disulfide bonds. One heavy chain is comprised of a heavy chain variable region (abbreviated herein as VH) and a heavy chain constant region. The heavy chain constant region can comprise e.g., three domains CH1, CH2 and CH3. The other heavy chain is comprised of a heavy chain constant region only. The light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4.

As used herein, the term "Complementarity Determining Regions" (CDRs; e.g., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (e.g. about residues 24-34 (L1), 50-56 (L2) and 89-97 (L3) in the light chain variable domain and 31-35 (H1), 50-65 (H2) and 95-102 (H3) in the heavy chain variable domain; (Kabat et al., Sequences of Proteins of Immunolological Interest, 5th Ed. Public Health Service, National Institutes of Health, Bethesda, MD. (1991)) and/or those residues from a "hypervariable loop" (e.g. about residues 26-32 (L1), 50-52 (L2) and 91-96 (L3) in the light chain variable domain and 26- 32 (H1), 53-55 (H2) and 96-101 (H3) in the heavy chain variable domain (Chothia and Lesk; J Mol Biol 196: 901-917 (1987)). In some instances, a complementarity determining region can include amino acids from both a CDR region defined according to Kabat and a hypervariable loop.

Depending on the amino acid sequence of the constant domain of their heavy chains, intact antibodies can be assigned to different "classes". There are five major classes of intact antibodies: IgA, IgD, IgE, IgG, and IgM, and several of these maybe further divided into "subclasses" (isotypes), e.g., IgG1, IgG2, IgG3, IgG4, IgA1, and IgA2. A preferred class of immunoglobulins for use in the present invention is IgG.

The heavy-chain constant domains that correspond to the different classes of antibodies are called [alpha], [delta], [epsilon], [gamma], and [mu], respectively. The subunit structures and three-dimensional configurations of different classes of immunoglobulins are well known. As used herein antibodies are conventionally known antibodies and functional fragments thereof.

A "functional fragment" or "antigen-binding antibody fragment" of an antibody/immunoglobulin hereby is defined as a fragment of an antibody/immunoglobulin (e.g., a variable region of an IgG) that retains the antigen-binding region. An "antigen-binding region" of an antibody typically is found in one or more hyper variable region(s) of an antibody, e.g., the CDR1, -2, and/or -3 regions; however, the variable "framework" regions can also play an important role in antigen binding, such as by providing a scaffold for the CDRs.

"Functional fragments", "antigen-binding antibody fragments", or "antibody fragments" of the invention include but are not limited to Fab, Fab', Fab'-SH, F(ab')₂, and Fv fragments; diabodies; single domain antibodies (DAbs), linear antibodies; single-chain antibody molecules (scFv); and multi-specific, such as bi- and tri-specific, antibodies formed from antibody fragments (C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). An antibody other than a "multi-specific" or "multi-functional" antibody is understood to have each of its binding sites identical. The F(ab')₂ or Fab may be engineered to minimize or completely remove the intermolecular disulfide interactions that occur between the CH1 and CL domains.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal Lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

Variants of the antibodies or antigen-binding antibody fragments contemplated in the invention are molecules in which the binding activity of the antibody or antigen-binding antibody fragment is maintained.

"Binding proteins" contemplated in the invention are for example antibody mimetics, such as Affibodies, Adnectins, Anticalins, DARPins, Avimers, Nanobodies (reviewed by Gebauer M. et al., Curr. Opinion in Chem. Biol. 2009; 13:245-255; Nuttall S.D. et al., Curr. Opinion in Pharmacology 2008; 8:608-617).

A "human" antibody or antigen-binding fragment thereof is hereby defined as one that is not chimeric (e.g., not "humanized") and not from (either in whole or in part) a non-human species. A human antibody or antigen-binding fragment thereof can be derived from a human or can be a synthetic human antibody. A "synthetic human antibody" is defined herein as an antibody having a sequence derived, in whole or in part, in silico from synthetic sequences that are based on the analysis of known human antibody sequences. In silico design of a human antibody sequence or fragment thereof can be achieved, for example, by analyzing a database of human antibody or antibody fragment sequences and devising a polypeptide sequence utilizing the data obtained there from. Another example of a human antibody or antigen-binding fragment thereof is one that is encoded by a nucleic acid isolated from a library of antibody sequences of human origin (e.g., such library being based on antibodies taken from a human natural source). Examples of human antibodies include antibodies as described in Söderlind et al., Nature Biotech. 2000, 18:853-856.

A "humanized antibody" or humanized antigen-binding fragment thereof is defined herein as one that is (i) derived from a non-human source (e.g., a transgenic mouse which bears a heterologous immune system), which antibody is based on a human germline sequence; (ii) where amino acids of the framework regions of a non-human antibody are partially exchanged to human amino acid sequences by genetic engineering or (iii) CDR-grafted, wherein the CDRs of the variable domain are from a non-human origin, while one or more frameworks of the variable domain are of human origin and the constant domain (if any) is of human origin.

A "chimeric antibody" or antigen-binding fragment thereof is defined herein as one, wherein the variable domains are derived from a non-human origin and some or all constant domains are derived from a human origin.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the term "monoclonal" indicates the character of the antibody as not being a mixture of discrete antibodies. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins. The term "monoclonal" is not to be construed as to require production of the antibody by any particular method. The term monoclonal antibody specifically includes chimeric, humanized and human antibodies.

An "isolated" antibody is one that has been identified and separated from a component of the cell that expressed it. Contaminant components of the cell are materials that would interfere with diagnostic or therapeutic uses of the antibody, and may include enzymes, hormones, and other proteinaceous or non-proteinaceous solutes.

An "isolated" nucleic acid is one that has been identified and separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

As used herein, an antibody "binds specifically to", is "specific to/for" or "specifically recognizes" an antigen of interest, e.g. a tumor-associated polypeptide antigen target or an antigen-binding polypeptide target (as e.g. an antigen-binding antibody), is one that binds the antigen-target with sufficient affinity such that the antibody is useful as a therapeutic agent in targeting a cell or tissue expressing the antigen or one that binds an antigen-binding polypeptide target with sufficient affinity such that the antibody is useful as a reversal agent to neutralize the therapeutic activity of this antigen-binding polypeptide (e.g. an antigen-binding antibody) and does not significantly cross-react with other proteins or does not significantly cross-react with proteins other than orthologs and variants (e.g. mutant forms, splice variants, or proteolytically truncated forms) of the aforementioned target. The term "specifically recognizes" or "binds specifically to" or is "specific to/for" a particular polypeptide or an epitope on a particular polypeptide target as used herein can be exhibited, for example, by an antibody, or antigen-binding fragment thereof, having a monovalent K_{D} for the antigen of less than about 10⁻⁴ M, alternatively less than about 10⁻⁵ M, alternatively less than about 10⁻⁶ M, alternatively less than about 10⁻⁷ M, alternatively less than about 10⁻⁸ M, alternatively less than about 10⁻⁹ M, alternatively less than about 10⁻¹⁰ M, alternatively less than about 10⁻¹¹ M, alternatively less than about 10⁻¹² M, or less. An antibody "binds specifically to," is "specific to/for" or "specifically recognizes" an antigen if such antibody is able to discriminate between such antigen and one or more reference antigen(s). In its most general form, "specific binding", "binds specifically to", is "specific to/for" or "specifically recognizes" is referring to the ability of the antibody to discriminate between the antigen of interest and an unrelated antigen, as determined, for example, in accordance with one of the following methods. Such methods comprise, but are not limited to, surface plasmon resonance (SPR), Western blots, ELISA-, RIA-, ECL-, IRMA-tests and peptide scans. For example, a standard ELISA assay can be carried out. The scoring may be carried out by standard color development (e.g., secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using not a single reference antigen, but a set of about three to five unrelated antigens, such as milk powder, BSA, transferrin or the like.

"Binding affinity" or "affinity" refers to the strength of the total sum of non-covalent interactions between a single binding site of a molecule and its binding partner. Unless indicated otherwise, as used herein, "binding affinity" refers to intrinsic binding affinity which reflects a 1:1 interaction between members of a binding pair (e.g., an antibody and an antigen). The dissociation constant "K_{D}" is commonly used to describe the affinity between a molecule (such as an antibody) and its binding partner (such as an antigen) i.e., how tightly a ligand binds to a particular protein. Ligand-protein affinities are influenced by non-covalent intermolecular interactions between the two molecules. Affinity can be measured by common methods known in the art, including those described herein. In one embodiment, the "K_{D}" or "K_{D} value" according to this invention is measured by using surface plasmon resonance assays using suitable devices including but not limited to Biacore instruments like Biacore T100, Biacore T200, Biacore 2000, Biacore 4000, a Biacore 3000 (GE Healthcare Biacore, Inc.), or a ProteOn XPR36 instrument (Bio-Rad Laboratories, Inc.).

"Antibody-dependent cell-mediated cytotoxicity" or "ADCC" refers to a form of cytotoxicity in which secreted Ig bound onto Fc gamma receptors (FcγRs) present on certain cytotoxic cells (e.g. NK cells, neutrophils, and macrophages) enable these cytotoxic effector cells to bind specifically to an antigen-bearing target cell and subsequently kill the target cell e.g. with cytotoxins. To assess ADCC activity of an antibody of interest, an in vitro ADCC assay, such as that described in US Patent No. 5,500,362 or 5,821,337 or U.S. Patent No. 6,737,056 (Presta), may be performed. Useful effector cells for such assays include PBMC and NK cells.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, e.g., as described in Gazzano-Santoro et al., J. Immunol. Methods 202: 163 (1996), may be performed. Polypeptide variants with altered Fc region amino acid sequences (polypeptides with a variant Fc region) and increased or decreased C1q binding are described, e.g., in US Patent No. 6,194,551 BI and WO 1999/51642.

"Percent (%) sequence identity" with respect to a reference polynucleotide or polypeptide sequence, respectively, is defined as the percentage of nucleic acid or amino acid residues, respectively, in a candidate sequence that are identical with the nucleic acid or amino acid residues, respectively, in the reference polynucleotide or polypeptide sequence, respectively, after aligning the sequences and introducing gaps, if necessary, to achieve the maximum percent sequence identity. Conservative substitutions are not considered as part of the sequence identity. Preferred are un-gapped alignments. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full-length of the sequences being compared.

Substantial sequence identity/similarity may be taken as having a sequence similarity/identity of at least 80% to the complete sequences and/or at least 90% to the specific binding regions (e.g., the CDR regions). Preferable sequence similarity or more preferably identity may be at least 92%, 95%, 97%, 98% or 99%. Sequence similarity and/or identity may be determined using the "BestFit" program of the Genetics Computer Group Version 10 software package from the University of Wisconsin. The program uses the local had algorithm of Smith and Waterman with default values: Gap creation penalty=8, Gap extension penalty=2, Average match=2.912, average mismatch 2.003.

The terms "polynucleotide" or "nucleic acid", as used interchangeably herein, refer to chains of nucleotides of any length, and include DNA and RNA. The nucleotides can be deoxyribonucleotides, ribonucleotides, modified nucleotides or bases, and/or their analogs, or any substrate that can be incorporated into a chain by DNA or RNA polymerase. A polynucleotide may comprise modified nucleotides, such as methylated nucleotides and their analogs.

"Sequence homology" indicates the percentage of amino acids that either is identical or that represent conservative amino acid substitutions.

In addition, the nucleic acid sequences encoding variable regions of the heavy and/or light chains can be converted, for example, to nucleic acid sequences encoding full-length antibody chains, Fab fragments, or to scFv. The VL- or VH-encoding DNA fragment can be operatively linked, (such that the amino acid sequences encoded by the two DNA fragments are in-frame) to another DNA fragment encoding, for example, an antibody constant region or a flexible linker. The sequences of human heavy chain and light chain constant regions are known in the art (see e.g., Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification.

To create a polynucleotide sequence that encodes a scFv, the VH- and VL-encoding nucleic acids can be operatively linked to another fragment encoding a flexible linker such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., Nature (1990) 348:552-554).

To express the antibodies, antigen binding fragments thereof or variants thereof standard recombinant DNA expression methods can be used (see, for example, Goeddel; Gene Expression Technology. Methods in Enzymology 185, Academic Press, San Diego, Calif. (1990)). For example, DNA encoding the desired polypeptide can be inserted into an expression vector which is then transfected into a suitable host cell. Suitable host cells are prokaryotic and eukaryotic cells. Examples for prokaryotic host cells are e.g., bacteria, examples for eukaryotic hosts cells are yeasts, insects and insect cells, plants and plant cells, transgenic animals, or mammalian cells. In some embodiments, the DNAs encoding the heavy and light chains are inserted into separate vectors. In other embodiments, the DNA encoding the heavy and light chains is inserted into the same vector. It is understood that the design of the expression vector, including the selection of regulatory sequences is affected by factors such as the choice of the host cell, the level of expression of protein desired and whether expression is constitutive or inducible.

Useful expression vectors for bacterial use are constructed by inserting a DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include but are not limited to E. coli, Bacillus subtilis, Salmonella typhimurium and various species within the genera Pseudomonas, Streptomyces, and Staphylococcus.

Bacterial vectors may be, for example, bacteriophage-, plasmid- or phagemid-based. These vectors can contain a selectable marker and a bacterial origin of replication derived from commercially available plasmids typically containing elements of the well-known cloning vector pBR322 (ATCC 37017). Following transformation of a suitable host strain and growth of the host strain to an appropriate cell density, the selected promoter is de-repressed/induced by appropriate means (e.g., temperature shift or chemical induction) and cells are cultured for an additional period. Cells are typically harvested by centrifugation, disrupted by physical or chemical means, and the resulting crude extract retained for further purification.

In bacterial systems, a number of expression vectors may be advantageously selected depending upon the use intended for the protein being expressed. For example, when a large quantity of such a protein is to be produced, for the generation of antibodies or to screen peptide libraries, for example, vectors which direct the expression of high levels of fusion protein products that are readily purified may be desirable.

Antibodies of the present invention or antigen-binding fragments thereof or variants thereof include naturally purified products, products of chemical synthetic procedures, and products produced by recombinant techniques from a prokaryotic host, including, for example, E. coli, Bacillus subtilis, Salmonella typhimurium and various species within the genera Pseudomonas, Streptomyces, and Staphylococcus, preferably, from E. coli cells.

Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. Expression of the antibodies may be constitutive or regulated (e.g. inducible by addition or removal of small molecule inductors such as Tetracyclin in conjunction with Tet system). For further description of viral regulatory elements, and sequences thereof, see e.g., U.S. 5,168,062 by Stinski, U.S. 4,510,245 by Bell et al. and U.S. 4,968,615 by Schaffner et al.. The recombinant expression vectors can also include origins of replication and selectable markers (see e.g., U.S. 4,399,216, 4,634,665 and U.S. 5,179,017). Suitable selectable markers include genes that confer resistance to drugs such as G418, puromycin, hygromycin, blasticidin, zeocin/bleomycin or methotrexate or selectable marker that exploit auxotrophies such as Glutamine Synthetase (Bebbington et al., Biotechnology (N Y). 1992 Feb;10(2):169-75), on a host cell into which the vector has been introduced. For example, the dihydrofolate reductase (DHFR) gene confers resistance to methotrexate, neo gene confers resistance to G418, the bsd gene from Aspergillus terreus confers resistance to blasticidin, puromycin N-acetyl-transferase confers resistance to puromycin, the Sh ble gene product confers resitance to zeocin, and resistance to hygromycin is conferred by the E. coli hygromycin resistance gene (hyg or hph). Selectable markers like DHFR or Glutamine Synthetase are also useful for amplification techniques in conjunction with MTX and MSX.

Transfection of the expression vector into a host cell can be carried out using standard techniques such as electroporation, nucleofection, calcium-phosphate precipitation, lipofection, polycation-based transfection such as polyethlylenimine (PEI)-based transfection and DEAE-dextran transfection.

Suitable mammalian host cells for expressing the antibodies, antigen binding fragments thereof or variants thereof provided herein include but are not limited to Chinese Hamster Ovary (CHO cells) such as CHO-K1, CHO-S, CHO-K1SV [including dhfr- CHO cells, described in Urlaub and Chasin, (1980) Proc. Natl. Acad. Sci. USA 77:4216-4220 and Urlaub et al., Cell. 1983 Jun;33(2):405-12, used with a DHFR selectable marker, e.g., as described in R. J. Kaufman and P. A. Sharp (1982) Mol. Biol. 159:601-621; and other knockout cells exemplified in Fan et al., Biotechnol Bioeng. 2012 Apr;109(4):1007-15], NSO myeloma cells, COS cells, HEK293 cells, HKB11 cells, BHK21 cells, CAP cells, EB66 cells, and SP2 cells.

Expression might also be transient or semi-stable in expression systems such as HEK293, HEK293T, HEK293-EBNA, HEK293E, HEK293-6E, HEK293-Freestyle, HKB11, Expi293F, 293EBNALT75, CHO Freestyle, CHO-S, CHO-K1, CHO-K1SV, CHOEBNALT85, CHOS-XE, CHO-3E7 or CAP-T cells (for instance Durocher et al., Nucleic Acids Res. 2002 Jan 15;30(2):E9).

In some embodiments, the expression vector is designed such that the expressed protein is secreted into the culture medium in which the host cells are grown. The antibodies, antigen binding fragments thereof or variants thereof can be recovered from the culture medium using standard protein purification methods.

Antibodies of the invention or antigen-binding fragments thereof or variants thereof can be recovered and purified from recombinant cell cultures by well-known methods including, but not limited to ammonium sulfate or ethanol precipitation, acid extraction, Protein A chromatography, Protein G chromatography, anion or cation exchange chromatography, phospho-cellulose chromatography, hydrophobic interaction chromatography, affinity chromatography, hydroxylapatite chromatography, mixed mode chromatography and lectin chromatography. High performance liquid chromatography ("HPLC") can also be employed for purification. See, e.g., Colligan, Current Protocols in Immunology, or Current Protocols in

Protein Science, John Wiley & Sons, NY, N.Y., (1997-2001), e.g., Chapters 1, 4, 6, 8, 9, 10.

Antibodies of the present invention or antigen-binding fragments thereof or variants thereof include naturally purified products, products of chemical synthetic procedures, and products produced by recombinant techniques from an eukaryotic host, including, for example, yeast, higher plant, insect and mammalian cells. Depending upon the host employed in a recombinant production procedure, the antibody of the present invention can be glycosylated or can be non-glycosylated. Such methods are described in many standard laboratory manuals, such as Sambrook, supra, Sections 17.37-17.42; Ausubel, supra, Chapters 10, 12, 13, 16, 18 and 20. In preferred embodiments, the antibody is purified (1) to greater than 95% by weight of antibody as determined e.g. by the Lowry method, UV-Vis spectroscopy or by by SDS-Capillary Gel electrophoresis (for example on a Caliper LabChip GXII, GX 90 or Biorad Bioanalyzer device), and in further preferred embodiments more than 99% by weight, (2) to a degree sufficient to obtain at least 15 residues of N-terminal or internal amino acid sequence, or (3) to homogeneity by SDS-PAGE under reducing or non-reducing conditions using Coomassie blue or, preferably, silver stain. Isolated naturally occurring antibody includes the antibody in situ within recombinant cells since at least one component of the antibody's natural environment will not be present. Ordinarily, however, isolated antibody will be prepared by at least one purification step.

As used in the context of the present invention, the term "Ac225-Macropa-Pelgifatamab" refers to the following compound: 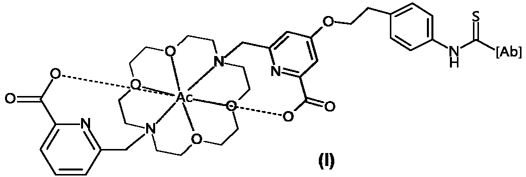 wherein [Ab] is the anti-PSMA monoclonal antibody Pelgifatamab or an antigen-binding fragment thereof which is bound to the rest of the molecule via a terminal amino group of a Lysine residue of the monoclonal antibody or antigen-binding fragment thereof.

As used in the context of the present invention, the term "Ac225-DOTA-Pelgifatamab" refers to the following compound: 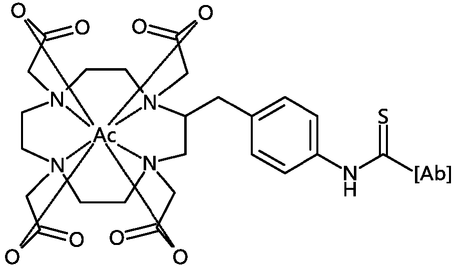 wherein [Ab] is the anti-PSMA monoclonal antibody Pelgifatamab or an antigen-binding fragment thereof which is bound to the rest of the molecule via a terminal amino group of a Lysine residue of the monoclonal antibody or antigen-binding fragment thereof.

The present invention comprises combinations comprising a tissue-targeting compound of formula (I) as described herein and one or more further compounds having biological activity (i.e., a further pharmaceutical agent), preferably against cancer, especially prostate cancer. These compounds are defined by their classes, thus allowing a skilled person to identify such compounds belonging to said class.

In the context of the present invention, a non-steroidal antiandrogen refers to an antiandrogen with a nonsteroidal chemical structure which is typically selective and a full or silent antagonist of the androgen receptor. Examples thereof include, but are not limited to, darolutamide, bicalutamide, enzalutamide, apalutamide, flutamide, nilutamide, topilutamide, proxalutamide or bavdegalutamide or any combination thereof. In the context of the present invention, the non-steroidal antiandrogen is preferably selected from darolutamide and enzalutamide, more preferably the non-steroidal antiandrogen is darolutamide. In certain embodiments, the non-steroidal antiandrogen is enzalutamide. In certain embodiments, the non-steroidal antiandrogen is apalutamide. In further embodiments the non-steroidal antiandrogen is bicalutamide, flutamide or nilutamide. In other further embodiments the non-steroidal antiandrogen is ARV-766, EPI-7386, CC-94676, AC-0176, HP-518 or TAS-3681. Preferably, the non-steroidal antiandrogen is darolutamide.

In the context of the present invention, a steroidal antiandrogen refers to an antiandrogen with a steroidal chemical structure which is typically an antagonist of the androgen receptor. Examples thereof include, but are not limited to, Cyproterone acetate, Allylestrenol, Chlormadinone acetate, Delmadinone acetate, Gestonorone caproate, Hydroxyprogesterone caproate, Medroxyprogesterone acetate, Megestrol acetate, Osaterone acetate, Oxendolone and Spironolactone In the context of the present invention, the steroidal antiandrogen is preferably Cyproterone acetate.

In the context of the present invention, an androgen synthesis inhibitor refers to enzyme inhibitors that prevent the biosynthesis of androgens. Examples thereof include, but are not limited to, ketoconazole, abiraterone, aminoglutethimide, goserelin and seviteronel. In the context of the present invention, the androgen synthesis inhibitor is preferably arbiraterone or goserelin.

In the context of the present invention, an antigonadotropin refers to a compound which suppresses the activity and/or downstream effects of one or both of the gonadotropins, follicle-stimulating hormone (FSH) and luteinizing hormone (LH). Examples thereof include, but are not limited to, Abarelix, Danazol, Gestrinone, Paroxypropione, Cetrorelix, Degarelix, Elagolix, Ganirelix, Linzagolix and Relugolix. In the context of the present invention, the antigonadotropin is preferably Degarelix or Relugolix.

In the context of the present invention, a PARP inhibitor refers to a compound which is a pharmacological inhibitor of the enzyme poly-ADP ribose polymerase (PARP). Examples thereof include, but are not limited to, Olaparib, Rucaparib, Veliparib, Niraparib, Talazoparib, Pamiparib, CEP 9722, E7016 and Iniparib. In the context of the present invention, the PARP inhibitor is preferably Olaparib or Rucaparib.

In the context of the present invention, an ATR inhibitor refers to a compound which is a pharmacological inhibitor of the enzyme ATR serine/threonine-protein kinase, also known as ataxia telangiectasia and Rad3-related protein or FRAP-related protein 1 (FRP1). Examples thereof include, but are not limited to, Berzosertib, BAY1895344 (Elimusertib), AZD6738, M6620, AZ20 and VE 821. In the context of the present invention, the PARP inhibitor is preferably Berzosertib or BAY1895344.

In the context of the present invention, an ATM inhibitor refers to a compound which is a pharmacological inhibitor of the enzyme ATM serine/threonine kinase. Examples thereof include, but are not limited to, AZD1390, KU-55933, KU-60019, KU-59403, CP-466722, AZ31/AZ32, AZD0156.

In the context of the present invention, a DNA-PK inhibitor refers to a compound which is a pharmacological inhibitor of the DNA-dependent protein kinase (DNA-PK). Examples thereof include, but are not limited to, AZD7648, Nedisertib, VX-984, CC-115, Samotolisib and BAY-8400. In the context of the present invention, the DNA-PK inhibitor is preferably Nedisertib or BAY-8400.

In the context of the present invention, an AKT inhibitor refers to a compound which is a pharmacological inhibitor of the serine/threonine kinase AKT. Examples thereof include, but are not limited to, Ipatasertib, afuresertib, miransertib, capivasertib, uprosertib and MK2206. In the context of the present invention, the AKT inhibitor is preferably Ipatasertib.

In the context of the present invention, a Pi3K inhibitor refers to a compound which is capable of inhibiting one or more of the phosphoinositide 3-kinase (PI3K) enzymes, which are part of the PI3K/AKT/mTOR pathway. Examples thereof include, but are not limited to, Copanlisib, Buparlisib, Duvelisib, Idelalisib, Paxalisib, Zandelisib, Inavolisib, Duvelisib, Alpelisib and Umbralisib. In the context of the present invention, the Pi3K inhibitor is preferably Copanlisib.

In the context of the present invention, a PSMA-targeting beta emitter refers to a compound comprising a radionuclide capable of emitting beta radiation. Examples thereof include, but are not limited to, (177Lu)-vipivotide tetraxetan (Pluvicto), 177Lu-PSMA-I&T and PNT2002.

In the context of the present invention, an immune checkpoint inhibitor refers to a compound capable of blocking proteins called checkpoints that are made by some types of immune system cells, such as T cells, and some cancer cells. Checkpoints help keep immune responses from being too strong and sometimes can keep T cells from killing cancer cells. Examples thereof include, but are not limited to, Atezolizumab, Durvalumab, Avelumab, Nivolumab, Pembrolizumab and Ipilimumab.

In the context of the present invention, an alpha emitter refers to a compound comprising a radionuclide capable of undergoing radioactive decay in which the radionuclide emits an alpha particle (helium nucleus) and thereby transforms or 'decays' into a different atomic nucleus, with a mass number that is reduced by four and an atomic number that is reduced by two. Examples thereof include, but are not limited to, 223Ra, J591-225Ac and PSMA-617-225Ac.

In the context of the present invention, a vaccine refers to a compound suitable for cell-based cancer immunotherapy for prostate cancer. Examples thereof include, but are not limited to, sipoleucel T.

In the context of the present invention, a chemotherapeutic agent refers to a compound suitable for chemotherapy of prostate cancer. Examples thereof include, but are not limited to, Paclitaxel, Docetaxel, Ixabepilone, Vinorelbine, Nocodazole, Vincristine, Colchicine and Eribulin. In the context of the present invention, the chemotherapeutic agent is preferably Paclitaxel or Docetaxel.

In the context of the present invention, external beam radiotherapy refers to radiation therapy in which an external source of ionizing radiation is pointed at and/or applied to a particular part of the body of a subject.

In the context of the present invention, the term "combination" means not only a dosage form which contains all the components (i.e., the tissue-targeting compound of formula (I) and a further pharmaceutical agent, also called fixed-dose combinations), and combination packs containing the components separate from one another, but also components which are administered simultaneously or sequentially, as long as they are employed for the prophylaxis or treatment of the same disease.

The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

### DESCRIPTION OF THE INVENTION

### Compounds

In accordance with a first aspect, the present invention relates to a tissue-targeting compound of formula (I) 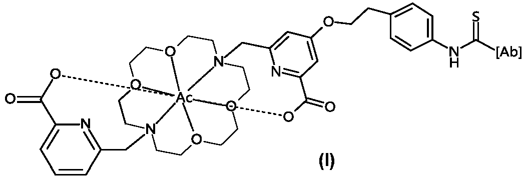 wherein [Ab] is a monoclonal antibody or an antigen-binding fragment thereof having binding affinity for the prostate-specific membrane antigen (PSMA).

In a further embodiment, the present invention relates to a tissue-targeting compound of formula (I) 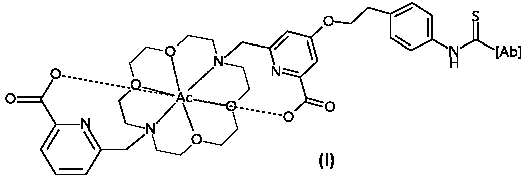 wherein [Ab] is a monoclonal antibody or an antigen-binding fragment thereof having binding affinity for the prostate-specific membrane antigen (PSMA), wherein [Ab] is bound to the rest of the molecule via a terminal amino group of a Lysine residue of the monoclonal antibody or antigen-binding fragment thereof.

In a further embodiment, the present invention relates to a tissue-targeting compound of formula (I) 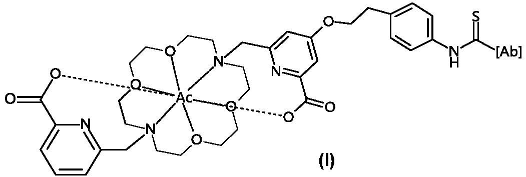 wherein [Ab] is a monoclonal antibody or an antigen-binding fragment thereof having binding affinity for the prostate-specific membrane antigen (PSMA) selected from Pelgifatamab, J591 and TLX592.

In a further embodiment, the present invention relates to a tissue-targeting compound of formula (I) 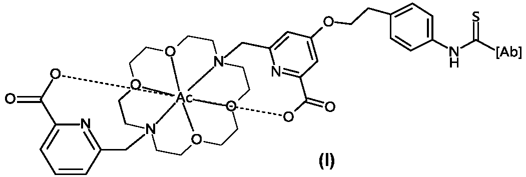 wherein [Ab] is an anti-PSMA monoclonal antibody selected from Pelgifatamab, J591 and TLX592 or an antigen-binding fragment thereof.

In a further embodiment, the present invention relates to a tissue-targeting compound of formula (I) 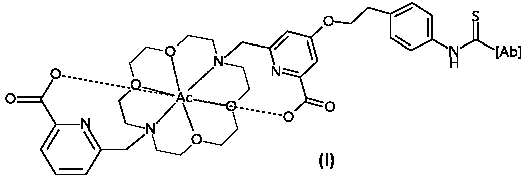 wherein [Ab] is a monoclonal antibody or an antigen-binding fragment thereof having binding affinity for the prostate-specific membrane antigen (PSMA) selected from Pelgifatamab and J591.

In a further embodiment, the present invention relates to a tissue-targeting compound of formula (I) 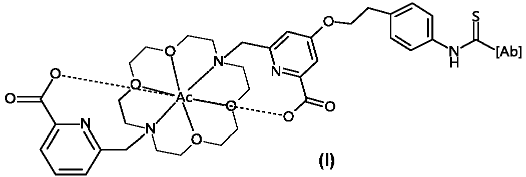 wherein [Ab] is an anti-PSMA monoclonal antibody selected from Pelgifatamab and J591 or an antigen-binding fragment thereof.

In a further embodiment, the present invention relates to a tissue-targeting compound of formula (I) 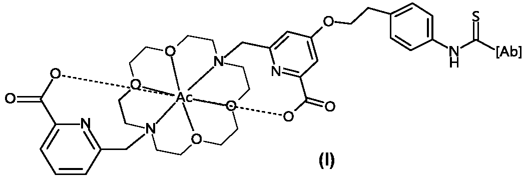 wherein [Ab] is the monoclonal antibody or an antigen-binding fragment thereof having binding affinity for the prostate-specific membrane antigen (PSMA) J591.

In a further embodiment, the present invention relates to a tissue-targeting compound of formula (I) 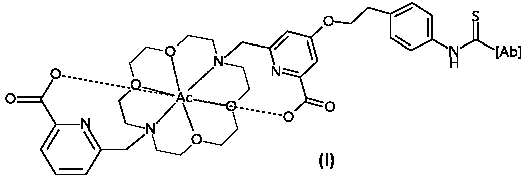 wherein [Ab] is the anti-PSMA monoclonal antibody J591 or an antigen-binding fragment thereof.

In a further embodiment, the present invention relates to a tissue-targeting compound of formula (I) 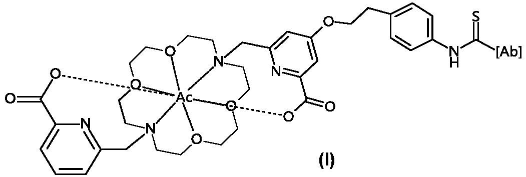 wherein [Ab] is the monoclonal antibody or an antigen-binding fragment thereof having binding affinity for the prostate-specific membrane antigen (PSMA) Pelgifatamab.

In a further embodiment, the present invention relates to a tissue-targeting compound of formula (I) 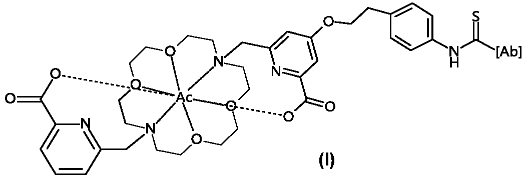 wherein [Ab] is the monoclonal antibody or an antigen-binding fragment thereof having binding affinity for the prostate-specific membrane antigen (PSMA) Pelgifatamab, wherein Pelgifatamab or an antigen-binding fragment thereof is bound to the rest of the molecule via a terminal amino group of a Lysine residue of the monoclonal antibody or antigen-binding fragment thereof.

In a further embodiment, the present invention relates to a tissue-targeting compound of formula (I) 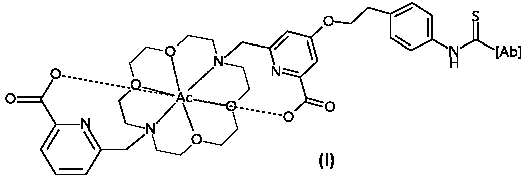 wherein [Ab] is the anti-PSMA monoclonal antibody Pelgifatamab or an antigen-binding fragment thereof.

In a further embodiment, the present invention relates to a tissue-targeting compound of formula (I) 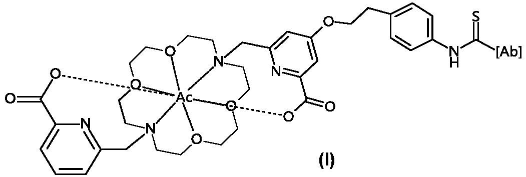 wherein [Ab] is the anti-PSMA monoclonal antibody Pelgifatamab or an antigen-binding fragment thereof, wherein Pelgifatamab or an antigen-binding fragment thereof is bound to the rest of the molecule via a terminal amino group of a Lysine residue of the monoclonal antibody or antigen-binding fragment thereof.

In a further embodiment, the present invention relates to a tissue-targeting compound of formula (I) 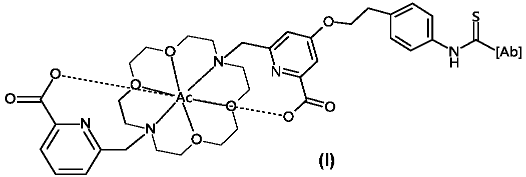 wherein [Ab] is the anti-PSMA monoclonal antibody Pelgifatamab or an antigen-binding fragment thereof comprising a heavy chain nucleotide sequence with sequence similarity or identity with SEQ ID NO. 1 and a light chain nucleotide sequence with sequence similarity or identity with SEQ ID NO. 2.

In a further embodiment, the present invention relates to a tissue-targeting compound of formula (I) 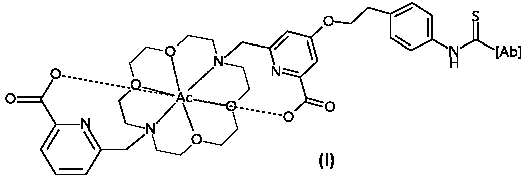 wherein [Ab] is the anti-PSMA monoclonal antibody Pelgifatamab or an antigen-binding fragment thereof comprising a heavy chain sequence according to SEQ ID NO. 1, and a light chain sequence according to SEQ ID NO. 2.

In a further embodiment, the present invention relates to a tissue-targeting compound of formula (I) 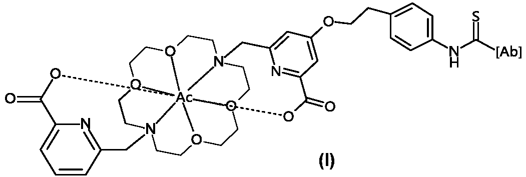 wherein [Ab] is the anti-PSMA monoclonal antibody Pelgifatamab or an antigen-binding fragment thereof comprising a heavy chain sequence according to SEQ ID NO. 1, and a light chain sequence according to SEQ ID NO. 2, wherein Pelgifatamab or an antigen-binding fragment thereof is bound to the rest of the molecule via a terminal amino group of a Lysine residue of the monoclonal antibody or antigen-binding fragment thereof.

In a further embodiment, the present invention relates to a tissue-targeting compound of formula (I) 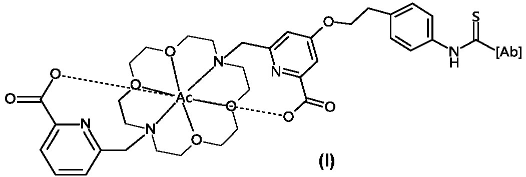 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10.

In a further embodiment, the present invention relates to a tissue-targeting compound of formula (I) 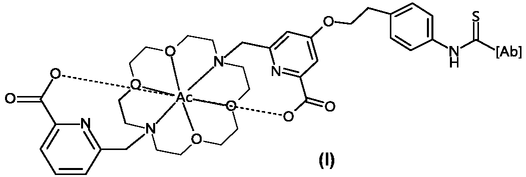 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, wherein Pelgifatamab or an antigen-binding fragment thereof is bound to the rest of the molecule via a terminal amino group of a Lysine residue of the monoclonal antibody or antigen-binding fragment thereof

In a further embodiment, the present invention relates to a tissue-targeting compound of formula (I) 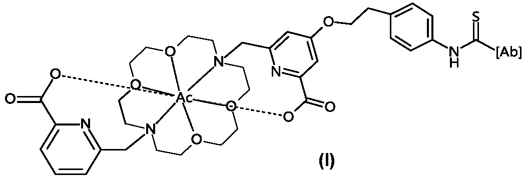 wherein [Ab] is the anti-PSMA monoclonal antibody Pelgifatamab or an antigen-binding fragment thereof comprising a heavy chain sequence according to SEQ ID NO. 1:
SEQ ID NO. 1 - Heavy Chain 
caggtgcagctcgtggaatcgggggggggcgtggtccagccggggcggtcgctccgcctctcgtgcgcagcctcgggcttcgc
cttcagccgctacggcatgcactgggtccgccaggcgcccggcaagggcctggagtgggtcgcggtcatctggtacgacgggt
ccaacaagtattatgcagactccgtcaagggtcggttcaccatcagccgggacaactccaagaacacccagtacctgcagatg
aacagccttcgagcggaggacaccgcggtctactactgcgcccggggcggggatttcctgtactactactactatggcatggacg
tctggggccagggtaccaccgtgaccgtatcctccgcatccactaagggcccgtccgttttcccgctggcaccttcgtcgaagagc
acgtccgggggcacagccgcgctgggctgcctcgtgaaggactactttccggagcccgtcaccgtgtcatggaactcgggcgc
gctcacgtccggcgtccacacgttcccggccgtattgcagagctcgggactctactccctgagctcggtcgtcaccgtgccctcctc
gagcctcggcacccagacctacatctgcaacgtgaaccacaaaccctccaatacgaaggtggacaagcgcgtcgaaccgaa
gtcgtgcgacaagacccacacgtgcccgccgtgcccggcaccggagctgctcggcgggccctcggtcttcctgttcccgccgaa
 
gcccaaggacaccctgatgatctcgcgcaccccggaggtcacatgcgtggtcgtggacgtgtcccacgaagacccggaggtc
aagttcaactggtacgtcgacggcgtcgaggtccacaacgcgaagaccaagccgcgggaggagcagtacaacagcacgta
ccgcgtcgtttccgtcctcaccgtcttgcaccaggactggctgaacggcaaggagtacaagtgtaaggtgtccaacaaggccttg
cccgcccccattgagaagaccatcagcaaggccaagggccagccccgcgagccgcaggtctacaccctgcccccgtcgcgc
gaagaaatgacgaagaaccaggtctccctgacctgcctcgtcaaggggttctacccgtcggacatcgcagtggagtgggagtc
gaacggccagcccgagaataactacaagacgacccccccagtcctggactccgacggcagcttcttcctttactccaagctgac
cgtcgacaagtcgcggtggcagcagggcaacgtcttctcgtgctcggtgatgcacgaggcgctgcacaaccactacacccaga
agtccctctcgctgtcgccgggcaagtga,
 and a light chain sequence according to SEQ ID NO. 2:
SEQ ID NO. 2 - Light Chain

gacatccagatgacccagagccccagctccctgtccgcgtcggtcggagaccgggtcaccatcacctgccgcgcgtcgcagg
gcatctcgaactacctggcgtggtaccagcagaagaccggcaaggtgccgaagttccttatctacgaggcctctacgctccagtc
gggagtcccgtcgcggttcagcgggggtggctccgggaccgacttcacgctgaccatctcgagtctccagccggaggacgtcg
cgacctattactgtcagaactacaactcggcccccttcaccttcggcccgggcaccaaggtcgacatcaagcgcaccgtcgcgg
cgccgtccgtcttcatcttccccccgtcggacgagcagctcaagtccggcaccgcctcggtcgtgtgcctgctcaacaacttctacc
cccgagaggcaaaggtgcagtggaaggtcgacaacgccctgcagtccggcaactcgcaggagagcgtcacggagcagga
cagcaaggacagtacctacagcctctcctccaccctgaccctgagtaaagccgactacgagaagcacaaggtgtacgcgtgc
gaagtcacccaccagggcctctcgtcccccgtgaccaagtccttcaaccggggcgagtgctga.

In a further embodiment, the present invention relates to a tissue-targeting compound of formula (I) 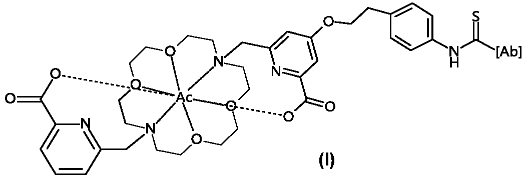 wherein [Ab] is the anti-PSMA monoclonal antibody Pelgifatamab or an antigen-binding fragment thereof comprising a heavy chain amino acid sequence with sequence similarity or identity with SEQ ID NO. 3 and a light chain amino acid sequence with sequence similarity or identity with SEQ ID NO. 4.

In a further embodiment, the present invention relates to a tissue-targeting compound of formula (I) 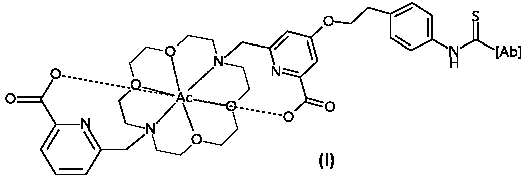 wherein [Ab] is the anti-PSMA monoclonal antibody Pelgifatamab or an antigen-binding fragment thereof comprising a heavy chain amino acid sequence according to SEQ ID NO. 3, and a light chain amino acid sequence according to SEQ ID NO. 4.

In a further embodiment, the present invention relates to a tissue-targeting compound of formula (I) 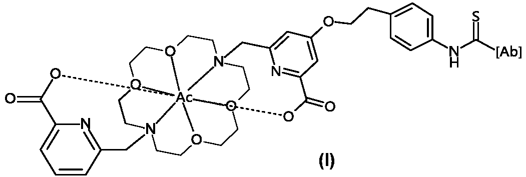 wherein [Ab] is the anti-PSMA monoclonal antibody Pelgifatamab or an antigen-binding fragment thereof comprising a heavy chain amino acid sequence according to SEQ ID NO. 3:
SEQ ID NO. 3 - Heavy Chain 
QVQLVESGGG VVQPGRSLRL SCAASGFAFS RYGMHWVRQA PGKGLEWVAV
IWYDGSNKYY ADSVKGRFTI SRDNSKNTQY LQMNSLRAED TAVYYCARGG
DFLYYYYYGM DVWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PG(K)
 and a light chain amino acid sequence according to SEQ ID NO. 4:
SEQ ID NO. 4 - Light Chain

DIQMTQSPSS LSASVGDRVT ITCRASQGIS NYLAWYQQKT GKVPKFLIYE
ASTLQSGVPS RFSGGGSGTD FTLTISSLQP EDVATYYCQN YNSAPFTFGP
GTKVDIKRTV AAPSVFIFPP SDEQLKSGTA SVVCLLNNFY PREAKVQWKV
DNALQSGNSQ ESVTEQDSKD STYSLSSTLT LSKADYEKHK VYACEVTHQG
LSSPVTKSFN RGEC.

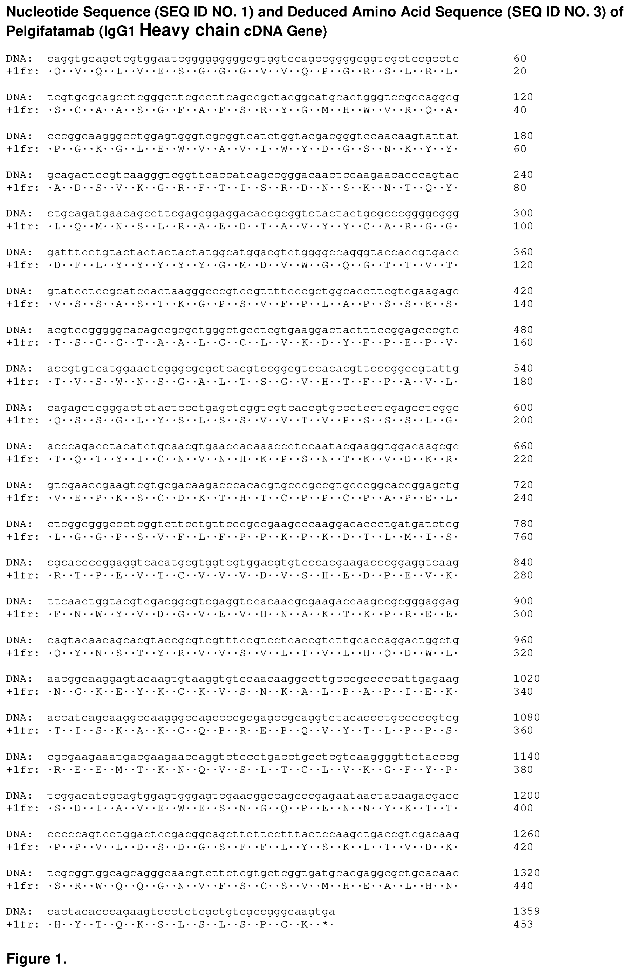

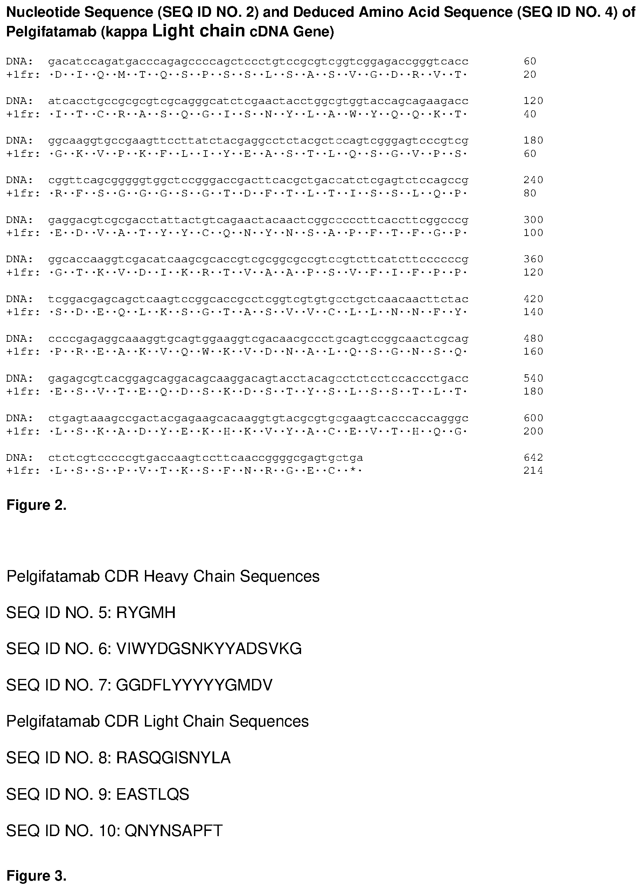

In a further embodiment, the present invention relates to a tissue-targeting compound of formula (I) 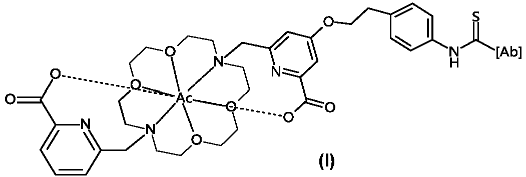 wherein [Ab] is the anti-PSMA monoclonal antibody Pelgifatamab or an antigen-binding fragment thereof comprising a heavy chain nucleotide sequence and/or a deduced amino acid sequence with similarity or identity with the sequences shown in Figure 1 and a light chain nucleotide sequence and/or a deduced amino acid sequence with similarity or identity with the sequences shown in Figure 2.

In a further embodiment, the present invention relates to a tissue-targeting compound of formula (I) 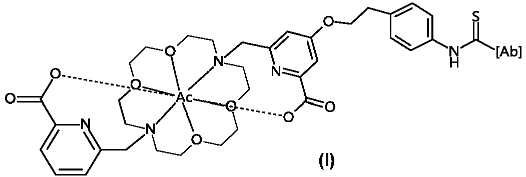 wherein [Ab] is the anti-PSMA monoclonal antibody Pelgifatamab or an antigen-binding fragment thereof comprising a heavy chain nucleotide sequence and/or a deduced amino acid sequence according to the sequences shown in Figure 1 and a light chain nucleotide sequence and/or a deduced amino acid sequence according to the sequences shown in Figure 2.

In a further embodiment, the present invention relates to a tissue-targeting compound of formula (I) 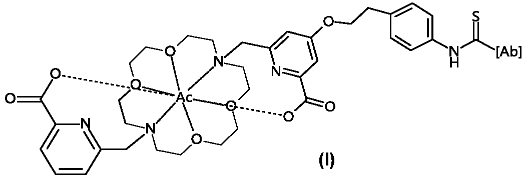 wherein [Ab] is the anti-PSMA monoclonal antibody Pelgifatamab or an antigen-binding fragment thereof comprising at least the three CDR heavy chain sequences and the three CDR light chain sequences according to the sequences shown in Figure 3.

In a further embodiment, the present invention relates to a tissue-targeting compound of formula (I) 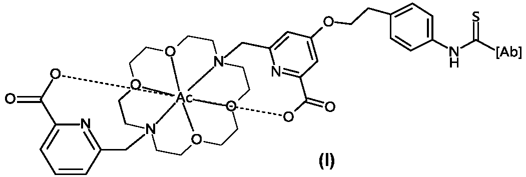 wherein [Ab] is the anti-PSMA monoclonal antibody Pelgifatamab or an antigen-binding fragment thereof comprising at least the three CDR heavy chain sequences and the three CDR light chain sequences according to the sequences shown in Figure 3, wherein Pelgifatamab or an antigen-binding fragment thereof is bound to the rest of the molecule via a terminal amino group of a Lysine residue of the monoclonal antibody or antigen-binding fragment thereof

The tissue-targeting compounds of formula (I) of the present invention comprise a chelating moiety capable of binding actinium. Preferably, the chelating moiety comprises an 18-membered chelating moiety. Preferably, the chelating moiety comprises the macrocyclic chelator N,N'-bis[(6-carboxy-2-pyridil)methyl]-4,13-diaza-18-crown-6 (macropa).

The macrocyclic chelator macropa has been described, for example, in Thiele et al., Angew. Chem. Int. Ed. 2017, 56(46), 14712 and references therein. Macropa chelators and derivatives thereof have also been described in WO2018/183906 and WO2020/106886 and references therein.

The actinium-complexating chelating moiety preferably terminates in an isothiocyanate or carboxylic acid moiety in order to facilitate its coupling with an anti-PSMA antibody or an antigen-binding fragment thereof. When the macropa chelator is used, one of the pyridine rings of the macropa chelator is typically substituted so that it can be coupled to the anti-PSMA antibody or an antigen-binding fragment thereof. This can be a simple functional group (e.g., a carboxylic acid -COOH) or a more complex chemical structure, as long as it is capable of being coupled to an anti-PSMA antibody or an antigen-binding fragment thereof. In particular, the substituent on the pyridine ring of the macropa chelator preferably terminates in an isothiocyanate or carboxylic acid moiety.

The anti-PSMA antibody or an antigen-binding fragment thereof is preferably coupled to the rest of the molecule forming the tissue-targeting compounds of the present invention through a Lysine residue of the antibody. Thus, the actinium-complexating chelating moiety and the anti-PSMA antibody or the antigen-binding fragment thereof can be coupled via an amide or thiourea moiety. When using the preferred macrocyclic chelator macropa, a terminal isothiocyanate or carboxylic acid moiety is coupled with a terminal amino group of a Lysine residue in the anti-PSMA antibody or an antigen-binding fragment thereof, leading to the formation of a thiourea or an amide group, respectively. Thus, the preferred actinium-complexating chelating moiety comprising a macropa chelator is attached to an anti-PSMA antibody or an antigen-binding fragment thereof via a thiourea or an amide bond derived from a terminal amino group of a Lysine residue of said anti-PSMA antibody or an antigen-binding fragment thereof.

Preferred antibodies or antigen-binding fragment thereof in the compounds of the present invention have binding affinity for PSMA (anti-PSMA antibodies). Prostate-specific membrane antigen (PSMA) is an enzyme that in humans is encoded by the FOLH1 (folate hydrolase 1) gene. Specific binding fragments such as Fab, Fab', F(ab')2 and single-chain specific binding antibodies are typical fragments. In particular, it is preferred that the anti-PSMA antibody is selected from Pelgifatamab, J591 and TLX592 or an antigen-binding fragment thereof. Preferably, the anti-PSMA antibody is selected from Pelgifatamab and J591 or an antigen-binding fragment thereof. More preferably, the anti-PSMA antibody is Pelgifatamab or an antigen-binding fragment thereof.

Pelgifatamab (CAS Registry Number 2414550-93-7, INN published in WHO Recommended List 86, WHO Drug Information, Vol.35, No-3, 2021) is a monoclonal antibody having binding affinity for the antigen PSMA (anti-PSMA antibody). Its preparation, isolation and purification has been described in e.g., US8114965 (Pelgifatamab=clone006).

Pelgifatamab comprises a heavy chain nucleotide sequence according to SEQ ID NO. 1:
SEQ ID NO. 1 - Heavy Chain 
caggtgcagctcgtggaatcgggggggggcgtggtccagccggggcggtcgctccgcctctcgtgcgcagcctcgggcttcgc
cttcagccgctacggcatgcactgggtccgccaggcgcccggcaagggcctggagtgggtcgcggtcatctggtacgacgggt
ccaacaagtattatgcagactccgtcaagggtcggttcaccatcagccgggacaactccaagaacacccagtacctgcagatg
aacagccttcgagcggaggacaccgcggtctactactgcgcccggggcggggatttcctgtactactactactatggcatggacg
tctggggccagggtaccaccgtgaccgtatcctccgcatccactaagggcccgtccgttttcccgctggcaccttcgtcgaagagc
acgtccgggggcacagccgcgctgggctgcctcgtgaaggactactttccggagcccgtcaccgtgtcatggaactcgggcgc
gctcacgtccggcgtccacacgttcccggccgtattgcagagctcgggactctactccctgagctcggtcgtcaccgtgccctcctc
gagcctcggcacccagacctacatctgcaacgtgaaccacaaaccctccaatacgaaggtggacaagcgcgtcgaaccgaa
gtcgtgcgacaagacccacacgtgcccgccgtgcccggcaccggagctgctcggcgggccctcggtcttcctgttcccgccgaa
gcccaaggacaccctgatgatctcgcgcaccccggaggtcacatgcgtggtcgtggacgtgtcccacgaagacccggaggtc
aagttcaactggtacgtcgacggcgtcgaggtccacaacgcgaagaccaagccgcgggaggagcagtacaacagcacgta
ccgcgtcgtttccgtcctcaccgtcttgcaccaggactggctgaacggcaaggagtacaagtgtaaggtgtccaacaaggccttg
cccgcccccattgagaagaccatcagcaaggccaagggccagccccgcgagccgcaggtctacaccctgcccccgtcgcgc
gaagaaatgacgaagaaccaggtctccctgacctgcctcgtcaaggggttctacccgtcggacatcgcagtggagtgggagtc
gaacggccagcccgagaataactacaagacgacccccccagtcctggactccgacggcagcttcttcctttactccaagctgac
cgtcgacaagtcgcggtggcagcagggcaacgtcttctcgtgctcggtgatgcacgaggcgctgcacaaccactacacccaga
agtccctctcgctgtcgccgggcaagtga,
 and a light chain nucleotide sequence according to SEQ ID NO. 2:
SEQ ID NO. 2 - Light Chain

gacatccagatgacccagagccccagctccctgtccgcgtcggtcggagaccgggtcaccatcacctgccgcgcgtcgcagg
gcatctcgaactacctggcgtggtaccagcagaagaccggcaaggtgccgaagttccttatctacgaggcctctacgctccagtc
gggagtcccgtcgcggttcagcgggggtggctccgggaccgacttcacgctgaccatctcgagtctccagccggaggacgtcg
cgacctattactgtcagaactacaactcggcccccttcaccttcggcccgggcaccaaggtcgacatcaagcgcaccgtcgcgg
cgccgtccgtcttcatcttccccccgtcggacgagcagctcaagtccggcaccgcctcggtcgtgtgcctgctcaacaacttctacc
cccgagaggcaaaggtgcagtggaaggtcgacaacgccctgcagtccggcaactcgcaggagagcgtcacggagcagga
cagcaaggacagtacctacagcctctcctccaccctgaccctgagtaaagccgactacgagaagcacaaggtgtacgcgtgc
gaagtcacccaccagggcctctcgtcccccgtgaccaagtccttcaaccggggcgagtgctga.

Further, Pelgifatamab comprises a heavy chain amino acid sequence according to SEQ ID NO. 3:
SEQ ID NO. 3 - Heavy Chain 
QVQLVESGGG VVQPGRSLRL SCAASGFAFS RYGMHWVRQA PGKGLEWVAV
IWYDGSNKYY ADSVKGRFTI SRDNSKNTQY LQMNSLRAED TAVYYCARGG
DFLYYYYYGM DVWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PG(K)
 and a light chain amino acid sequence according to SEQ ID NO. 4:
SEQ ID NO. 4 - Light Chain

DIQMTQSPSS LSASVGDRVT ITCRASQGIS NYLAWYQQKT GKVPKFLIYE
ASTLQSGVPS RFSGGGSGTD FTLTISSLQP EDVATYYCQN YNSAPFTFGP
GTKVDIKRTV AAPSVFIFPP SDEQLKSGTA SVVCLLNNFY PREAKVQWKV
DNALQSGNSQ ESVTEQDSKD STYSLSSTLT LSKADYEKHK VYACEVTHQG
LSSPVTKSFN RGEC

Further, Pelgifatamab comprises a heavy chain nucleotide sequence according to the sequence shown in Figure 1 and a light chain nucleotide sequence according to the sequence shown in Figure 2.

Further, Pelgifatamab comprises a heavy chain amino acid sequence according to the sequence shown in Figure 1 and a light chain amino acid sequence according to the sequence shown in Figure 2.

Further, Pelgifatamab comprises at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10:
Pelgifatamab CDR Heavy Chain Sequences
SEQ ID NO. 5: RYGMH
SEQ ID NO. 6: VIWYDGSNKYYADSVKG
SEQ ID NO. 7: GGDFLYYYYYGMDV
Pelgifatamab CDR Light Chain Sequences
SEQ ID NO. 8: RASQGISNYLA
SEQ ID NO. 9: EASTLQS
SEQ ID NO. 10: QNYNSAPFT

Further, Pelgifatamab comprises at least the three CDR heavy chain sequences and the three CDR light chain sequences according to the sequences shown in Figure 3.

J591 is a monoclonal antibody having binding affinity for the antigen PSMA (anti-PSMA antibody). Its preparation, isolation and purification has been described e.g., in WO2002/098897 and EP2277542.

Further, J591 comprises a heavy chain sequence according to SEQ ID NO. 11 and a light chain sequence according to SEQ ID NO. 12:
SEQ ID NO. 11: 
EVQLVQSGPE VKKPGATVKI SCKTSGYTFT EYTIHWVKQA PGKGLEWIGN INPNNGGTT
Y NQKFEDKATL TVDKSTDTAY MELSSLRSED TAVYYCAAGW NFDYWGQGTL LTVSSAS
TKG PSVFPLAPSS KSTSGGTAAL GCLVKDYFPE PVTVSWNSGA LTSGVHTFPA VLQSS
GLYSL SSVVTVPSSS LGTQTYICNV NHKPSNTKVD KKVEPKSCDK THTCPPCPAP ELLG
GPSVFL FPPKPKDTLM ISRTPEVTCV VVDVSHEDPE VKFNWYVDGV EVHNAKTKPR EE
QYNSTYRV VSVLTVLHQD WLNGKEYKCK VSNKALPAPI EKTISKAKGQ PREPQVYTLP P
 
SRDELTKNQ VSLTCLVKGF YPSDIAVEWE SNGQPENNYK TTPPVLDSDG SFFLYSKLTV
DKSRWQQGNV FSCSVMHEAL HNHYTQKSLS LSPG
SEQ ID NO. 12:

DIQMTQSPSS LSTSVGDRVT LTCKASQDVG TAVDWYQQKP GPSPKLLIYW ASTRHTGIP
S RFSGSGSGTD FTLTISSLQP EDFADYYCQQ YNSYPLTFGP GTKVDIKRTV AAPSVFIFP
P SDEQLKSGTA SVVCLLNNFY PREAKVQWKV DNALQSGNSQ ESVTEQDSKD STYSLSS
TLT LSKADYEKHK VYACEVTHQG LSSPVTKSFN RGEC

Further, J591 comprises at least the three CDR heavy chain sequences according to SEQ ID NO. 13, SEQ ID NO. 14 and SEQ ID NO. 15 and the three CDR light chain sequences according to SEQ ID NO. 16, SEQ ID NO. 17 and SEQ ID NO. 18:
J591CDR Heavy Chain Sequences
SEQ ID NO. 13: YTIH
SEQ ID NO. 14: N INPNNGGTTY NQKFED
SEQ ID NO. 15: GW NFDY
J591CDR Light Chain Sequences
SEQ ID NO. 16: KASQDVG TAVD
SEQ ID NO. 17: W ASTRHT
SEQ ID NO. 18: QQ YNSYPLT

TLX592 is a monoclonal antibody having binding affinity for the antigen PSMA (anti-PSMA antibody) developed by Telix Pharmaceuticals which has been described in e.g., WO2021/000018.

TLX592 comprises a heavy chain sequence according to SEQ ID NO. 19 and a light chain sequence according to SEQ ID NO. 20:
SEQ ID NO. 19: 
EVQLVQSGAE VKKPGASVKV SCKASGYTFT EYTIHWVRQA PGKGLEWIGN INPNNGGTT
Y NQKFEDRVTI TVDKSTSTAY MELSSLRSED TAVYYCAAGW NFDYWGQGTT VTVSSAS
TKG PSVFPLAPSS KSTSGGTAAL GCLVKDYFPE PVTVSWNSGA LTSGVHTFPA VLQSS
GLYSL SSVVTVPSSS LGTQTYICNV NHKPSNTKVD KRVEPKSCDK THTCPPCPAP ELLG
GPSVFL FPPKPKDTLM ISRTPEVTCV VVDVSHEDPE VKFNWYVDGV EVHNAKTKPR EE
 
QYNSTYRV VSVLTVLAQD WLNGKEYKCK VSNKALPAPI EKTISKAKGQ PREPQVYTLP P
SREEMTKNQ VSLTCLVKGF YPSDIAVEWE SNGQPENNYK TTPPVLDSDG SFFLYSKLTV
DKSRWQQGNV FSCSVMHEAL HNQYTQKSLS LSPGK
SEQ ID NO. 20:

DIQMTQSPST LSASVGDRVT ITCKASQDVG TAVDWYQQKP GQAPKLLIYW ASTRHTGVP
D RFSGSGSGTD FTLTISRLQP EDFAVYYCQQ YNSYPLTFGQ GTKVDIKRTV AAPSVFIFP
P SDEQLKSGTA SVVCLLNNFY PREAKVQWKV DNALQSGNSQ ESVTEQDSKD STYSLSS
TLT LSKADYEKHK VYACEVTHQG LSSPVTKSFN RGEC

Further, TLX592 comprises at least the three CDR heavy chain sequences according to SEQ ID NO. 21, SEQ ID NO. 22 and SEQ ID NO. 23 and the three CDR light chain sequences according to SEQ ID NO. 24, SEQ ID NO. 25 and SEQ ID NO. 26:
TLX592 CDR Heavy Chain Sequences
SEQ ID NO. 21: EYTIH
SEQ ID NO. 22: N INPNNGGTTY NQKFED
SEQ ID NO. 23: GW NFDY
TLX592 CDR Light Chain Sequences
SEQ ID NO. 24: KASQDVG TAVD
SEQ ID NO. 25: W ASTRHT
SEQ ID NO. 26: QQ YNSYPLT

### Combinations

In a further aspect, the present invention relates to combinations, preferably pharmaceutical combinations comprising a tissue-targeting compound of formula (I) as described above and a further pharmaceutical agent. Said combinations are useful in therapy, preferably in treating hyperproliferative diseases such as cancer.

Combinations comprising effective amounts of a tissue-targeting compound of formula (I) as described above and a further pharmaceutical agent achieve a greater therapeutic efficacy than when either compound is used alone.

The relative ratios of each compound in the combination can also be selected based on their respective mechanisms of action and the disease biology. The relative ratios of each compound can vary widely.

The release of one or more agents of the combination can also be controlled, where appropriate, to provide the desired therapeutic activity when in a single dosage form, combination pack, kit or when in separate independent dosage forms.

Generally, the combinations of the present invention will serve to:
(1) yield better efficacy in reducing the growth of a tumor or even eliminate the tumor as compared to administration of either agent alone,
(2) provide for the administration of lesser amounts of the administered targeted compounds and/or chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time until tumor progression, and/or
(8) yield efficacy and tolerability results at least as good as those of the agents used alone, compared to known instances where other cancer agent combinations produce inhibitory effects.

The tissue-targeting compound of formula (I) is a compound of the present invention as described herein.

The further pharmaceutical agent is selected from a non-steroidal antiandrogen, a steroidal antiandrogen, an androgen synthesis inhibitor, an antigonadotropin, a PARP inhibitor, an ATR inhibitor, an ATM inhibitor, a DNA-PK inhibitor, an AKT inhibitor, a Pi3K inhibitor, a PSMA-targeting beta emitter, an immune checkpoint inhibitor, an alpha emitter, a vaccine and a chemotherapeutic agent.

Preferably, the further pharmaceutical agent is selected from a non-steroidal antiandrogen, a steroidal antiandrogen, an androgen synthesis inhibitor, an antigonadotropin, a PARP inhibitor, an AKT inhibitor, a Pi3K inhibitor, a PSMA-targeting beta emitter, an immune checkpoint inhibitor, an alpha emitter, a vaccine and a chemotherapeutic agent.

More preferably, the further pharmaceutical agent is selected from a non-steroidal antiandrogen, a steroidal antiandrogen, an androgen synthesis inhibitor, an antigonadotropin, a PARP inhibitor, a PSMA-targeting beta emitter, an alpha emitter, a vaccine and a chemotherapeutic agent.

More preferably, the further pharmaceutical agent is selected from a non-steroidal antiandrogen, a steroidal antiandrogen, an androgen synthesis inhibitor, an antigonadotropin and a PARP inhibitor.

Even more preferably, the further pharmaceutical agent is selected from a non-steroidal antiandrogen, a steroidal antiandrogen, an androgen synthesis inhibitor, and an antigonadotropin.

Even more preferably, the further pharmaceutical agent is a non-steroidal antiandrogen.

If the further pharmaceutical agent is a non-steroidal antiandrogen, it is preferably selected from darolutamide, bicalutamide, enzalutamide, apalutamide, flutamide, nilutamide, topilutamide, proxalutamide or bavdegalutamide or any combination thereof. More preferably, the non-steroidal antiandrogen is selected from darolutamide and enzalutamide, most preferably the non-steroidal antiandrogen is darolutamide. In certain embodiments, the non-steroidal antiandrogen is enzalutamide. In certain embodiments, the non-steroidal antiandrogen is apalutamide. In further embodiments the non-steroidal antiandrogen is bicalutamide, flutamide or nilutamide. In other further embodiments the non-steroidal antiandrogen is ARV-766, EPI-7386, CC-94676, AC-0176, HP-518 or TAS-3681. Preferably, the non-steroidal antiandrogen is darolutamide.

If the further pharmaceutical agent is a steroidal antiandrogen, it is preferably selected from Cyproterone acetate, Allylestrenol, Chlormadinone acetate, Delmadinone acetate, Gestonorone caproate, Hydroxyprogesterone caproate, Medroxyprogesterone acetate, Megestrol acetate, Osaterone acetate, Oxendolone and Spironolactone. More preferably, the steroidal antiandrogen is Cyproterone acetate.

If the further pharmaceutical agent is an androgen synthesis inhibitor, it is preferably selected from ketoconazole, abiraterone, aminoglutethimide, goserelin and seviteronel. More preferably, the androgen synthesis inhibitor is arbiraterone or goserelin.

If the further pharmaceutical agent is an antigonadotropin, it is preferably selected from Abarelix, Danazol, Gestrinone, Paroxypropione, Cetrorelix, Degarelix, Elagolix, Ganirelix, Linzagolix and Relugolix. More preferably, the antigonadotropin is Degarelix or Relugolix.

If the further pharmaceutical agent is a PARP inhibitor, it is preferably selected from Olaparib, Rucaparib, Veliparib, Niraparib, Talazoparib, Pamiparib, CEP 9722, E7016 and Iniparib. More preferably, the PARP inhibitor is Olaparib or Rucaparib.

If the further pharmaceutical agent is an ATR inhibitor, it is preferably selected from Berzosertib, BAY1895344, AZD6738, M6620, AZ20 and VE 821. More preferably, the PARP inhibitor is Berzosertib or BAY1895344 (Elimusertib).

If the further pharmaceutical agent is an ATM inhibitor, it is preferably selected from AZD1390, KU-55933, KU-60019, KU-59403, CP-466722, AZ31/AZ32 and AZD0156.

If the further pharmaceutical agent is a DNA-PK inhibitor, it is preferably selected from ZD7648, Nedisertib, VX-984, CC-115, Samotolisib and BAY-8400. More preferably, the DNA-PK inhibitor is Nedisertib or BAY-8400.

If the further pharmaceutical agent is an AKT inhibitor, it is preferably selected from Ipatasertib, afuresertib, miransertib, capivasertib, uprosertib and MK2206. More preferably, the AKT inhibitor is Ipatasertib.

If the further pharmaceutical agent is a Pi3K inhibitor, it is preferably selected from Copanlisib, Buparlisib, Duvelisib, Idelalisib, Paxalisib, Zandelisib, Inavolisib, Duvelisib, Alpelisib and Umbralisib. More preferably, the Pi3K inhibitor is Copanlisib.

If the further pharmaceutical agent is a PSMA-targeting beta emitter, it is preferably (177Lu)-vipivotide tetraxetan (Pluvicto), 177Lu-PSMA-I&T and PNT2002.

If the further pharmaceutical agent is an immune checkpoint inhibitor, it is preferably selected from Atezolizumab, Durvalumab, Avelumab, Nivolumab, Pembrolizumab and Ipilimumab.

If the further pharmaceutical agent is an alpha emitter, it is preferably selected from 223Ra, J591-225Ac and PSMA-617-225Ac.

If the further pharmaceutical agent is a vaccine, it is preferably Sipoleucel T.

If the further pharmaceutical agent is a chemotherapeutic agent, it is preferably selected from Paclitaxel, Docetaxel, Ixabepilone, Vinorelbine, Nocodazole, Vincristine, Colchicine and Eribulin. More preferably, the chemotherapeutic agent is Paclitaxel or Docetaxel.

Thus, in one embodiment, the present invention relates to a combination comprising
(i) a tissue-targeting compound of formula (I) 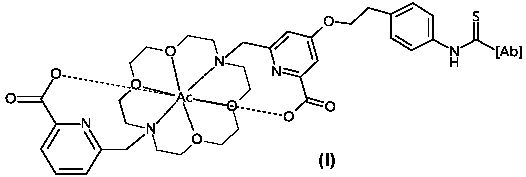 wherein [Ab] is a monoclonal antibody or an antigen-binding fragment thereof having binding affinity for the prostate-specific membrane antigen (PSMA), and
(ii) a pharmaceutical agent selected from a non-steroidal antiandrogen, a steroidal antiandrogen, an androgen synthesis inhibitor, an antigonadotropin, a PARP inhibitor, an ATR inhibitor, an ATM inhibitor, a DNA-PK inhibitor, an AKT inhibitor, a Pi3K inhibitor, a PSMA-targeting beta emitter, an immune checkpoint inhibitor, an alpha emitter, a vaccine and a chemotherapeutic agent.

In a further embodiment, the present invention relates to a combination comprising
(i) a tissue-targeting compound of formula (I) 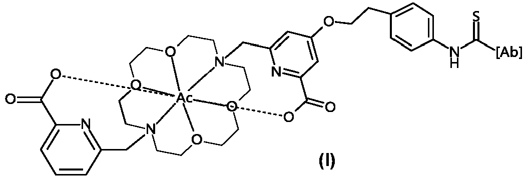 wherein [Ab] is the anti-PSMA monoclonal antibody Pelgifatamab or an antigen-binding fragment thereof, and
(ii) a pharmaceutical agent selected from a non-steroidal antiandrogen, a steroidal antiandrogen, an androgen synthesis inhibitor, an antigonadotropin, a PARP inhibitor, an ATR inhibitor, an ATM inhibitor, a DNA-PK inhibitor, an AKT inhibitor, a Pi3K inhibitor, a PSMA-targeting beta emitter, an immune checkpoint inhibitor, an alpha emitter, a vaccine and a chemotherapeutic agent.

In a further embodiment, the present invention relates to a combination comprising
(i) a tissue-targeting compound of formula (I) 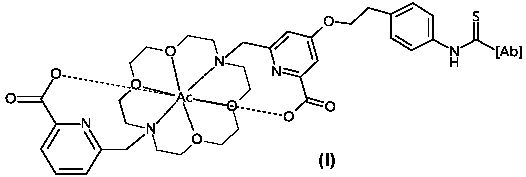 wherein [Ab] is the anti-PSMA monoclonal antibody Pelgifatamab or an antigen-binding fragment thereof, wherein Pelgifatamab or an antigen-binding fragment thereof is bound to the rest of the molecule via a terminal amino group of a Lysine residue of the monoclonal antibody or antigen-binding fragment thereof, and
(ii) a pharmaceutical agent selected from a non-steroidal antiandrogen, a steroidal antiandrogen, an androgen synthesis inhibitor, an antigonadotropin, a PARP inhibitor, an ATR inhibitor, an ATM inhibitor, a DNA-PK inhibitor, an AKT inhibitor, a Pi3K inhibitor, a PSMA-targeting beta emitter, an immune checkpoint inhibitor, an alpha emitter, a vaccine and a chemotherapeutic agent.

In a further embodiment, the present invention relates to a combination comprising
(i) a tissue-targeting compound of formula (I) 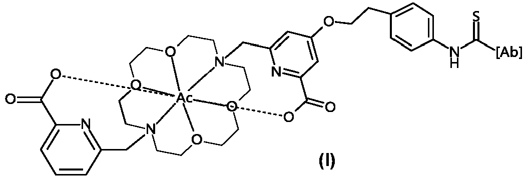 wherein [Ab] is the anti-PSMA monoclonal antibody Pelgifatamab or an antigen-binding fragment thereof comprising a heavy chain sequence according to SEQ ID NO. 1, and a light chain sequence according to SEQ ID NO. 2, and
(ii) a pharmaceutical agent selected from a non-steroidal antiandrogen, a steroidal antiandrogen, an androgen synthesis inhibitor, an antigonadotropin, a PARP inhibitor, an ATR inhibitor, an ATM inhibitor, a DNA-PK inhibitor, an AKT inhibitor, a Pi3K inhibitor, a PSMA-targeting beta emitter, an immune checkpoint inhibitor, an alpha emitter, a vaccine and a chemotherapeutic agent.

In a further embodiment, the present invention relates to a combination comprising
(i) a tissue-targeting compound of formula (I) 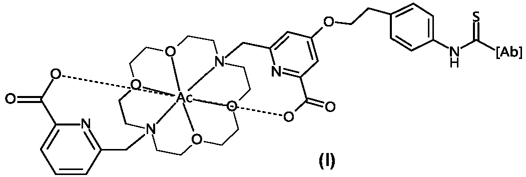 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, and
(ii) a pharmaceutical agent selected from a non-steroidal antiandrogen, a steroidal antiandrogen, an androgen synthesis inhibitor, an antigonadotropin, a PARP inhibitor, an ATR inhibitor, an ATM inhibitor, a DNA-PK inhibitor, an AKT inhibitor, a Pi3K inhibitor, a PSMA-targeting beta emitter, an immune checkpoint inhibitor, an alpha emitter, a vaccine and a chemotherapeutic agent.

In a further embodiment, the present invention relates to a combination comprising
(i) a tissue-targeting compound of formula (I) 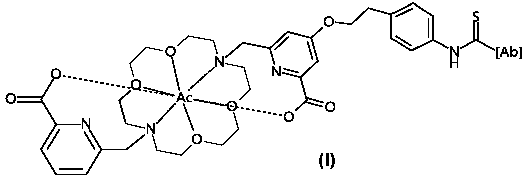 wherein [Ab] is the anti-PSMA monoclonal antibody Pelgifatamab or an antigen-binding fragment thereof comprising a heavy chain amino acid sequence according to SEQ ID NO. 3, and a light chain amino acid sequence according to SEQ ID NO. 4, and
(ii) a pharmaceutical agent selected from a non-steroidal antiandrogen, a steroidal antiandrogen, an androgen synthesis inhibitor, an antigonadotropin, a PARP inhibitor, an ATR inhibitor, an ATM inhibitor, a DNA-PK inhibitor, an AKT inhibitor, a Pi3K inhibitor, a PSMA-targeting beta emitter, an immune checkpoint inhibitor, an alpha emitter, a vaccine and a chemotherapeutic agent.

In a further embodiment, the present invention relates to a combination comprising
(i) a tissue-targeting compound of formula (I) 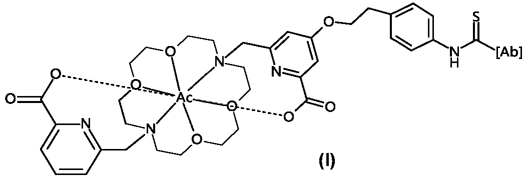 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, and
(ii) a pharmaceutical agent selected from a non-steroidal antiandrogen, a steroidal antiandrogen, an androgen synthesis inhibitor, an antigonadotropin, a PARP inhibitor, a PSMA-targeting beta emitter, an alpha emitter, a vaccine and a chemotherapeutic agent.

In a further embodiment, the present invention relates to a combination comprising
(i) a tissue-targeting compound of formula (I) 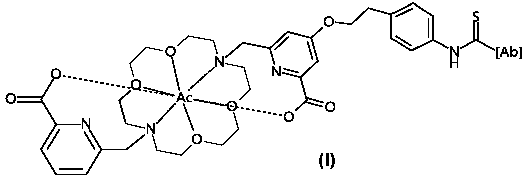 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, and
(ii) a pharmaceutical agent selected from a non-steroidal antiandrogen, a steroidal antiandrogen, an androgen synthesis inhibitor, an antigonadotropin and a PARP inhibitor.

In a further embodiment, the present invention relates to a combination comprising
(i) a tissue-targeting compound of formula (I) 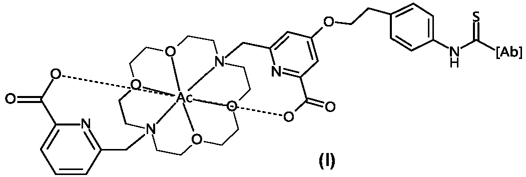 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, and
(ii) a pharmaceutical agent selected from a non-steroidal antiandrogen, a steroidal antiandrogen, an androgen synthesis inhibitor, and an antigonadotropin.

In a further embodiment, the present invention relates to a combination comprising
(i) a tissue-targeting compound of formula (I) 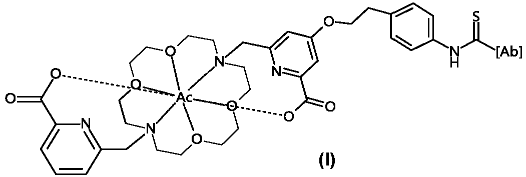 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, and
(ii) a non-steroidal antiandrogen.

In a further embodiment, the present invention relates to a combination comprising
(i) a tissue-targeting compound of formula (I) 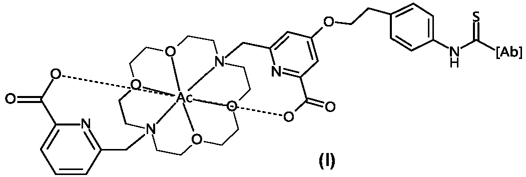 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, and
(ii) a pharmaceutical agent selected from a non-steroidal antiandrogen selected from the group consisting of darolutamide, bicalutamide, enzalutamide, apalutamide, flutamide, nilutamide and topilutamide, a steroidal antiandrogen selected from the group consisting of Cyproterone acetate, Allylestrenol, Chlormadinone acetate, Delmadinone acetate, Gestonorone caproate, Hydroxyprogesterone caproate, Medroxyprogesterone acetate, Megestrol acetate, Osaterone acetate, Oxendolone and Spironolactone, an androgen synthesis inhibitor selected from the group consisting of ketoconazole, abiraterone, aminoglutethimide, goserelin and seviteronel, an antigonadotropin selected from the group consisting of Abarelix, Danazol, Gestrinone, Paroxypropione, Cetrorelix, Degarelix, Elagolix, Ganirelix, Linzagolix and Relugolix, a PARP inhibitor selected from the group consisting of Olaparib, Rucaparib, Veliparib, Niraparib, Talazoparib, Pamiparib, CEP 9722, E7016 and Iniparib, the PSMA-targeting beta emitter (177Lu)-vipivotide tetraxetan (Pluvicto), an alpha emitter selected from the group consisting of 223Ra, J591-225Ac and PSMA-617-225Ac, the vaccine Sipoleucel T and a chemotherapeutic agent selected from the group consisting of Paclitaxel, Docetaxel, Ixabepilone, Vinorelbine, Nocodazole, Vincristine, Colchicine and Eribulin.

In a further embodiment, the present invention relates to a combination comprising
(i) a tissue-targeting compound of formula (I) 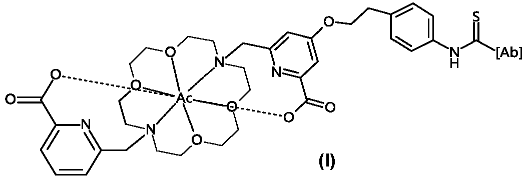 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, and
(ii) a pharmaceutical agent selected from a non-steroidal antiandrogen selected from the group consisting of darolutamide, bicalutamide, enzalutamide, apalutamide, flutamide, nilutamide and topilutamide, a steroidal antiandrogen selected from the group consisting of Cyproterone acetate, Allylestrenol, Chlormadinone acetate, Delmadinone acetate, Gestonorone caproate, Hydroxyprogesterone caproate, Medroxyprogesterone acetate, Megestrol acetate, Osaterone acetate, Oxendolone and Spironolactone, an androgen synthesis inhibitor selected from the group consisting of ketoconazole, abiraterone, aminoglutethimide, goserelin and seviteronel, an antigonadotropin selected from the group consisting of Abarelix, Danazol, Gestrinone, Paroxypropione, Cetrorelix, Degarelix, Elagolix, Ganirelix, Linzagolix and Relugolix and a PARP inhibitor selected from the group consisting of Olaparib, Rucaparib, Veliparib, Niraparib, Talazoparib, Pamiparib, CEP 9722, E7016 and Iniparib.

In a further embodiment, the present invention relates to a combination comprising
(i) a tissue-targeting compound of formula (I) 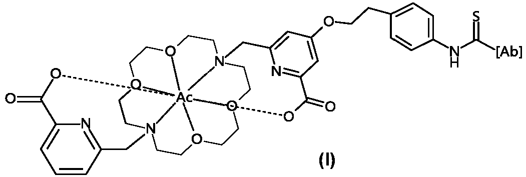 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, and
(ii) a pharmaceutical agent selected from a non-steroidal antiandrogen selected from the group consisting of darolutamide, bicalutamide, enzalutamide, apalutamide, flutamide, nilutamide and topilutamide, a steroidal antiandrogen selected from the group consisting of Cyproterone acetate, Allylestrenol, Chlormadinone acetate, Delmadinone acetate, Gestonorone caproate, Hydroxyprogesterone caproate, Medroxyprogesterone acetate, Megestrol acetate, Osaterone acetate, Oxendolone and Spironolactone, an androgen synthesis inhibitor selected from the group consisting of ketoconazole, abiraterone, aminoglutethimide, goserelin and seviteronel and an antigonadotropin selected from the group consisting of Abarelix, Danazol, Gestrinone, Paroxypropione, Cetrorelix, Degarelix, Elagolix, Ganirelix, Linzagolix and Relugolix.

In a further embodiment, the present invention relates to a combination comprising
(i) a tissue-targeting compound of formula (I) 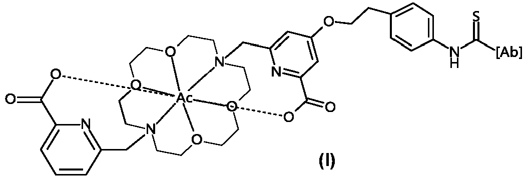 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, and
(ii) a pharmaceutical agent selected from the non-steroidal antiandrogen darolutamide, the steroidal antiandrogen Cyproterone acetate, the androgen synthesis inhibitor abiraterone or goserelin and the antigonadotropin Degarelix or Relugolix.

### Synthesis

In a further aspect, the present invention relates to a process for the preparation of a tissue-targeting compound of formula (I) 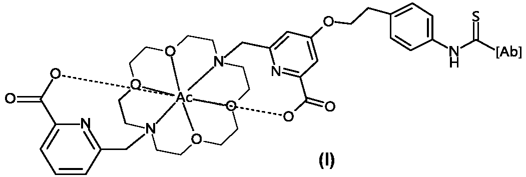
wherein [Ab] is a monoclonal antibody or an antigen-binding fragment thereof having binding affinity for PSMA,
said method comprising
   (i) coupling a chelating moiety of formula (II) 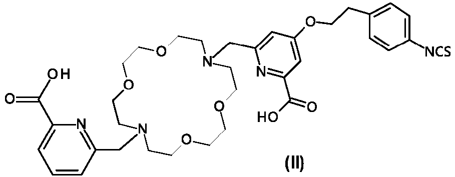 with a monoclonal antibody or an antigen-binding fragment thereof having binding affinity for PSMA, and
   (ii) contacting the resulting product with an aqueous solution comprising an ion of at least one alpha-emitting actinium isotope.

In a further embodiment, the present invention relates to a process for the preparation of a tissue-targeting compound of formula (I) 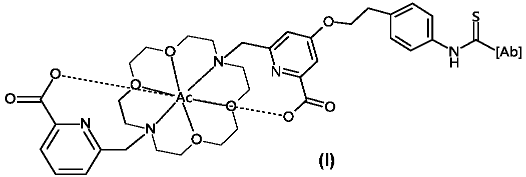
wherein [Ab] is a monoclonal antibody or an antigen-binding fragment thereof having binding affinity for PSMA,
said method comprising
   (i) coupling a chelating moiety of formula (II) 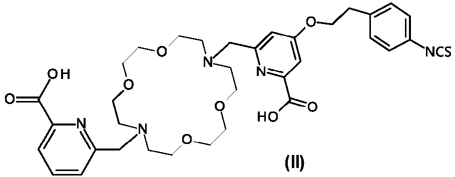 with a monoclonal antibody or an antigen-binding fragment thereof having binding affinity for PSMA via a Lysine residue of the monoclonal antibody or antigen-binding fragment thereof, and
   (ii) contacting the resulting product with an aqueous solution comprising an ion of at least one alpha-emitting actinium isotope.

In a further embodiment, the present invention relates to a process for the preparation of a tissue-targeting compound of formula (I) 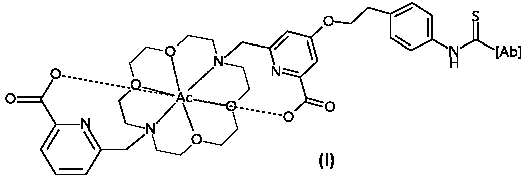 wherein [Ab] is an anti-PSMA antibody selected from Pelgifatamab, J591 and TLX592 or an antigen-binding fragment thereof, said method comprising
(i) coupling a chelating moiety of formula (II) 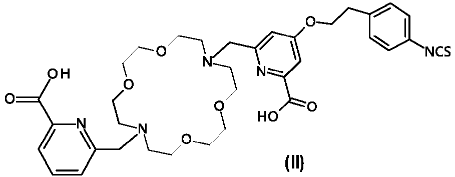 with an anti-PSMA antibody selected from Pelgifatamab, J591 and TLX592 or an antigen-binding fragment thereof via a Lysine residue of the anti-PSMA antibody or antigen-binding fragment thereof, and
(ii) contacting the resulting product with an aqueous solution comprising an ion of at least one alpha-emitting actinium isotope.

In a further embodiment, the present invention relates to a process for the preparation of a tissue-targeting compound of formula (I) 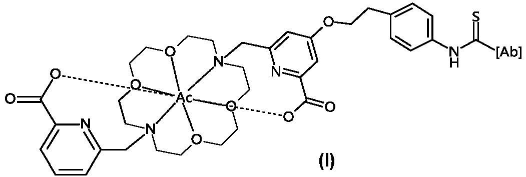 wherein [Ab] is the anti-PSMA antibody Pelgifatamab or an antigen-binding fragment thereof, said method comprising
(i) coupling a chelating moiety of formula (II) 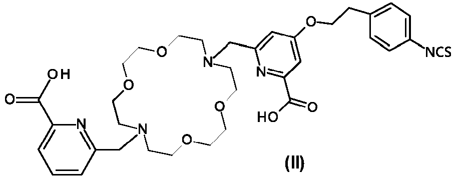 with the anti-PSMA antibody Pelgifatamab or an antigen-binding fragment thereof via a Lysine residue of the monoclonal antibody or antigen-binding fragment thereof, and
(ii) contacting the resulting product with an aqueous solution comprising an ion of at least one alpha-emitting actinium isotope.

In step (i), a chelating moiety of formula (II) 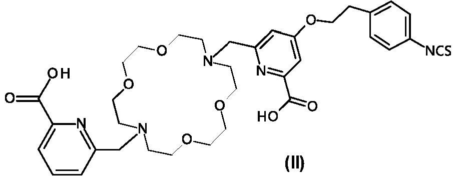 is coupled with a monoclonal antibody or an antigen-binding fragment thereof having binding affinity for PSMA. Preferably, the anti-PSMA antibody is selected from Pelgifatamab, J591 and TLX592 or an antigen-binding fragment thereof. More preferably, the anti-PSMA antibody is Pelgifatamab or an antigen-binding fragment thereof.

Preferably, the chelating moiety of formula (II) is coupled to the anti-PSMA antibody or an antigen-binding fragment thereof via a Lysine residue of the monoclonal antibody, thus forming a thiourea moiety that links the chelating moiety with the anti-PSMA antibody or antigen-binding fragment thereof.

Preferably, the coupling in step (i) is carried out under basic pH, i.e., at a pH higher than 7. More preferably, the coupling in step (i) is carried out at a pH of from 8 to 10, most preferably at a pH of 9.

After step (i), a tissue-targeting chelator of formula (III) is generated. Thus, in further embodiments, the present invention relates to a tissue-targeting chelator of formula (III) 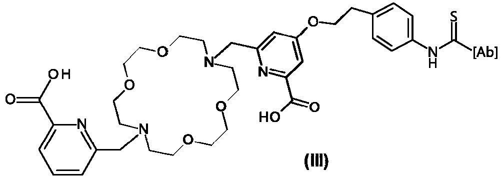 wherein [Ab] is a monoclonal antibody or an antigen-binding fragment thereof having binding affinity for PSMA.

Preferably, the anti-PSMA antibody or antigen-binding fragment thereof is selected from Pelgifatamab, J591 and TLX592 or an antigen-binding fragment thereof. More preferably, the anti-PSMA antibody is Pelgifatamab or an antigen-binding fragment thereof.

In step (ii), the product resulting from the coupling in step (i) is contacted with an aqueous solution comprising an ion of at least one alpha-emitting actinium isotope. Preferably, the alpha-emitting actinium isotope is 225Ac. Preferably, the ion of at least one alpha-emitting actinium isotope is ²²⁵Ac having a triple positive charge, i.e., ²²⁵Ac³⁺. The present invention allows for the execution of step (ii) at room temperature, i.e., at a temperature that is lower than the one required for the preparation of the corresponding DOTA conjugates (i.e. Ac225-DOTA-Pelgifatamab), which require higher temperatures (60°C).

### Uses

The references to methods of treatment in this section are to be interpreted as references to the compounds or combinations of the present invention for use in a method for treatment of the human (or animal) body by therapy.

In a further aspect, the tissue-targeting compounds of formula (I) of the present invention and/or the combinations comprising tissue-targeting compounds of formula (I) and a further pharmaceutical agent of the present invention may be used for the treatment of disease in a human or in a non-human animal subject. In particular, the tissue-targeting compounds of formula (I) of the present invention and/or the combinations comprising tissue-targeting compounds of formula (I) and a further pharmaceutical agent of the present invention may be used for the treatment of hyperplastic and/or neoplastic diseases, such as cancer. There are a number of targets which are known to be associated with hyperplastic and neoplastic disease. These include certain receptors, cell surface proteins, transmembrane proteins and proteins/peptides found in the extracellular matrix in the vicinity of diseased cells. Examples of cell-surface receptors and antigens which may be associated with neoplastic disease include but are not limited to CD22, CD33, FGFR2 (CD332), PSMA, HER2, GPC3 and Mesothelin. In one preferred embodiment, the tissue-targeting compounds of formula (I) of the present invention and/or the combinations comprising tissue-targeting compounds of formula (I) of the present invention comprise a tissue-targeting moiety comprising a monoclonal antibody having specificity and/or binding affinity for PSMA. This may be reflected, for example by having 50 or more times greater binding affinity for cells expressing PSMA than for non-PSMA expressing cells (preferably at least 100 time greater, more preferably at least 300 times greater). It is believed that PSMA is expressed and/or over-expressed in cells having certain disease states (as indicated herein) and thus the PSMA specific binder may serve to target the complex to such disease-affected cells. Similarly, a tissue targeting moiety may bind to cell-surface markers (e.g. PSMA receptors) present on cells in the vicinity of disease affected cells. PSMA cell-surface markers may be more heavily expressed on diseased cell surfaces than on healthy cell surfaces or more heavily expressed on cell surfaces during periods of growth or replication than during dormant phases. In one embodiment, a PSMA-specific tissue-targeting binder may be used in combination with another binder for a disease-specific cell-surface marker, thus giving a dual- binding complex. Tissue-targeting binders for PSMA will typically be peptides or proteins, as discussed herein.

The various aspects of the invention as described herein relate to treatment of disease, particularly for the selective targeting of diseased tissue, as well as relating to complexes, conjugates, medicaments, formulation, kits etc. useful in such methods. In all aspects, the diseased tissue may reside at a single site in the body (for example in the case of a localized solid tumor) or may reside at a plurality of sites (for example where several joints are affected in arthritis or in the case of a distributed or metastasized cancerous disease).

The diseased tissue to be targeted may be at a soft tissue site, at a calcified tissue site or a plurality of sites which may all be in soft tissue, all in calcified tissue or may include at least one soft tissue site and/or at least one calcified tissue site. In one embodiment, at least one soft tissue site is targeted. The sites of targeting and the sites of origin of the disease may be the same, but alternatively may be different (such as where metastatic sites are specifically targeted). Where more than one site is involved, this may include the site of origin or may be a plurality of secondary sites.

The term "soft tissue" is used herein to indicate tissues which do not have a "hard" mineralized matrix. In particular, soft tissues as used herein may be any tissues that are not skeletal tissues. Correspondingly, "soft tissue disease" as used herein indicates a disease occurring in a "soft tissue" as used herein. The invention is particularly suitable for the treatment of cancers and "soft tissue disease" thus encompasses carcinomas, sarcomas, myelomas, leukemias, lymphomas and mixed type cancers occurring in any "soft" (i.e. non-mineralized) tissue, as well as other non-cancerous diseases of such tissue. Cancerous "soft tissue disease" includes solid tumors occurring in soft tissues as well as metastatic and micro-metastatic tumors. Indeed, the soft tissue disease may comprise a primary solid tumor of soft tissue and at least one metastatic tumor of soft tissue in the same patient. Alternatively, the "soft tissue disease" may consist of only a primary tumor or only metastases with the primary tumor being a skeletal disease. Particularly suitable for treatment and/or targeting in all appropriate aspects of the invention are hematological neoplasms and especially neoplastic diseases of lymphoid cells, such as lymphomas and lymphoid leukemias, including Non-Hodgkin's Lymphoma, B-cell neoplasms of B-cell lymphomas. Similarly, any neoplastic diseases of bone marrow, spine (especially spinal cord) lymph nodes and/or blood cells are suitable for treatment and/or targeting in all appropriate aspects of the invention.

Some examples of B-cell diseases (e.g. neoplasms) that are suitable for treatment and/or targeting in appropriate aspects of the present invention include:
Chronic lymphocytic leukemia/Small lymphocytic lymphoma, B-cell prolymphocytic leukemia, Lymphoplasmacytic lymphoma (such as Waldenström macroglobulinemia), Splenic marginal zone lymphoma, Plasma cell neoplasms (e.g. Plasma cell myeloma, Plasmacytoma, Monoclonal immunoglobulin deposition diseases, Heavy chain diseases), Extranodal marginal zone B cell lymphoma (MALT lymphoma), Nodal marginal zone B cell lymphoma (NMZL), Follicular lymphoma, Mantle cell lymphoma, Diffuse large B cell lymphoma, Mediastinal (thymic) large B cell lymphoma, Intravascular large B cell lymphoma, Primary effusion lymphoma and Burkitt lymphoma/leukemia.

The tissue-targeting compounds of formula (I) of the present invention and/or the combinations comprising tissue-targeting compounds of formula (I) and a further pharmaceutical agent of the present invention preferably comprise a tissue-targeting moiety comprising a monoclonal antibody having binding affinity for the prostate specific membrane antigen (PSMA). PSMA expression in tumor cells has been observed in different types of diseases, in particular hyperproliferative diseases such as cancer (Uijen et al., Eur. J. Nucl. Med. Mol. Imaging 2021, 48, 4350, doi: 10.1007/s00259-021-05433-w). Examples of diseases and/or neoplasms suitable for treatment using the preferred tissue-targeting compounds of formula (I) of the present invention and/or the combinations comprising tissue-targeting compounds of formula (I) of the present invention include prostate cancer, salivary gland cancer, glioblastoma, brain cancer, thyroid cancer, hepatocellular carcinoma and clear cell renal carcinoma.

In some embodiments, the present invention includes a method of using a tissue-targeting compound of formula (I) and/or a combination comprising a tissue-targeting compound of formula (I) and a further pharmaceutical agent of the present invention for the treatment of diseases, more preferably hyperproliferative diseases, more particularly wherein the hyperproliferative disease is cancer, and yet even more particularly wherein the cancer disease is prostate cancer. In some embodiments, the present invention includes a method of using a tissue-targeting compound of formula (I) and/or a combination comprising a tissue-targeting compound of formula (I) and a further pharmaceutical agent of the present invention for the treatment of diseases, preferably hyperproliferative diseases, more preferably cancer, characterized by an overexpression of PSMA in the tumor cells.

In some embodiments, the present invention includes a method of treating a hyperproliferative disease, more particularly cancer, more particularly prostate cancer, said method comprising administering an effective amount of at least one tissue-targeting compound of formula (I) and/or a combination comprising a tissue-targeting compound of formula (I) and a further pharmaceutical agent of the present invention to a subject in need thereof. In some embodiments, the present invention includes a method of treating a hyperproliferative disease characterized by an overexpression of PSMA in the tumor cells, more particularly wherein the hyperproliferative disease is cancer, more particularly prostate cancer, said method comprising administering an effective amount of at least one tissue-targeting compound of formula (I) and/or a combination comprising a tissue-targeting compound of formula (I) and a further pharmaceutical agent of the present invention to a subject in need thereof.

In some embodiments, the present invention provides a tissue-targeting compound of formula (I), as described *supra,* and/or a combination comprising a tissue-targeting compound of formula (I) and a further pharmaceutical agent of the present invention as described *supra,* for use in the treatment and/or prophylaxis of diseases, in particular hyperproliferative disorders, more particularly cancer, even more particularly wherein the cancer disease is prostate cancer. In some embodiments, the present invention includes a tissue-targeting compound of formula (I), as described *supra,* and/or a combination comprising a tissue-targeting compound of formula (I) and a further pharmaceutical agent of the present invention as described *supra,* for use in the treatment and/or prophylaxis of hyperproliferative diseases characterized by an overexpression of PSMA in the tumor cells.

In some embodiments, the present invention includes a tissue-targeting compound of formula (I) and/or a combination comprising a tissue-targeting compound of formula (I) and a further pharmaceutical agent of the present invention for use in a method of inhibiting proliferation of a cell and/or the induction of apoptosis in a cell, comprising contacting the cell with a tissue-targeting compound of formula (I) and/or with a combination comprising a tissue-targeting compound of formula (I) and a further pharmaceutical agent. In some embodiments, the present invention includes a tissue-targeting compound of formula (I) and/or a combination comprising a tissue-targeting compound of formula (I) and a further pharmaceutical agent of the present invention for use in a method of inhibiting proliferation of tumor cells and/or the induction of apoptosis in tumor cells, comprising contacting the tumor cell with a tissue-targeting compound of formula (I) and/or with a combination comprising a tissue-targeting compound of formula (I) and a further pharmaceutical agent, wherein PSMA is overexpressed in the tumor cells.

In some embodiments, the present invention includes a tissue-targeting compound of formula (I) and/or a combination comprising a tissue-targeting compound of formula (I) and a further pharmaceutical agent for use in a method of treating a hyperproliferative disease, more particularly wherein the hyperproliferative disease is cancer, and yet even more particularly wherein the cancer disease is prostate cancer. In some embodiments, the present invention includes a tissue-targeting compound of formula (I) and/or a combination comprising a tissue-targeting compound of formula (I) and a further pharmaceutical agent for use in a method of treating a hyperproliferative disease characterized by an overexpression of PSMA in the tumor cells.

In some embodiments, the present invention includes the use of a tissue-targeting compound of formula (I) and/or a combination comprising a tissue-targeting compound of formula (I) and a further pharmaceutical agent for the manufacture of a medicament for the treatment and/or prophylaxis of a hyperproliferative disease. In some embodiments, the present invention includes the use of a tissue-targeting compound of formula (I) and/or a combination comprising a tissue-targeting compound of formula (I) and a further pharmaceutical agent for the manufacture of a medicament for the treatment and/or prophylaxis of a hyperproliferative disease characterized by an overexpression of PSMA in the tumor cells.

In some embodiments, the present invention includes the use of a tissue-targeting compound of formula (I) and/or a combination comprising a tissue-targeting compound of formula (I) and a further pharmaceutical agent for the manufacture of a medicament for the treatment of a hyperproliferative disease, particularly cancer and more particularly prostate cancer.

### Administration

In the combinations comprising a tissue-targeting compound of formula (I) and a further pharmaceutical agent, the tissue-targeting compound of formula (I) and the further pharmaceutical agent may be administered sequentially in either order, or simultaneously.

The tissue-targeting compounds of formula (I) or the combinations of the present invention comprising a tissue-targeting compound of formula (I) and a further pharmaceutical agent can be administered in any form by any effective route, including, e.g., oral, parenteral, enteral, intravenous, intraperitoneal, topical, transdermal (e.g., using any standard patch), ophthalmic, nasally, local, non-oral, such as aerosol, inhalation, subcutaneous, intramuscular, buccal, sublingual, rectal, vaginal, intra-arterial, and intrathecal, etc. They can be administered alone, or in combination with any ingredient(s), active or inactive. Preferably, the tissue-targeting compounds of formula (I) or the combinations of the present invention are administered intravenously.

The tissue-targeting compounds of formula (I) or the combinations of the present invention comprising a tissue-targeting compound of formula (I) and a further pharmaceutical agent can be converted in a known manner into the usual pharmaceutical formulations, which may be liquid or solid formulations e.g., without limitation normal and enteric coated tablets, capsules, pills, powders, granules, elixirs, tinctures, solutions, suspensions, syrups, solid and liquid aerosols and emulsions.

The relative ratios of each compound in the combinations of the present invention comprising a tissue-targeting compound of formula (I) and a further pharmaceutical agent can also be selected based on their respective mechanisms of action and the disease biology. The relative ratios of each compound can vary widely.

The release of one or more agents of the combinations of the present invention comprising a tissue-targeting compound of formula (I) and a further pharmaceutical agent can also be controlled, where appropriate, to provide the desired therapeutic activity when in a single dosage form, combination pack, kit or when in separate independent dosage forms.

The present invention includes pharmaceutical compositions which are comprised of a pharmaceutically acceptable carrier and a pharmaceutically effective amount of the tissue-targeting compounds of formula (I) or the combinations of the present invention i.e., a tissue-targeting compound of formula (I) as described above and a further pharmaceutical agent. A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated.

For oral administration, the tissue-targeting compounds of formula (I) or the combinations of the present invention comprising a tissue-targeting compound of formula (I) and a further pharmaceutical agent can be formulated into solid or liquid preparations such as solid dispersion, capsules, pills, tablets, troches, lozenges, melts, powders, solutions, suspensions, or emulsions, and may be prepared according to methods known to the art for the manufacture of pharmaceutical compositions. The solid unit dosage forms can be a capsule which can be of the ordinary hard- or soft-shelled gelatin type containing, for example, surfactants, lubricants, and inert fillers such as lactose, sucrose, calcium phosphate, and corn starch.

If administered in the form of a tablet, the tissue-targeting compounds of formula (I) or the combinations of the present invention comprising a tissue-targeting compound of formula (I) and a further pharmaceutical agent may be tableted with conventional tablet bases such as lactose, sucrose and cornstarch in combination with binders such as acacia, corn starch or gelatin, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance, tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavoring and coloring agents described above, may also be present.

The tissue-targeting compounds of formula (I) or the combinations of the present invention comprising a tissue-targeting compound of formula (I) and a further pharmaceutical agent may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soybean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more coloring agents; one or more flavoring agents; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavoring and coloring agents.

The tissue-targeting compounds of formula (I) or the combinations of the present invention comprising a tissue-targeting compound of formula (I) and a further pharmaceutical agent may also be administered parenterally, that is, subcutaneously, intravenously, intraocularly, intrasynovially, intramuscularly, or intraperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Preferably, the tissue-targeting compounds of formula (I) or the combinations of the present invention comprising a tissue-targeting compound of formula (I) and a further pharmaceutical agent are administered intravenously and/or orally, either together or separately. Preferably, in the combinations of the present invention comprising a tissue-targeting compound of formula (I) and a further pharmaceutical agent, the tissue-targeting compound of formula (I) is administered intravenously, and the further pharmaceutical agent is administered orally.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The tissue-targeting compounds of formula (I) or the combinations of the present invention comprising a tissue-targeting compound of formula (I) and a further pharmaceutical agent may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

The tissue-targeting compounds of formula (I) or the combinations of the present invention comprising a tissue-targeting compound of formula (I) and a further pharmaceutical agent may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such material is, for example, cocoa butter and polyethylene glycol.

Controlled release formulations for parenteral administration include liposomal, polymeric microsphere and polymeric gel formulations which are known in the art.

The tissue-targeting compounds of formula (I) or the combinations of the present invention comprising a tissue-targeting compound of formula (I) and a further pharmaceutical agent may also be in the form of a solid dispersion. The solid dispersion may be a solid solution, glass solution, glass suspension, amorphous precipitation in a crystalline carrier, eutectic or monotectic, compound or complex formation and combinations thereof.

The tissue-targeting compounds of formula (I) or the combinations of the present invention comprising a tissue-targeting compound of formula (I) and a further pharmaceutical agent can also contain other conventional pharmaceutically acceptable compounding ingredients, generally referred to as carriers or diluents, as necessary or desired. Conventional procedures for preparing such compositions in appropriate dosage forms can be utilized.

Commonly used pharmaceutical ingredients which can be used as appropriate to formulate the composition for its intended route of administration include:
- acidifying agents (examples include but are not limited to acetic acid, citric acid, fumaric acid, hydrochloric acid, nitric acid);
- alkalinizing agents (examples include but are not limited to ammonia solution, ammonium carbonate, diethanolamine, monoethanolamine, potassium hydroxide, sodium borate, sodium carbonate, sodium hydroxide, triethanolamine, trolamine);
- adsorbents (examples include but are not limited to powdered cellulose and activated charcoal);
- aerosol propellants (examples include but are not limited to carbon dioxide, CCl2F2, F2CIC-CCIF2 and CCIF3)
- air displacement agents (examples include but are not limited to nitrogen and argon);
- antifungal preservatives (examples include but are not limited to benzoic acid, butylparaben, ethylparaben, methylparaben, propylparaben, sodium benzoate);
- antimicrobial preservatives (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);
- antioxidants (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);
- binding materials (examples include but are not limited to block polymers, natural and synthetic rubber, polyacrylates, polyurethanes, silicones, polysiloxanes and styrenebutadiene copolymers);
- buffering agents (examples include but are not limited to potassium metaphosphate, dipotassium phosphate, sodium acetate, sodium citrate anhydrous and sodium citrate dihydrate)
- carrying agents (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection)
- chelating agents (examples include but are not limited to edetate disodium and edetic acid)
- colorants (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
- clarifying agents (examples include but are not limited to bentonite);
- emulsifying agents (examples include but are not limited to acacia, cetomacrogol, cetyl alcohol, glyceryl monostearate, lecithin, sorbitan monooleate, polyoxyethylene 50 monostearate);
- encapsulating agents (examples include but are not limited to gelatin and cellulose acetate phthalate)
- flavorants (examples include but are not limited to anise oil, cinnamon oil, cocoa, menthol, orange oil, peppermint oil and vanillin);
- humectants (examples include but are not limited to glycerol, propylene glycol and sorbitol);
- levigating agents (examples include but are not limited to mineral oil and glycerin);
- oils (examples include but are not limited to arachis oil, mineral oil, olive oil, peanut oil, sesame oil and vegetable oil);
- ointment bases (examples include but are not limited to lanolin, hydrophilic ointment, polyethylene glycol ointment, petrolatum, hydrophilic petrolatum, white ointment, yellow ointment, and rose water ointment);
- penetration enhancers (transdermal delivery) (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas)
- plasticizers (examples include but are not limited to diethyl phthalate and glycerol);
- scavenging compounds which reduce and/or prevent radiolysis;
- solvents (examples include but are not limited to ethanol, corn oil, cottonseed oil, glycerol, isopropanol, mineral oil, oleic acid, peanut oil, purified water, water for injection, sterile water for injection and sterile water for irrigation);
- stiffening agents (examples include but are not limited to cetyl alcohol, cetyl esters wax, microcrystalline wax, paraffin, stearyl alcohol, white wax and yellow wax);
- suppository bases (examples include but are not limited to cocoa butter and polyethylene glycols (mixtures));
- surfactants (examples include but are not limited to benzalkonium chloride, nonoxynol 10, oxtoxynol 9, polysorbate 80, sodium lauryl sulfate and sorbitan mono-palmitate);
- suspending agents (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
- sweetening agents (examples include but are not limited to aspartame, dextrose, glycerol, mannitol, propylene glycol, saccharin sodium, sorbitol and sucrose);
- tablet anti-adherents (examples include but are not limited to magnesium stearate and talc);
- tablet binders (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
- tablet and capsule diluents (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
- tablet coating agents (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
- tablet direct compression excipients (examples include but are not limited to dibasic calcium phosphate);
- tablet disintegrants (examples include but are not limited to alginic acid, carboxymethylcellulose calcium, microcrystalline cellulose, polacrillin potassium, cross-linked polyvinylpyrrolidone, sodium alginate, sodium starch glycollate and starch);
- tablet glidants (examples include but are not limited to colloidal silica, corn starch and talc);
- tablet lubricants (examples include but are not limited to calcium stearate, magnesium stearate, mineral oil, stearic acid and zinc stearate);
- tablet/capsule opaquants (examples include but are not limited to titanium dioxide);
- tablet polishing agents (examples include but are not limited to carnauba wax and white wax);
- thickening agents (examples include but are not limited to beeswax, cetyl alcohol and paraffin);
- tonicity agents (examples include but are not limited to dextrose and sodium chloride);
- viscosity increasing agents (examples include but are not limited to alginic acid, bentonite, carbomers, carboxymethylcellulose sodium, methylcellulose, polyvinyl pyrrolidone, sodium alginate and tragacanth); and
- wetting agents (examples include but are not limited to heptadecaethylene oxycetanol, lecithin, sorbitol monooleate, polyoxyethylene sorbitol monooleate, and polyoxyethylene stearate).

One skilled in the art, utilizing the preceding information, can utilize the present invention to its fullest extent.

### DESCRIPTION OF THE FIGURES

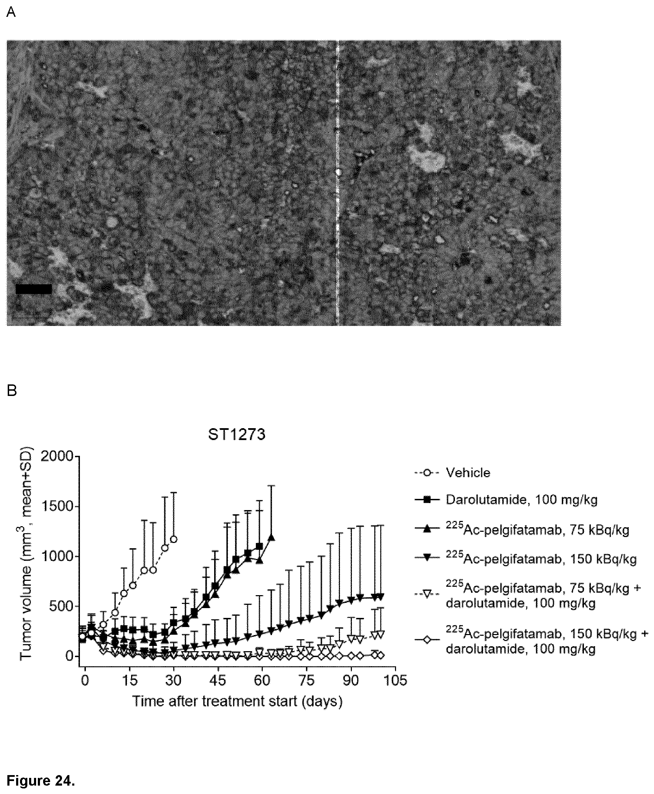

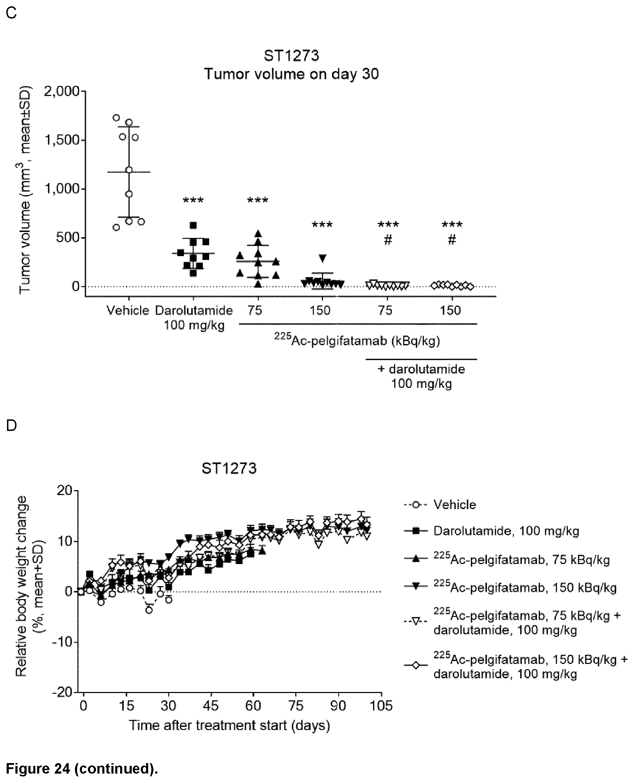

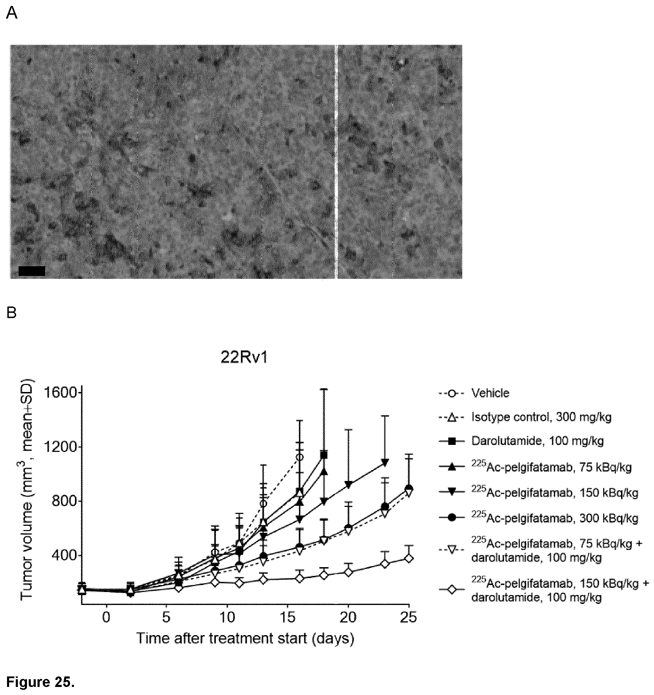

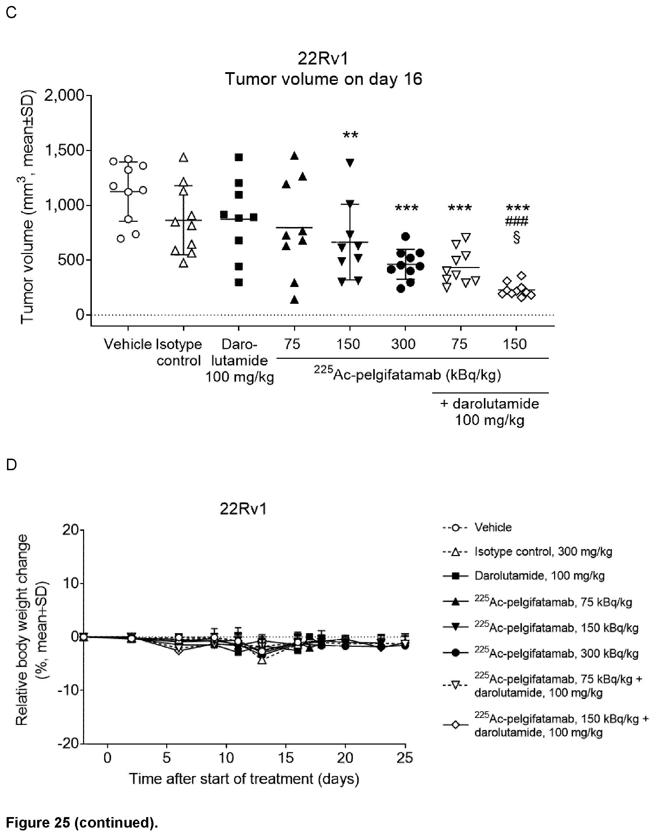

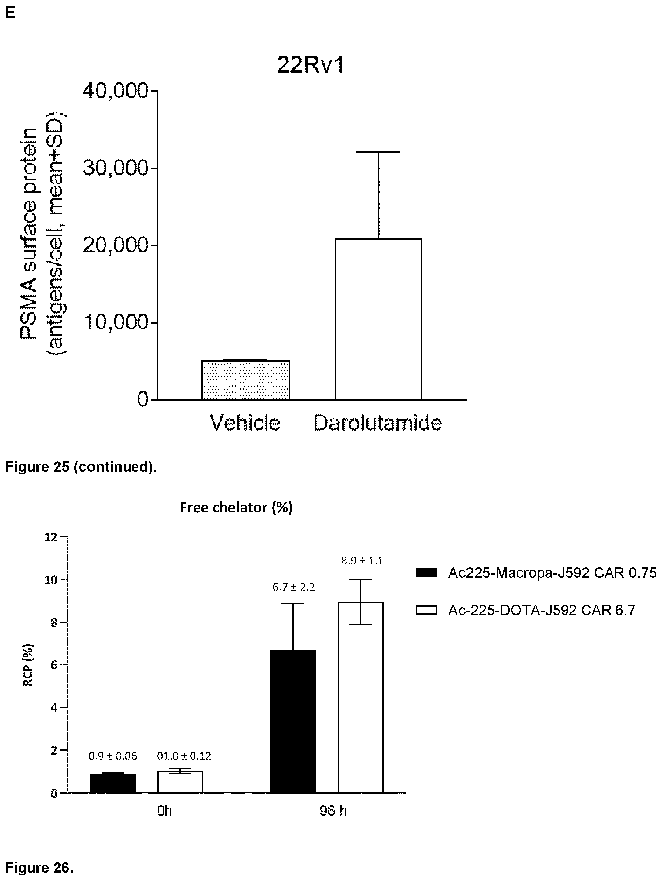

Figure 1. Heavy Chain Nucleotide Sequence (SEQ ID NO. 1) and the Heavy Chain Deduced Amino Acid Sequence (SEQ ID NO. 2) for Pelgifatamab (IgG1 Heavy chain cDNA Gene).
Figure 2. Light Chain Nucleotide Sequence (SEQ ID NO. 3) and the Light Chain Deduced Amino Acid Sequence (SEQ ID NO. 4) for Pelgifatamab (kappa Light chain cDNA Gene).
Figure 3. CDR heavy chain sequences (SEQ ID NO. 5, SEQ ID NO. 6 and SEQ ID NO. 7) and CDR light chain sequences (SEQ ID NO. 8, SEQ ID NO. 9 and SEQ ID NO. 10) for Pelgifatamab.

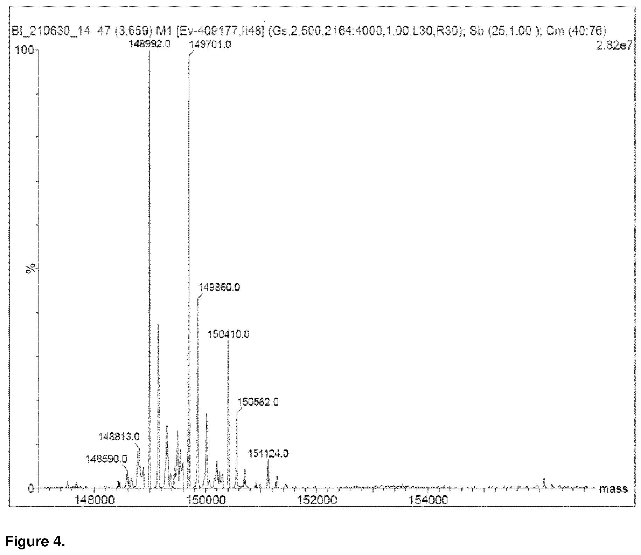

Figure 4. Electrospray mass spectrum of macropa-Pelgifatamab ACC (antibody-chelator conjugate) (CAR 0.8)

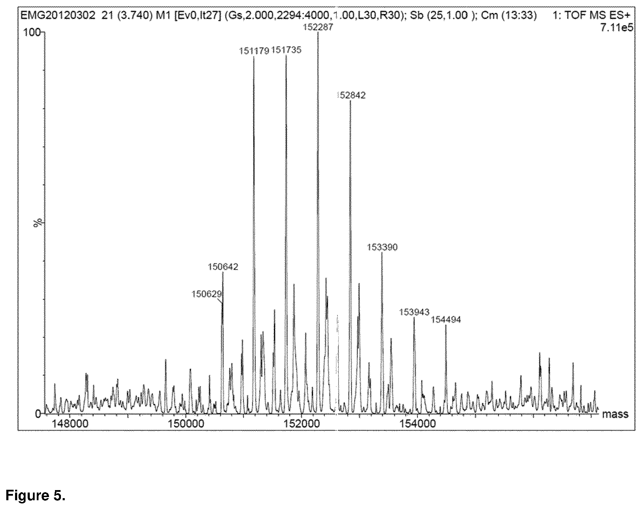

Figure 5. Electrospray mass spectrum of DOTA-Pelgifatamab ACC (antibody-chelator conjugate) (CAR 5.7)

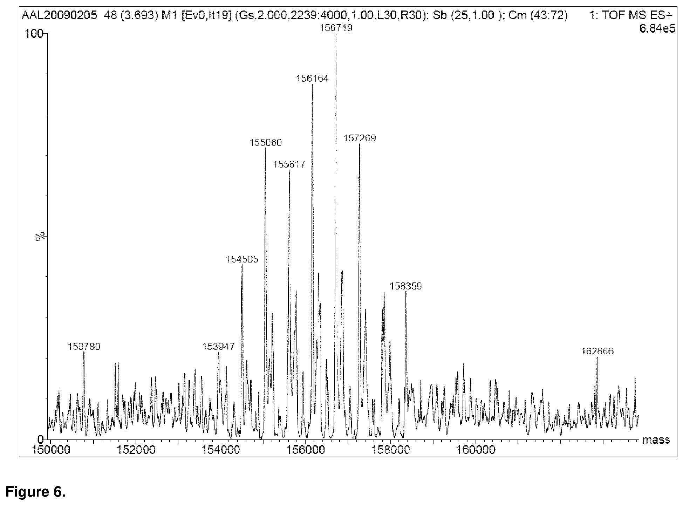

Figure 6. Electrospray mass spectrum of DOTA-Pelgifatamab ACC (antibody-chelator conjugate) (CAR 12.7)
Figure 7. Size-exclusion chromatogram of the targeted actinium conjugates (TACs) Ac225-DOTA-Pelgifatamab (CAR 12.7), Ac225-DOTA-Pelgifatamab (CAR 5.7) and Ac225-Macropa-Pelgifatamab (CAR 0.8).
Figure 8. Radiochemical purity defined as the amount of actinium-225 bound to ²²⁵Ac-pelgifatamab in comparison to actinium-225-labeled DOTA-J591, as determined by instant thin-layer chromatography (iTLC).
Figure 9. Binding of the PSMA-targeting antibody pelgifatamab, macropa-pelgifatamab (non-radiolabeled ACC), and non-radiolabeled isotype control ACC to PSMA-expressing LNCaP prostate cancer cells as determined by flow cytometry.
Figure 10. Internalization of ²²⁵Ac-pelgifatamab in LNCaP prostate cancer cells.
Figure 11. Immunoreactive Fraction (IRF) of Ac225-Macropa-Pelgifatamab (CAR 0.8), Ac225-DOTA-Pelgifatamab (CAR 5.7) and Ac225-DOTA-Pelgifatamab (CAR 12.7).
Figure 12. Cell cytotoxicity of Ac225-Macropa-Pelgifatamab (CAR 0.8), Ac225-DOTA-Pelgifatamab (CAR 5.7) and Ac225-DOTA-Pelgifatamab (CAR 12.7) against PSMA-expressing prostate cancer cell line C4-2.
Figure 13. Cell cytotoxicity of Ac225-Macropa-Pelgifatamab (CAR 0.8), Ac225-DOTA-Pelgifatamab (CAR 5.7) and Ac225-DOTA-Pelgifatamab (CAR 12.7) against PSMA-expressing prostate cancer cell line MDA-PCa-2b.
Figure 14. Cell cytotoxicity of Ac225-Macropa-Pelgifatamab in different prostate cancer cell lines.
Figure 15. Biodistribution of Ac-225-macropa-Pelgifatamab (CAR 0.8) and Ac-225-DOTA-Pelgifatamab (CAR 12.7) in different tissues in the prostate cancer xenograft model MDA-PCA-2b. Animals were dosed with total antibody dose 0.75 mg/kg, 250kBq/kg single dose i.v.
Figure 16. (a) anti-tumor activity of Ac-225-macropa-Pelgifatamab (CAR 0.8) and Ac-225-DOTA-Pelgifatamab (CAR 12.7) in the prostate cancer xenograft model C4-2 in Balb/c nude male mice, single i.v. injection of 300 kBq/kg, 0.14 mg/kg total antibody (b) body weight changes (in percentage, %) (c) white blood cell counts at the end of the experiment.
Figure 17. *In vivo* mode-of action of ²²⁵Ac-pelgifatamab in the C4-2 model. Examples of γH2AX expression as determined by immunohistochemistry in **(A)** untreated C4-2 tumors and in C4-2 tumors treated with 100 kBq/kg ²²⁵Ac-pelgifatamab for **(B)** one day or (C) four days. Scale bars indicate 200 µm. D. γH2AX expression in untreated C4-2 tumors (shown in panel A) and in C4-2 tumors treated with 100 kBq/kg ²²⁵Ac-pelgifatamab (n = 3) for 1 (shown in panel B) or 4 days (shown in panel C), quantified with HSA software.
Figure 18. Growth curves of LNCaP tumors in male SCID mice (n = 10/group) treated with vehicle or ²²⁵Ac-pelgifatamab (70, 125, or 250 kBq/kg; single dose, i.v.; total antibody dose of 0.75 mg/kg). Statistical analyses were performed using two-way ANOVA followed by Tukey's test. ***, *P*<0.001 vs. vehicle (days 0-40).
Figure 19. Growth curves of KUCaP-1 tumors in male scid/scid mice (n = 9-10/group) treated with isotype control (300 kBq/kg; single dose, i.v.; total antibody dose 0.14 mg/kg), or ²²⁵Ac-pelgifatamab (70, 150, or 300 kBq/kg; single dose, i.v.; total antibody dose 0.75 mg/kg). Statistical analyses were performed using two-way ANOVA followed by Tukey's test. ***, *P*<0.001 vs. vehicle (days 0-21); ###, *P*<0.001 vs. isotype control (days 0-21); §§, *P<0.01;* §§§, *P*<0.001 vs. ²²⁵Ac-pelgifatamab, 300 kBq/kg (days 0-21); $$$, *P<0.001* vs. ²²⁵Ac-pelgifatamab, 150 kBq/kg (days 0-21).
Figure 20. Growth curves of large KUCaP-1 tumors (mean volume at treatment start 470 mm³) in male scid/scid mice (n = 9-10/group) treated with vehicle or ²²⁵Ac-pelgifatamab (300 kBq/kg; single dose, i.v.; total antibody dose 0.75 mg/kg).
Figure 21. Volumes of the KUCaP-1 tumors described in (E) on the last day of the isotype control group (day 21). Statistical analyses were performed using two-way ANOVA followed by Tukey's test. ***, *P*<0.001 vs. vehicle (days 0-21); ###, *P*<0.001 vs. isotype control (days 0-21); §§, *P*<0.01; §§§, *P*<0.001 vs. ²²⁵Ac-pelgifatamab, 300 kBq/kg (days 0-21); $$$, *P*<0.001 vs. ²²⁵Ac-pelgifatamab, 150 kBq/kg (days 0-21).
Figure 22. Growth curves of 22Rv1 tumors treated with vehicle, darolutamide (100 mg/kg, BID, p.o.) and/or 225Ac-pelgifatamab 75 (A, upper panel) or 150 kBq/kg (B, lower panel), single dose, i.v., total protein dose 0.75 mg/kg).
Figure 23. Darolutamide enhances PSMA expression and combining it with 225Ac-pelgifatamab results in increased efficacy in prostate cancer cells.
   A.-B. Cell surface PSMA expression in (A) VCaP and (B) 22Rv1 cells treated with increasing concentrations of darolutamide for 7 days, as determined by flow cytometry. DMSO served as baseline control and is depicted with a dashed line.
   C.-H. RNA expression of (C-D) FOLH1, (E-F) CDKN1A, and (G-H) XRCC2 in 22Rv1 cells after a 24-h or 48-h treatment with 2 µM darolutamide, 0.15 kBq/mL 225Ac-pelgifatamab, or their combination as determined by RT-qPCR. Statistical analysis was performed using one-way analysis of variance (ANOVA) with Tukey's multiple comparisons test and correction. *, p<0.05; **, p<0.01 compared with untreated cells.
   I.-J. Isobolograms for the combination effect of darolutamide and 225Ac-pelgifatamab on the proliferation of (E) VCaP and (F) 22Rv1 cells. Cl, combination index
Figure 24. Antitumor efficacy of 225Ac-pelgifatamab and darolutamide in the ST1273 prostate cancer PDX model.
   A. PSMA expression in an ST1273 tumor xenograft (untreated) as detected by IHC using the PSMA antibody GCP-04. Darker color indicates PSMA staining. Scale bar 50 µm.
   B. Growth of ST1273 tumors in mice treated with vehicle, 225Ac-pelgifatamab (single dose, 75 or 150 kBq/kg, i.v.), darolutamide (100 mg/kg, BID, p.o.), or their combination.
   C. Volumes of individual ST1273 tumors shown in (B) on day 30. Statistical analysis was performed using one-way ANOVA followed by Tukey's test. ***, p<0.001 vs. vehicle. #, p<0.05 vs. darolutamide monotherapy.
   D. Relative body weight change of the mice shown in (B) over the course of the study.
Figure 25. Antitumor efficacy of 225Ac-pelgifatamab and darolutamide in the 22Rv1 prostate cancer model.
   A. PSMA expression in an 22Rv1 tumor xenograft (untreated) as detected by IHC using the PSMA antibody GCP-04. Darker color indicates PSMA staining. Scale bar 50 µm.
   B. Growth of 22Rv1 tumors in mice treated with vehicle, isotype control (300 mg/kg, a single dose, i.v.), 225Ac-pelgifatamab (75, 150, or 300 kBq/kg, a single dose, i.v.), darolutamide (100 mg/kg, BID, p.o.), or their combination.
   C. Volumes of individual 22Rv1 tumors shown in (B) on day 16. Statistical analysis was performed using one-way ANOVA followed by Tukey's test. **, p<0.01; ***, p<0.001 vs. vehicle. ###, p<0.001 vs. darolutamide monotherapy. §, p<0.05 vs. the respective 225Ac-pelgifatamab monotherapy.
   D. Relative body weight change of the mice shown in (B) over the course of the study.
   E. PSMA expression in dissociated 22Rv1 cancer cells obtained from 22Rv1 tumor-bearing mice treated with vehicle or darolutamide for 18 days before sacrifice (n=2), as determined by flow cytometry.
Figure 26. Ac-225-Macropa-J592 and Ac-225-DOTA-J592 radiolabelled free chelator iTLC showing % radiolabeled free chelator (RFC) at t = 0h and after 96h.

### EXPERIMENTAL SECTION

Chemical names were generated using the BIOVIA Draw 2019 from Dassault Systèmes. In some cases, generally accepted names of commercially available reagents were used in place of BIOVIA Draw generated names.

The following Table 1 lists the abbreviations used in this paragraph and in the Examples section as far as they are not explained within the text body. Other abbreviations have their meanings customary *per se* to the skilled person.

**Table 1. Abbreviations**

| The following table lists the abbreviations used herein. | |
|---|---|
| 225Ac | actinium-225 |
| Ac-225 | actinium-225 |
| ACC | antibody-chelator conjugate |
| ACN | acetonitrile |
| BCA | Bicinchoninic acid |
| BRFF-HPC1 | Serum free medium |
| CAR | chelator-to-antibody ratio |
| DCTA | 1,2-diaminocyclohexanetetraacetic acid |
| DMA | N,N-dimethylacetamide |
| DMSO | dimethyl sulfoxide |
| DOTA | 1,4,7,10-tetraazacyclododecane-1,4,7,10-tetraacetic acid |
| ESI | electrospray ionization |
| EtOH | ethanol |
| FBS | fetal bovine serum |
| FPLC | fast protein liquid chromatography |
| HCl | hydrochloric acid |
| HPGe | high purity germanium |
| HPLC | high performance liquid chromatography |
| iTLC | instant thin layer chromatography |
| IRF | immunoreactive fraction |
| mAb | monoclonal antibody |
| MEM-NEAA | minimum essential medium-non-essential amino acids |
| min | minutes |
| MS | mass spectrometry |
| NaCl | sodium chloride |
| nm | nanometer |
| nmol | nanomole |
| pelgi | Pelgifatamab, prostate-specific membrane antigen IgG1 antibody |
| Pelgifatamab | prostate-specific membrane antigen IgG1 antibody |
| PBS | phosphate buffered saline |
| PSMA | prostate-specific membrane antigen |
| RAC | radioactive concentration |
| RCP | radiochemical purity |
| RPMI | Roswell Park Memorial Institute 1640 medium |
| SEC | size exclusion chromatography |
| TAC | targeted actinium conjugate (Ac-225 labelled ACC) |
| TFA | trifluoroacetic acid |
| TOF | time of flight |
| UPLC | ultra performance liquid chromatography |
| UV | ultraviolet |

All reagents, for which the synthesis is not described in the experimental part, are either commercially available, or known compounds or may be formed from known compounds by known methods by a person skilled in the art.

The various aspects of the invention described in this application are illustrated by the following examples

The example testing experiments described herein serve to illustrate the present invention

### Tissue-targeting compounds comprising Pelgifatamab

### ACC characterization by size exclusion chromatography and mass spectrometry

CAR of the ACCs was determined by SEC-MS (Water Acquity HPLC connected to Waters XEVO TOF; running buffer: 50/50/0.1 (v/v/v) ACN/Water/TFA; flow rate: 0.06 mL/min, column for on-line desalting: Waters Acquity BEH SEC, 1.7 µm, 2.1 x 150 mm; ESI+ mode) by using MS peak heights in percentage of major peak height for the components mAb, mAb + 1 chelator, mAb + 2 chelators, mAb + 3 chelators etc. and using the formula CAR = Sum(n*An)/Sum An, where n equals the number of chelators and An equals the intensity of the antibody conjugate with n chelators. Purity and concentration of the ACC were determined by SEC-UV (Agilent 1260 Infinity HPLC system, running buffer: 10% DMSO/PBS; flow rate: 0.3 mL/min, column: Waters Acquity BEH SEC, 1.7 µm, 4.6 x 300 mm, detection: UV at 280 nm). The method separates higher aggregate, dimer and fragments from the ACC monomer.

### Example 1: Preparation of macropa-Pelgifatamab ACC

6-[[16-[(6-carboxy-2-pyridyl)methyl]-1,4,10,13-tetraoxa-7,16-diazacyclooctadec-7-yl]methyl]-4-[2-(4-isothiocyanatophenyl)ethoxy]pyridine-2-carboxylic acid (5.8 mg, 8,2 µmol) dissolved in DMA (728 µL) was added to Pelgifatamab (493 mg, 3.3 µmol) in PBS (25 mL). Solution was adjusted to pH 9 by adding 1M carbonate buffer, pH 9.5, (5 mL) and solution shaken overnight. Product was purified by FPLC (column: HiLoad 16/600 Superdex 200 pg column; running buffer: 100 mM acetate/100 mM NaCl 1:1, pH 5; flow: 1 mL/min; detection: UV 214/254 nm) to afford 386 mg (77% yield) of macropa-Pelgifatamab ACC in 100 mM acetate/100 mM NaCl 1:1 (2.3 mg/mL). CAR was determined by MS to be 0.8. Monomeric purity was determined by SEC-UV to be 99%.

### Example 2: Preparation of DOTA-Pelgifatamab ACC CAR 5.7

p-SCN-Bn-DOTA (Macrocyclics, B-205) was dissolved in PBS to 10 mg/mL. 261 µL was added to 990 µL Pelgifatamab (10 mg/mL) dissolved in PBS. The pH was adjusted to 9 with 1M carbonate buffer. The mixture was incubated on a thermomixer shaking at 550 rpm, 37 °C for 2 hours. The conjugate was purified by FPLC (column: HiLoad 16/600 Superdex 200 pg column; running buffer: 100 mM acetate/100 mM NaCl 1:1, pH 5; flow: 1 mL/min; detection: UV 280 nm). The concentration of the combined DOTA-Pelgifatamab fractions was measured by SEC-UV to be 2.6 mg/mL. CAR was determined by MS to be 5.7. Monomeric purity was determined by SEC-UV to be 99%.

### Example 3: Preparation of DOTA-Pelgifatamab ACC CAR 12.7

p-SCN-Bn-DOTA (Macrocyclics, B-205) was dissolved in PBS to 10 mg/mL. 470 µL was added to 990 µL Pelgifatamab (10 mg/mL) dissolved in PBS. The pH was adjusted to 9 with 1M carbonate buffer. The mixture was incubated on a thermomixer shaking at 550 rpm at 22 °C in the dark overnight. The conjugate was purified by FPLC (column: HiLoad 16/600 Superdex 200 pg column; running buffer: 100 mM acetate/100 mM NaCl 1:1, pH 5; flow: 1 mL/min; detection: UV 280 nm). The concentration of the combined DOTA-Pelgifatamab fractions was measured by BCA protein assay to be 2.5 mg/mL. CAR was determined by MS to be 12.7. Monomeric purity was determined by SEC-UV to be 98%.

### Radiolabeling

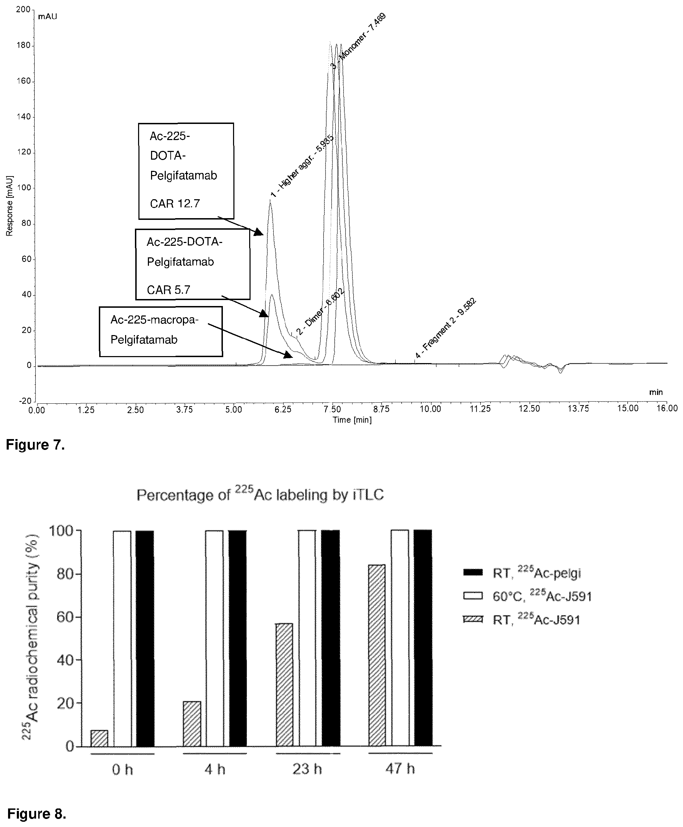

Macropa-Pelgifatamab, DOTA-Pelgifatamab CAR = 5.7 and DOTA-Pelgifatamab CAR = 12.7 were radiolabeled with Ac-225 at a specific activity of 2 MBq/mg and diluted with 0.1 M acetate buffer pH 5 to a radioactive concentration of 1.5 kBq/µL. The mixture was incubated for one hour at room temperature for macropa-Pelgifatamab while the two DOTA-Pelgifatamab ACCs were incubated for one hour at 60 °C on a heating block. The RCP of the TACs was measured by instant thin layer chromatography using citrate buffer for elution, three replicates for each sample. Unlabeled Ac-225 elutes in the solvent front while the radiolabeled compounds do not move from the application section. The measurement of radioactivity was done with a Digital Gamma-Ray Spectrometer (DSPEC-50, Ortec). The activities were measured at least 6 hours after radiolabeling to allow daughter nuclides to be in equilibrium with Ac-225. The RCP of the samples (defined as [(radioactivity of application section)/(total radioactivity on strip)] x 100) was 99.9% ± 0.013; 99.2% ± 0.049 and 99.5% ± 0.110 for Ac-225-macropa-Pelgifatamab, Ac-225-DOTA-Pelgifatamab CAR 5.7 and Ac-225-DOTA-Pelgifatamab CAR 12.7, respectively. Pelgifatamab-macropa and the PSMA binding antibody J591 conjugated with DOTA were both labeled with actinium-225. Labeling was performed at room temperature. J591-DOTA was also labeled at 60°C. Radiochemical purity was determined by instant thin-layer chromatography (iTLC). Labeled and free actinium-225 were counted in a germanium detector using actinium-225 specific energy lines. Actinium-225-labeling of pelgifatamab was efficient at room temperature as compared with actinium-225-labeling of the murine monoclonal PSMA antibody J591 (225Ac-J591, Figure 8). After radiolabeling, the radiochemical purity of 225Ac-pelgifatamab was determined to be 100%, as assessed by (iTLC) (Figure 8).

### Size Exclusion Chromatography of TACs

Purity of the TACs were determined by SEC-UV. The method separates higher aggregate, dimer and fragments from the ACC monomer. The separation was done using a Acquity UPLC Protein BEH SEC, 1.7 µm, 200Å, 4.6 x 300 mm column operated at 30 °C. The mobile phase was 170 mM Ammonium acetate and 300 mM NaCl containing 10% 2-propanol and 0.1 mM DCTA. The flowrate was 0.3 mL/min, and the ACCs were detected at UV 280 nm. The analysis was performed on an Agilent 1200 series HPLC with 35 µL injections.

**Table 2. Purity of TACs by SEC-UV**

| TAC | Area % (UV) | | |
|---|---|---|---|
| | Aggregate | Dimer | Monomer |
| Ac-225-macropa-Pelgifatamab | ND | 0.5 | 99.5 |
| Pelgifatamab-DOTA-Ac-225 CAR 5.7 | 18.4 | 3.0 | 78.6 |
| Pelgifatamab-DOTA-Ac-225 CAR 12.7 | 30.5 | 4.8 | 64.7 |

### Immunoreactive Fraction (IRF)

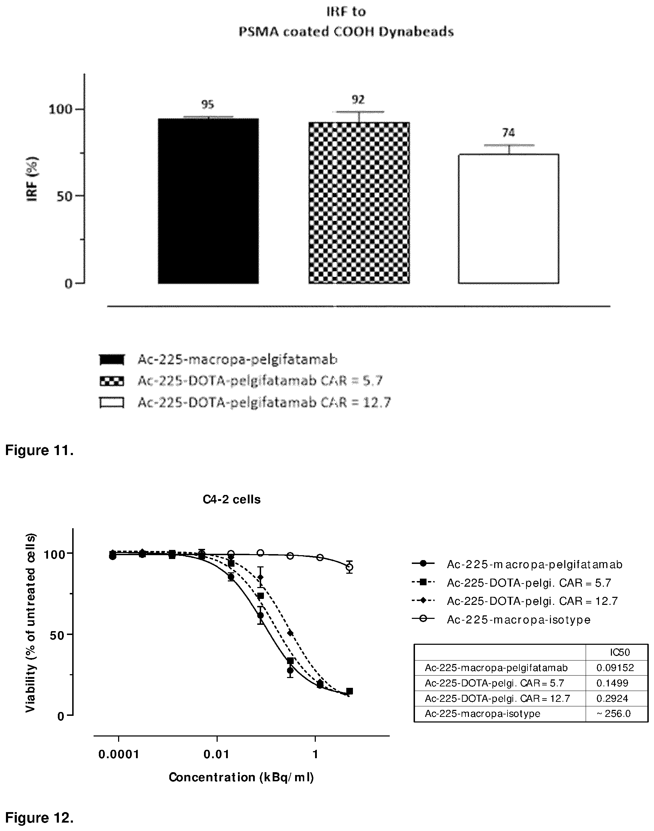

Shortly after completion of the radiolabeling, the binding of each sample to PSMA-coated M-270-carboxy Dynabeads was determined. In short, 250 µg beads were placed in 2 mL Eppendorf tubes in 50 µL buffer (PBS/3% BSA). The samples were run in triplicate and samples blocked with naked PSMA mAb in excess were included. The blocking step lasted for 40 min, while shaking the tubes on a Thermomixer (Eppendorf) at 750 rpm at room temperature. The radiolabeled compounds were diluted to 5 Bq/µL in buffer and added to each bead-containing tube. The binding lasted for 80 min while shaking at 750 rpm at room temperature. After completion of the binding step, 40 µL of buffer was added to each tube and a magnet (DynaMag-2) was used to separate the beads from half of the supernatant. The radio activities in the supernatant and in the beads of each sample were measured using a Digital Gamma-Ray Spectrometer. The activities were measured after at least 6 hours, to allow daughter nuclides to be in equilibrium with Ac-225. Total binding was defined as [(activity in beads - activity in supernatant)/(total activity)] x 100. The unspecific binding was obtained from the same calculation from blocked samples, and IRF was defined as [specific binding - unspecific binding]. The IRF was 95%, 92% and 74% for Ac-225-macropa-Pelgifatamab, Ac-225-DOTA-Pelgifatamab CAR 5.7 and Ac-225-DOTA-Pelgifatamab CAR 12.7, respectively (Figure 11).

### Flow cytometry

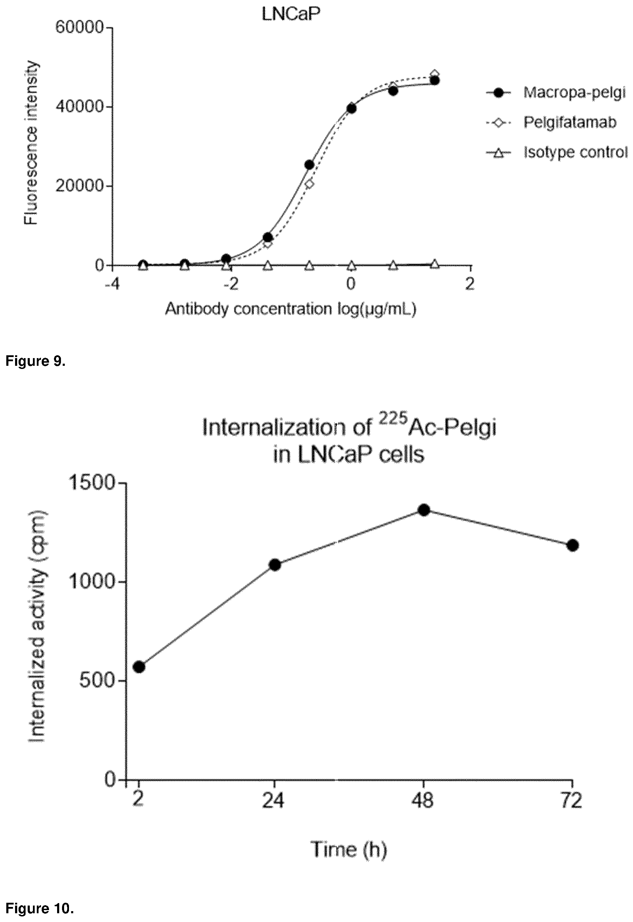

The *in vitro* binding properties of the fully human PSMA-targeting antibody pelgifatamab, the antibody chelator conjugate (ACC) pelgi-macropa, and isotype control were assessed in PSMA-expressing LNCaP prostate cancer cells. Determination of EC₅₀ values and antibodies bound per cell were performed by flow cytometry. Binding of the PSMA-targeting antibody pelgifatamab, macropa-pelgifatamab (non-radiolabeled ACC), and non-radiolabeled isotype control ACC to PSMA-expressing LNCaP prostate cancer cells as determined by flow cytometry (Figure 9).

### Internalization

The internalization of ²²⁵Ac-pelgifatamab was determined using LNCaP cells after a 2-h incubation. The cell-bound and internalized activity of actinium-225 was measured using a gamma counter (Wizard 2470, PerkinElmer) (Figure 10).

### Cell cytotoxicity

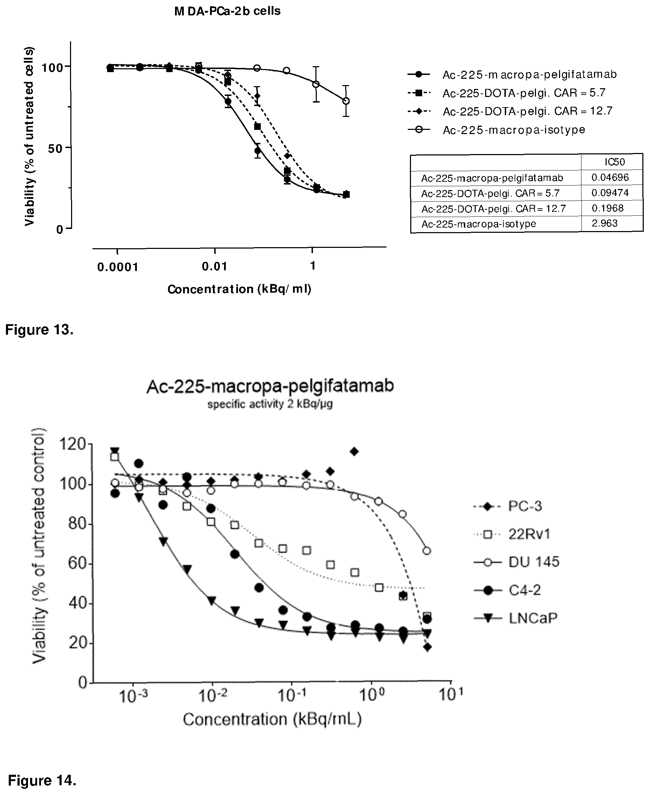

In-vitro cytotoxicity of Ac-225-macropa-Pelgifatamab, Ac-225-DOTA-Pelgifatamab CAR=5.7 and Ac-225-DOTA-Pelgifatamab CAR=12.7 was measured in the PSMA expressing prostate cancer cell lines C4-2 and MDA-PCa-2b. Cells were seeded at an appropriate cell density in cell medium (RPMI/10% FBS/1% HEPES/1% MEM NEAA/1% NaPyruvate/1% L-glutamine and BRFF-HPC1/20% FBS, respectively). One day after seeding, the Ac-225 labeled compounds at a specific activity of 2 MBq/mg were titrated in parallel with a relevant negative isotype control which was radiolabeled in the same conditions. Cells were exposed for two hours to the radioligands. Cell viability was determined six days later using CellTiterGlo (Promega) and luminescence was measured on EnVision plate reader. The IC50 values were 0.09, 0.15 and 0.29 kBq/ml for Ac-225-macropa-Pelgifatamab, Ac-225-DOTA-Pelgifatamab CAR 5.7 and Ac-225-DOTA-Pelgifatamab CAR 12.7, respectively in C4-2 cells (Figure 9) and 0.05, 0.09 and 0.2 kBq/ml for Ac-225-macropa-Pelgifatamab, Ac-225-DOTA-Pelgifatamab CAR 5.7 and Ac-225-DOTA-Pelgifatamab CAR 12.7, respectively in MDA-PCa-2b cells (Figure 10). IC50 values of Ac-225-macropa-isotype were ~256 kBq/ml in C4-2 cells and 3 kBq/ml in MDA-PCa-2b cells (Figure 12+13).

### Cell cytotoxicity

*In-vitro* cytotoxicity of Ac-225-macropa-Pelgifatamab was measured in prostate cancer cell lines expressing different levels of PSMA. PC-3, DU-145, 22Rv1, C4-2 and LNCaP cells were seeded at an appropriate cell density in the following cell media respectively: DMEM/ Ham's F12 + 10% FCS; DMEM + 10% FCS; RPMI + 10% FCS; DMEM/ Ham's F12 + 10% FCS + Insulin (human) (5µg/ml, Sigma #I9278), Transferrin (human); (final: 5µg/ml), D-Biotin (0,25µg/ml final, Sigma:B4639), Adenine (final: 25µg/ml, Sigma: A9795), L-3,3-,5-Triiodothyronine, Sodium Salt, Sigma: T6397 (13.6pg/ml final); RPMI + 20% FCS suggested by the respective suppliers. One day after seeding, Ac-225-macropa-Pelgifatamab at a specific activity of 2 MBq/mg were titrated in parallel with a relevant negative isotype control which was radiolabeled in the same conditions. Cells were treated for 5 days and subsequentially cell viability was determined using CellTiterGlo (Promega). Strong cytotoxicity was observed in LNCaP, C4-2 and 22R1 cells with little cytotoxicity in PMSA negative PC-3 and DU-145 cells (Figure 14)

### Biodistribution study of Ac-225-macropa-Pelgifatamab

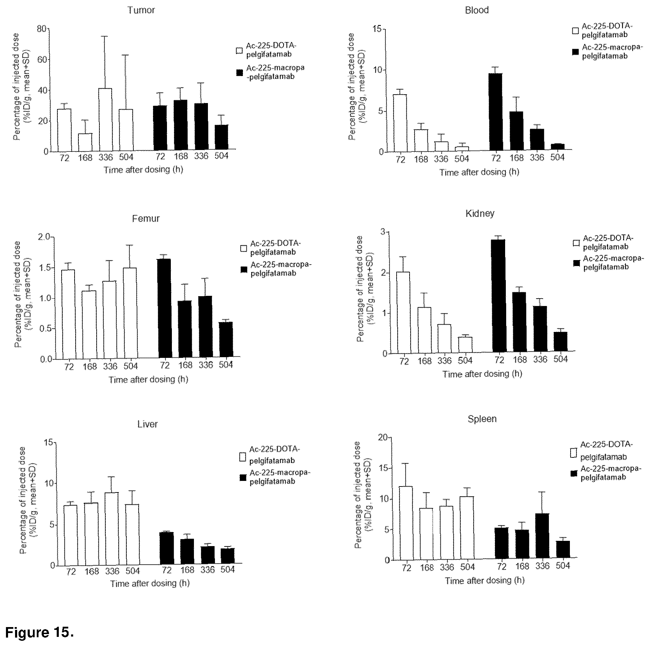

Biodistribution of Ac-225-macropa-Pelgifatamab was investigated in the PSMA-expressing prostate cancer xenograft model MDA PCa 2b. Female RJ:NMRI-Foxn1nu/nu mice were implanted with testosterone pellets subcutaneously and were inoculated with 1 × 10⁶ MDA PCa 2b cells on the right flank. Animals were pre-treated with 200 µg/mouse unspecific IgG2a antibody 24 hours prior to hot dosing. Animals were treated with 300 kBq/kg Ac-225-macropa-Pelgifatamab IV at a total protein dose of 0.75 mg/kg. In a separate *in vivo* study the xenograft model MDA PCa 2b was treated with 250 kBq/kg Ac-225-DOTA-Pelgifatamab at a protein dose of 0.14 mg/kg. In both studies, animals were sacrificed 72, 168, 336, and 504 hours post treatment and 225Ac in blood, tumor, liver, kidney, spleen and femur was determined. For Ac-225-macropa-Pelgifatamab-treated animals, the data indicate strong and persistent tumor accumulation whereas accumulation in normal organs was generally low and transient with clearance over time. Compared with 225Ac-DOTA-Pelgifatamab-treated animals, improved clearance from the bone, lower and over time decreasing radioactivity in the liver and less uptake in the spleen was seen (Figure 15). These data might be explained with an improved *in vivo* complex stability with 225Ac for macropa compared with DOTA.

### In vivo efficacy study comparing Ac-225-macropa-Pelgifatamab and Ac-225-DOTA-Pelgifatamab

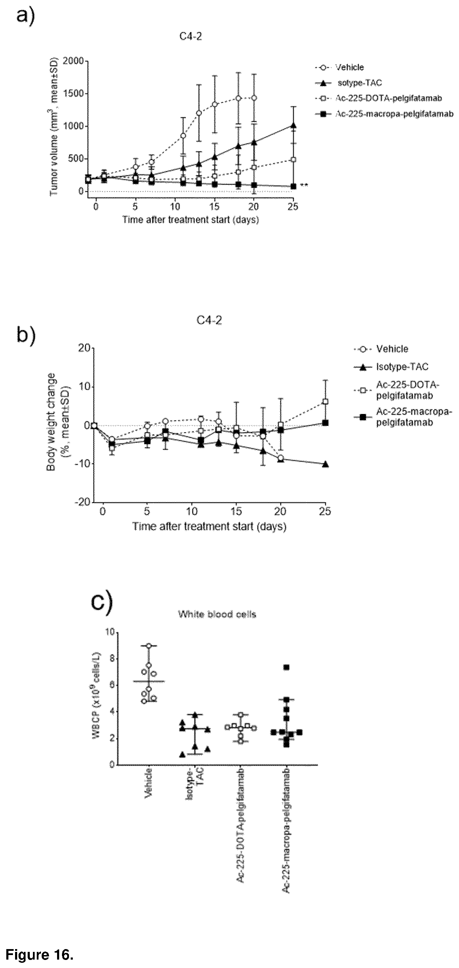

The compounds Ac-225-macropa-Pelgifatamab, Ac-225-DOTA-Pelgifatamab and isotype-macropa were labeled with 225Ac. Mice xenografted with C4-2 prostate cancer cells were treated with a single dose IV of 300 kBq/kg with a total antibody dose of 0.14 mg/kg. As presented in Figure 16 (A) and (B), higher antitumor activity of Ac-225-macropa-Pelgifatamab was seen compared with Ac-225-DOTA-Pelgifatamab. Body weight loss was less than 10% for all targeted treatment groups. Effects on the hematopoietic system were determined by WBC counts at the end of the experiment (Figure 16 (C)).

### Immunohistochemistry

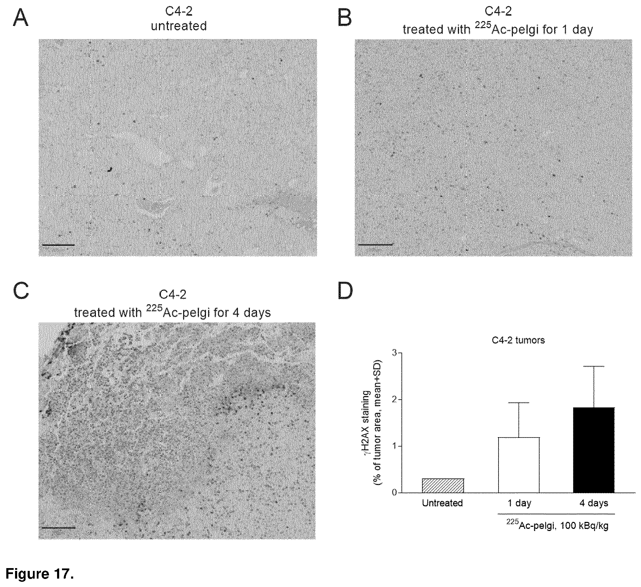

C4-2 tumors were harvested 24 or 96 h after treatment with 120 kBq/kg ²²⁵Ac-pelgifatamab (total antibody dose of 0.75 mg/kg), and the level of phosphorylated histone protein H2AX (γ-H2AX) was evaluated in tissue sections of paraffin-embedded C4-2 tumors (n = 3). Examples of γH2AX expression as determined by immunohistochemistry in (17A) untreated C4-2 tumors and in C4-2 tumors treated with 100 kBq/kg ²²⁵Ac-pelgifatamab for (17B) one day or (17C) four days. Scale bars indicate 200 µm. 17D. γH2AX expression in untreated C4-2 tumors (shown in panel A) and in C4-2 tumors treated with 100 kBq/kg ²²⁵Ac-pelgifatamab (n = 3) for 1 (shown in panel B) or 4 days (shown in panel C), quantified with HSA software (Figure 17 A, B, C, D).

### In vivo antitumor efficacy of ²²⁵Ac-pelgifatamab in the LNCaP, and KUCaP-1 prostate cancer models.

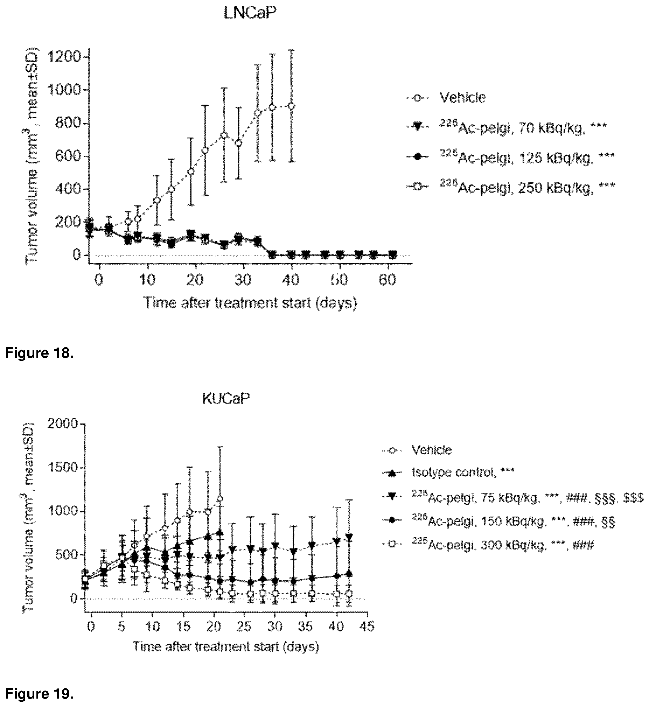

Antitumor efficacy of 225Ac-pelgifatamab was assessed in the LNCaP prostate cancer model: Male SCID mice (6 weeks, 22 g, Janvier Labs) were implanted s.c. with testosterone pellets (12.5 mg, 4 mm) and 1-3 days later, inoculated s.c. with 5x10⁶ LNCaP cells. When the tumors reached an average size of 160 mm³, mice were randomized (n = 10 mice/group), and two days later, treated with vehicle or a single i.v. dose of ²²⁵Ac-pelgifatamab (70, 125, or 250 kBq/kg, total antibody dose 0.75 mg/kg). The study was terminated on day 112 after tumor cell inoculation. In the LNCaP model, ²²⁵Ac-pelgifatamab at 70, 125, and 250 kBq/kg (single dose) showed very strong antitumor efficacy with a T/Cᵥₒₗᵤₘₑ (treatment/control) ratio of 0.00 at all doses tested (Fig. 18).

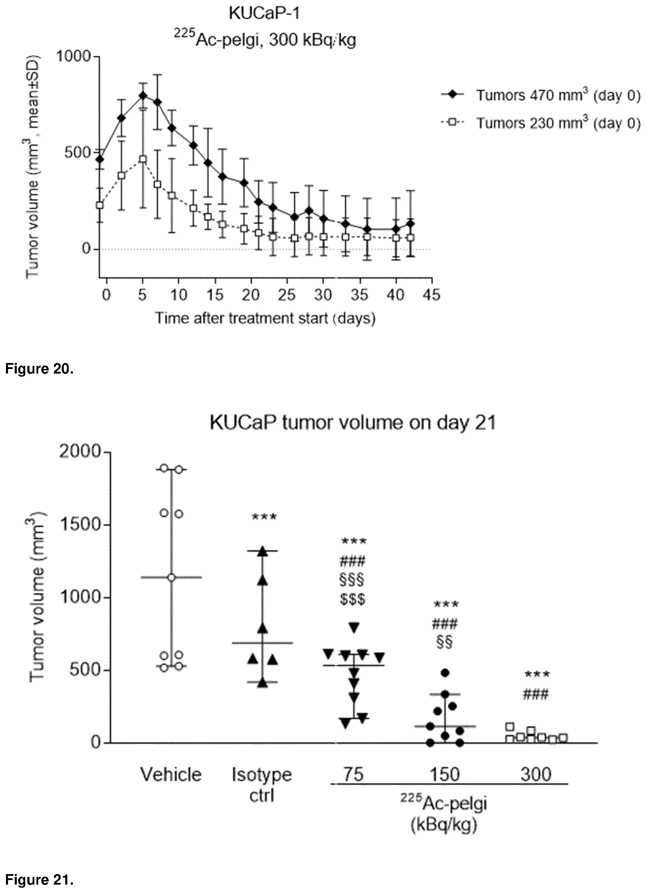

In a KUCaP-1 efficacy study, male scid/scid mice (8 weeks, 22 g, Janvier Labs) were implanted s.c. with 5x5x5 mm KUCaP-1 tumor fragments. When the tumors reached an average size of 220 mm³, mice were randomized (*n* = 9-10 mice/group), and the next day, treated with vehicle or a single i.v. injection of isotype control (300 kBq/kg, total antibody dose 0.14 mg/kg) or ²²⁵Ac-pelgifatamab at 70, 150, or 300 kBq/kg (total antibody dose 0.75 mg/kg). In addition, for one treatment group, tumors were allowed to reach a size of 470 mm³, before treatment with a single dose of 300 kBq/kg ²²⁵Ac- pelgi. The study was terminated on day 72 after tumor cell inoculation. In the patient-derived KuCaP model, ²²⁵Ac-pelgifatamab at 75, 150, and 300 kBq/kg (single dose) showed dose-dependent efficacy resulting in T/Cᵥₒₗᵤₘₑ ratios of 0.55, 0.44, and 0.26, respectively (Fig. 19+21). Notably, a single dose of 300 kBq/kg ²²⁵Ac-pelgifatamab induced tumor regression even in mice with large tumors (Fig. 20).

### Tissue-targeting compounds comprising TLX592/J592

### ACC characterization by size exclusion chromatography and mass spectrometry

CAR of the ACCs was determined by LCMS. For Ac225-Macropa-J592 CAR was 0.75 and for Ac225-DOTA-J592 CAR was 6.7.

### Macropa-NCS-J592

6-[[16-[(6-carboxy-2-pyridyl)methyl]-1,4,10,13-tetraoxa-7,16-diazacyclooctadec-7-yl]methyl]-4-[2-(4-isothiocyanatophenyl)ethoxy]pyridine-2-carboxylic acid was dissolved in DMA to 10 mg/ml. Anti-J592 antibody was dissolved in PBS to 10 mg/mL and the pH was adjusted to 9 with 1M carbonate buffer. Chelator and antibody were mixed and incubated on a thermomixer shaking at 350 rpm at RT for one hour. Product was purified by FPLC (column: Superdex^{®} 200 Increase 10/300 GL; running buffer: 6 mM citrate/10 mM histidine/10 mM glycine/100 mM NaCl; flow: 1 mL/min; detection: UV 214/254 nm). Concentration and monomeric purity were determined by SEC-UV CAR was determined by SEC-MS using positive electrospray ionization and the following equation: CAR=[(A*An)/A]0, A=Intensity of all signals of the maximum entropy envelope, An=signals of the maximum entropy envelope containing chelators. The CAR was determined to 0.8.

### DOTA-NCS-J592

p-SCN-Bn-DOTA (Macrocyclics, B-205) and anti-J592 antibody were dissolved in PBS to 10 mg/mL. The pH of the antibody was adjusted to 9 with 1M carbonate buffer. p-SCN-Bn-DOTA was added to the antibody and the mixture was incubated on a thermomixer shaking on a thermomixer shaking at 350 rpm at RT for one hour. The conjugate was purified by FPLC (column: Superdex^{®} 200 Increase 10/300 GL; running buffer: 6 mM citrate/10 mM histidine/10 mM glycine/100 mM NaCl; flow: 1 mL/min; detection: UV 280 nm). The concentration and monomeric purity of the combined DOTA-antibody fraction were measured by SEC-UV. CAR was determined by SEC-MS using positive electrospray ionization and the following equation: CAR=[(A*An)/A]0, A=Intensity of all signals of the maximum entropy envelope, An=signals of the maximum entropy envelope containing chelators. The CAR was determined to 6.7.

### Buffer exchange

Amicon Ultra-4 30K (Millipore, cat# UFC803096, lot#R4JA84392) was used to buffer exchange J592-DOTA and J592-Macropa. The ultrafiltration membranes in Amicon^{®} Ultra-4 devices were rinsed with 3.5 mL 0.1M acetate buffer (ref J145-046, 21Nov22AHJ). Spin at 7000 × g, 2 min. Removed acetate buffer. Added samples and 3.5 mL 0.1M acetate buffer, spin 7000 × g, 10 min for buffer exchange. Recovered the samples and dilute in 0.5 mL 0.1M acetate buffer. New concentration was determined by SE-HPLC by comparing UV-signal from previous pre-Amicon samples with known concentration.

### Radiolabeling

Macropa-J592 CAR 0.75 and DOTA-J592 CAR 6.7 were radiolabeled with Ac-225 at a specific activity of 2 MBq/mg and diluted with 0.1 M acetate buffer pH 5 to a radioactive concentration of 1.5 kBq/µL. The mixture was incubated for one hour at room temperature for J592-Macropa while the DOTA-J592 was incubated for one hour at 60 °C on a heating block. The RCP of the TACs was measured by instant thin layer chromatography using citrate buffer for elution, three replicates for DOTA and one for Macropa. Unlabeled Ac-225 elutes in the solvent front while the radiolabeled compounds do not move from the application section. The measurement of radioactivity was done with a HPGe Detector (DSPEC-50, Ortec). The activities were measured at least 6 hours after radiolabeling to allow daughter nuclides to be in equilibrium with Ac-225. Two timepoint was analyzed, at 0h and after 96h stored in room temperature. The RCP of the samples (defined as [(radioactivity of application section)/ (total radioactivity on strip)] x 100) was 99.9% (0h) and 99.8 % (96h); 99.5% ± 0.06 (0h) and 96.0% ± 0.35 (96h) for Ac-225-macropa-J592 and Ac225-DOTA-J592, respectively. This data shows that Ac-225-macropa-J592 releases less 225Ac than Ac225-DOTA-J592 at t = 96h and is therefore more stable towards radionuclide leakage.

### Size Exclusion Chromatography of TACs

Purity of the TACs were determined by SEC. The method separates higher aggregate, dimer and fragments from the ACC monomer. The separation was done using a Acquity UPLC Protein BEH SEC, 1.7 µm, 200Å, 4.6 x 300 mm column operated at 30 °C. The mobile phase was 200 mM Ammonium acetate and 300 mM NaCl containing 10% 2-propanol and 0.1 mM DCTA. The flowrate was 0.3 mL/min, and the ACCs were detected at UV 280 nm. The analysis was performed on an Agilent 1200 series HPLC with 40 µL injections.

**Table 3. Purity of TACs by SEC-UV**

| | Area % (UV) | | | | | | |
|---|---|---|---|---|---|---|---|
| | 0h | | | 96h | | | |
| | Aggregate | Dimer | Monomer | Aggregate | Dimer | Monomer | Fragmentation |
| Ac-225-Macropa -NCS-J592 | 0,0 | 0.1 | 99,9 | 0,7 | 7,7 | 91,4 | 0,2 |
| Ac-225-DOTA -NCS-J592 | 0,8 | 4,2 | 99,8 | 2,5 | 12,0 | 85,7 | 0,2 |

The results from Table 3 show the increased stability of a tissue-targeting compound of formula (I) when compared to its DOTA counterpart, as indicated by the lower amounts of by-products (i.e., aggregate and dimer) present after 96h.

### iTLC (RFC)

A free chelator iTLC was preformed to measure the amount of free chelator in the conjugate solution over time. In brief, an iTLC.SG.plate was cut to obtain 12 cm x 1 cm strips. Application point, cut point and end of run point was marked at 1 cm, 4 cm and 10 cm, respectively. 2 µL of TAC sample was applied on n = 3 iTLC strips for elution with a freshly made 1:1 1M ammonium acetate /100% methanol mobile phase The strips were developed in 3 mL mobile phase in LSC Vials. When the liquid front had reached the marked end point, they were removed from the vial and placed on an aluminium foil to dry. The samples were read off after 6h, to obtain secular equilibrium in the sample. iTLC Scanner AR-2000 from Eckert & Siegler was used to measure the iTLC strips, 1 sec each strip at 1000 volt.

Figure 26 shows the increased stability of a tissue-targeting compound of formula (I) towards decomplexation when compared to its DOTA counterpart, as indicated by the lower amounts of free chelator present after 96h.

### Combination study of 225Ac-pelgifatamab with darolutamide

The effect of darolutamide and 225Ac-pelgifatamab on PSMA mRNA and protein expression *in vitro* was studied in VCaP and 22Rv1 prostate cancer cells. To assess PSMA protein expression, VCaP and 22Rv1 cells were treated with 0.08-5 µM darolutamide for 7 days and PSMA expression was determined by flow cytometry using the murine PSMA-specific monoclonal antibody (mAb) J591 (prepared in-house). To assess FOLH1 gene expression, 22Rv1 cells were treated with 2 µM darolutamide and/or 0.15 kBq/mL 225Ac-pelgifatamab for 24 or 48 h. RNA was extracted with the RNeasy Plus Mini Kit (Qiagen), transcribed to cDNA using SuperScript IV VILO Master Mix (Invitrogen), and analyzed by qPCR.

To assess the combinatory effect of 225Ac-pelgifatamab and darolutamide *in vitro,* VCaP and 22Rv1 cells were treated with darolutamide, 225Ac-pelgifatamab, or their combination for 5 days. Cell viability was determined with CellTiter-Glo^{®} (Promega). Combination indexes (Cls) were calculated according to the median-effect model of Chou-Talalay (8) with Cl < 0.9 defined as synergistic effect.

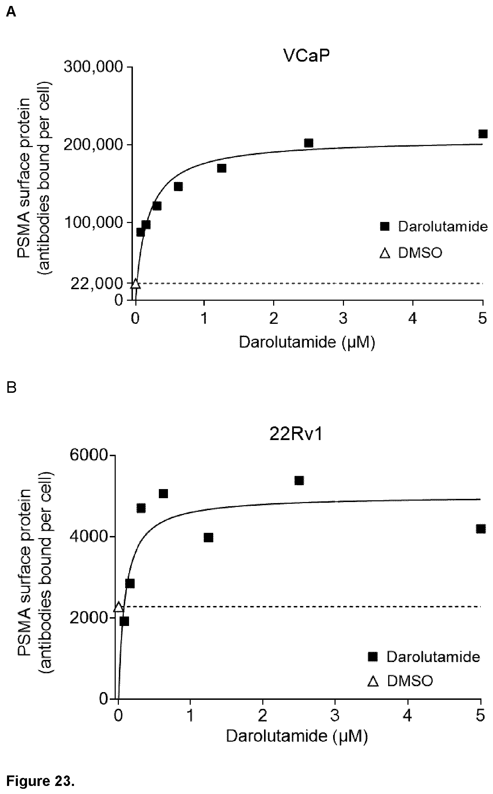

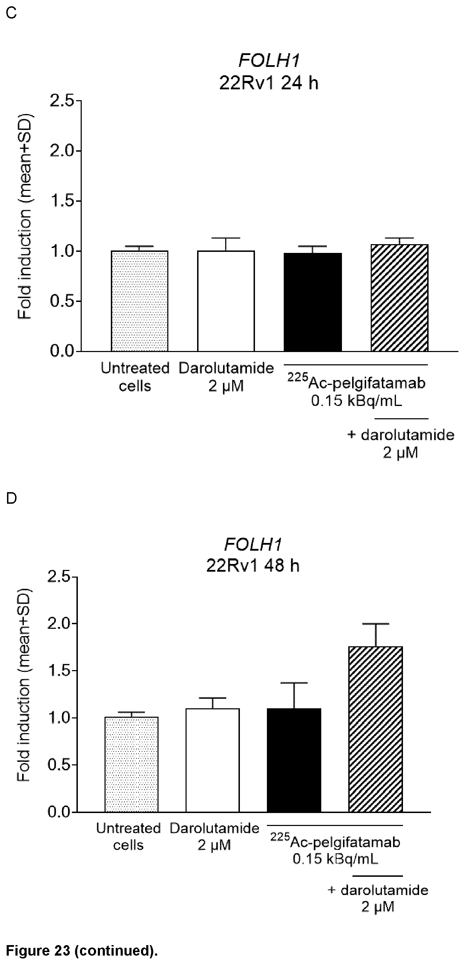

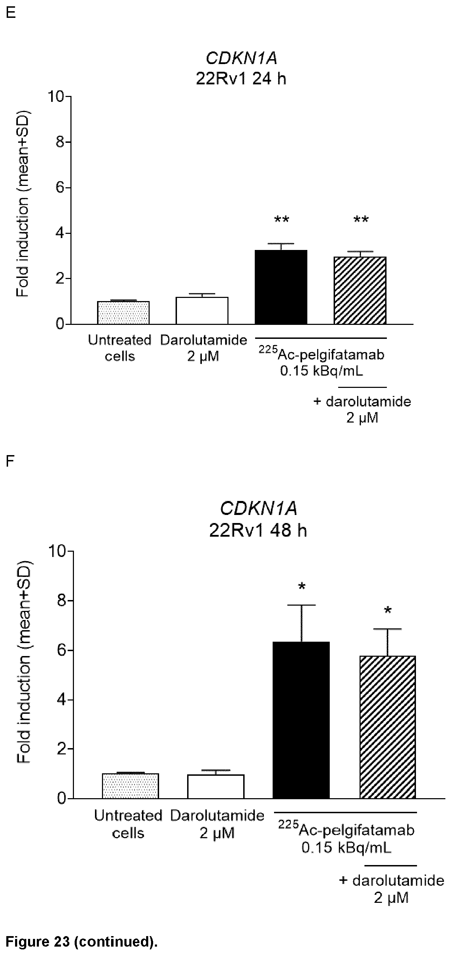

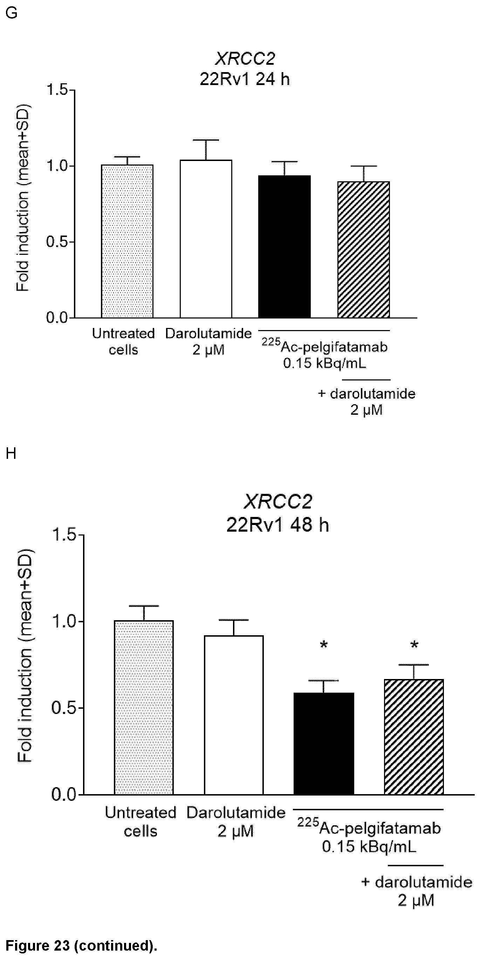

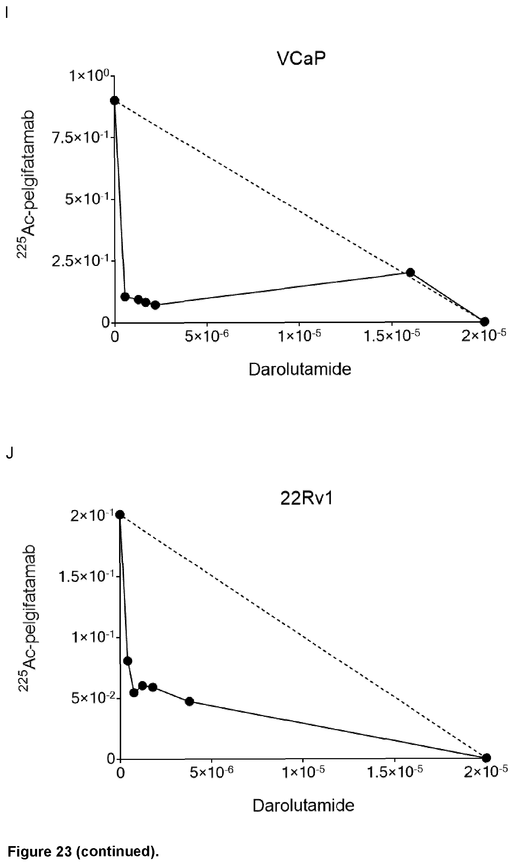

*In vitro,* darolutamide induced the expression of PSMA in androgen-dependent VCaP and androgen-independent 22Rv1 cells by more than 10-fold and 2-fold, respectively (Fig. 23).

The combination treatment of darolutamide and 225Ac-pelgifatamab showed a 76% increase in FOLH1 gene expression in 22Rv1 cells after a 48-h treatment (Fig. 23). No increase was observed with the respective monotherapies.

The 225Ac-pelgifatamab monotherapy and the 225Ac-pelgifatamab and darolutamide combination treatment increased RNA expression of CDKN1A (Fig. 23) and decreased expression of XRCC2 (Fig. 23) in 22Rv1 cells.

The efficacy of darolutamide in combination with 225Ac-pelgifatamab was synergistic in VCaP and 22Rv1 cells with combination indexes of 0.34 and 0.38, respectively (Fig. 23).

Darolutamide enhances PSMA expression and combining it with 225Ac-pelgifatamab results in increased efficacy in prostate cancer cells.

PSMA expression in isolated, untreated 22Rv1 and ST1273 tumors was determined by immunohistochemistry (IHC) using the murine PSMA-specific monoclonal antibody GCP-04 (Novus Biologicals) in formalin-fixed, paraffin-embedded (FFPE) tissue sections.

The *in vivo* efficacy of 225Ac-pelgifatamab in combination with darolutamide was studied in the androgen-dependent ST1273 and androgen-independent 22Rv1 prostate cancer models in mice.

In the ST1273 study, female NMRI nude mice (n=10/group) were implanted with ST1273 patient-derived tumor fragments, and after 19 days, treatments with vehicle (PEG400, propyleneglycol, glucose; v/v % 50:30:20), 225Ac-pelgifatamab (75 or 150 kBq/kg, a single dose, i.v.), darolutamide (100 mg/kg, twice daily (BID) for 34 days, p.o.), or their combination.

In the 22Rv1 study, male NMRI nude mice (n=10/group) were inoculated with 22Rv1 cells, and after 15 days, treatments with vehicle (acetate buffer), 225Ac-pelgifatamab (75 kBq/kg, a single dose, i.v.; total antibody dose 75 mg/kg), darolutamide (100 mg/kg, BID, p.o.), or their combination.

*In vivo* expression of PSMA protein was evaluated in 22Rv1 prostate tumor-bearing male nude (nu/nu) mice treated with vehicle or darolutamide (100 mg/kg, BID, p.o) for 18 days. 22Rv1 tumors were extracted (n=2) and dissociated tumor cells were analyzed by flow cytometry using the J591 antibody.

### Combining 225Ac-pelgifatamab with darolutamide enhances treatment efficacy in the androgen-dependent ST1273 prostate cancer PDX model

In the PSMA-expressing (Fig. 24A), androgen-dependent, patient-derived ST1273 model, a single i.v. injection of 225Ac-pelgifatamab at 75 or 150 kBq/kg resulted in high antitumor efficacy with treatment/control (T/C) ratios of 0.22 and 0.05 respectively. Darolutamide monotherapy at 100 mg/kg (BID, p.o.) showed good antitumor efficacy with a T/C ratio of 0.29 (Fig. 24B-C, Table 4).

Notably, the combination of 225Ac-pelgifatamab at either 75 or 150 kBq/kg with darolutamide (100 mg/kg) resulted in additive antitumor efficacy with a T/C ratio of 0.01 for both doses (Fig. 24B-C, Table 4). Furthermore, 5/10 and 6/10 mice were tumor-free 100 days after the single 225Ac-pelgifatamab injection.

All treatments were well tolerated, as evidenced by stable body weights throughout the study (Fig. 24D).

**Table 4. In vivo efficacy of ²²⁵Ac-pelgifatamab and darolutamide in the ST1273 and 22Rv1 prostate cancer models.**

| **Model** | **Treatment** | **T/C** |
|---|---|---|
| **ST1273** | | **On day 30** |
| | Vehicle | 1.00 |
| | ²²⁵Ac-pelgifatamab (75 kBq/kg, single dose, i.v.) | 0.22 *** |
| | ²²⁵Ac-pelgifatamab (150 kBq/kg, single dose, i.v.) | 0.05 *** |
| | Darolutamide (100 mg/kg, BID, p.o.) | 0.29 *** |
| | Darolutamide (100 mg/kg, BID, p.o.) + ²²⁵Ac-pelgifatamab (75 kBq/kg, single dose, i.v.) | 0.01 ***, ^{#} |
| | Darolutamide (100 mg/kg, BID, p.o.) + ²²⁵Ac-pelgifatamab (150 kBq/kg, single dose, i.v.) | 0.01 ***, ^{#} |
| | | |

| **22Rv1** | | **On day 16** |
|---|---|---|
| | Vehicle | 1.00 |
| | Isotype control (300 kBq/kg) | 0.77 |
| | ²²⁵Ac-pelgifatamab (75 kBq/kg, single dose, i.v.) | 0.71 |
| | ²²⁵Ac-pelgifatamab (150 kBq/kg, single dose, i.v.) | 0.59 ** |
| | ²²⁵Ac-pelgifatamab (300 kBq/kg, single dose, i.v.) | 0.41 *** |
| | Darolutamide (100 mg/kg, BID, p.o.) | 0.78 |
| | Darolutamide (100 mg/kg, BID, p.o.) + ²²⁵Ac-pelgifatamab (75 kBq/kg, single dose, i.v.) | 0.38 *** |
| | Darolutamide (100 mg/kg, BID, p.o.) + ²²⁵Ac-pelgifatamab (150 kBq/kg, single dose, i.v.) | 0.20 ***, ^{###}, ^{§} |

| | | |
|---|---|---|
| T/C, treatment/control ratio as determined from tumor volumes. Statistical analyses were performed using one-way ANOVA followed by Tukey's test. **, p<0.01; ***, p<0.001 vs. vehicle. ^{#}, p<0.05; ^{###}, p<0.001 vs. darolutamide monotherapy. ^{§}, p<0.05 vs. the respective ²²⁵Ac-pelgifatamab monotherapy. | | |

**The antitumor efficacy of 225Ac-pelgifatamab is enhanced by darolutamide also in the androgen-independent 22Rv1 prostate cancer model**

In the PSMA-expressing (Fig. 25A), androgen-independent cell line-derived 22Rv1 model, a single i.v. dose of 225Ac-pelgifatamab at 150 kBq/kg showed antitumor efficacy with a T/C ratio of 0.59 (Fig. 25B-C, Table 4). Darolutamide (100 mg/kg, p.o., BID) monotherapy was not efficacious as demonstrated by a T/C ratio of 0.78 on day 16.

Remarkably, the combination of 225Ac-pelgifatamab (150 kBq/kg) and darolutamide resulted in enhanced efficacy with a T/C ratio of 0.20 (Fig. 25B-C, Table 4).

All treatments were well tolerated as evidenced by stable body weights over the course of study (Fig. 25D).

Flow cytometric analysis of isolated 22Rv1 tumors showed a 50-fold increase in PSMA expression upon darolutamide treatment compared with vehicle (Fig. 25E).

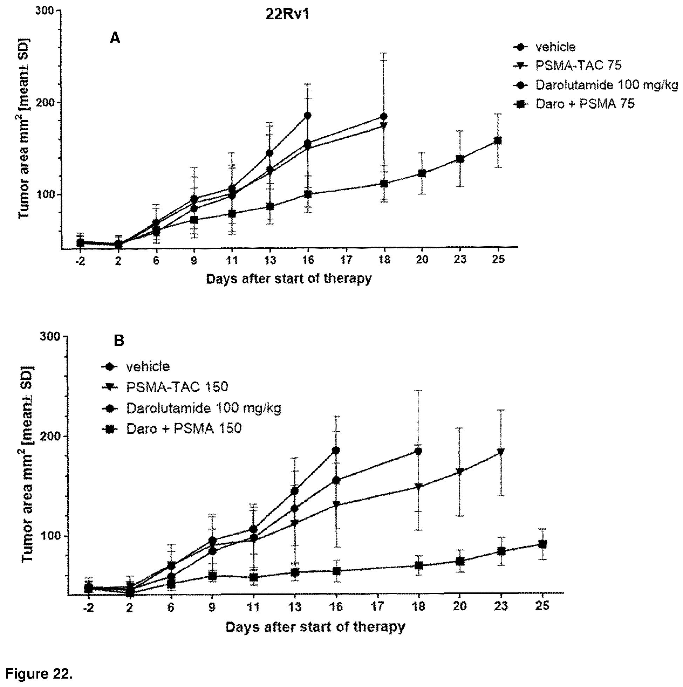

The *in vivo* antitumor efficacy of ²²⁵Ac-pelgi in combination with darolutamide was evaluated in the androgen-independent prostate cancer model 22Rv1. Male nude mice (n = 10-12 mice/group, nude (nu/nu) were treated with a single injection of ²²⁵Ac-pelgi (75 or 150 kBq/kg, i.v., total protein dose 0.75 mg/kg) and/or darolutamide (100 mg/kg, BID, p.o.) starting from day 15 after 22Rv1 tumor cell inoculation. Antitumor efficacy was significantly improved in the combination group compared to both monotherapy groups (Figure 22).

## Claims

1. A tissue-targeting compound of formula (I) 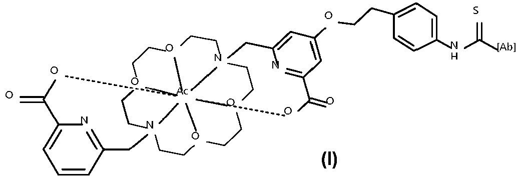 wherein [Ab] is a monoclonal antibody or an antigen-binding fragment thereof having binding affinity for the prostate-specific membrane antigen (PSMA).

2. The tissue-targeting compound of formula (I) according to claim 1, wherein [Ab] is the anti-PSMA-monoclonal antibody Pelgifatamab or an antigen-binding fragment thereof, the anti-PSMA-monoclonal antibody J591 or an antigen-binding fragment thereof or the anti-PSMA-monoclonal antibody TLX592 or an antigen-binding fragment thereof.

3. The tissue-targeting compound of formula (I) according to claim 1, wherein [Ab] is the anti-PSMA-monoclonal antibody Pelgifatamab or an antigen-binding fragment thereof.

4. The tissue-targeting compound of formula (I) according to any of claims 1 to 3, wherein [Ab] is the anti-PSMA-antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10.

5. The tissue-targeting compound of formula (I) according to any of claims 1 or 2, wherein [Ab] is the anti-PSMA-antibody J591 or an antigen-binding fragment thereof comprising at least the three CDR heavy chain sequences according to SEQ ID NO. 13, SEQ ID NO. 14 and SEQ ID NO. 15 and the three CDR light chain sequences according to SEQ ID NO. 16, SEQ ID NO. 17 and SEQ ID NO. 18.

6. The tissue-targeting compound of formula (I) according to any of claims 1 or 2, wherein [Ab] is the anti-PSMA-antibody TLX592 or an antigen-binding fragment thereof comprising at least the three CDR heavy chain sequences according to SEQ ID NO. 21, SEQ ID NO. 22 and SEQ ID NO. 23 and the three CDR light chain sequences according to SEQ ID NO. 24, SEQ ID NO. 25 and SEQ ID NO. 26.

7. A process for the preparation of a tissue-targeting compound of formula (I) 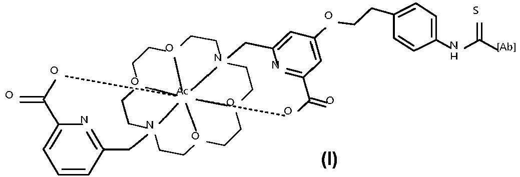
wherein [Ab] is a monoclonal antibody or an antigen-binding fragment thereof having binding affinity for PSMA,
said method comprising
(i) coupling a chelating moiety of formula (II) 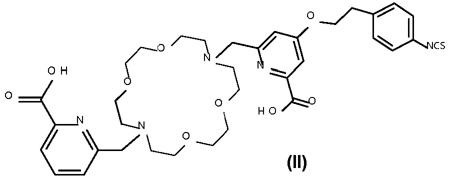 with a monoclonal antibody or an antigen-binding fragment thereof having binding affinity for PSMA, and
(ii) contacting the resulting product with an aqueous solution comprising an ion of at least one alpha-emitting actinium isotope.

8. The process according to claim 7, wherein [Ab] is the anti-PSMA antibody Pelgifatamab or an antigen-binding fragment thereof,
said method comprising
(i) coupling a chelating moiety of formula (II) 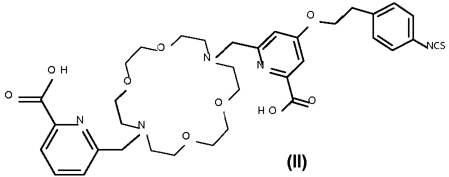 with the anti-PSMA antibody Pelgifatamab or an antigen-binding fragment thereof via a Lysine residue of the monoclonal antibody or antigen-binding fragment thereof, and
(ii) contacting the resulting product with an aqueous solution comprising an ion of at least one alpha-emitting actinium isotope.

9. The process according to claim 7, wherein [Ab] is the anti-PSMA antibody Pelgifatamab or an antigen-binding fragment thereof,
said method comprising
(i) coupling a chelating moiety of formula (II) 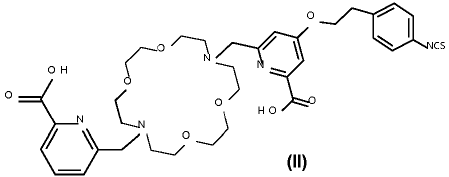 with the anti-PSMA antibody Pelgifatamab or an antigen-binding fragment thereof via a Lysine residue of the monoclonal antibody or antigen-binding fragment thereof, and
(ii) contacting the resulting product with an aqueous solution comprising an ion of at least one alpha-emitting actinium isotope,
wherein the anti-PSMA antibody Pelgifatamab or an antigen-binding fragment thereof comprises at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10.

10. A tissue-targeting chelator of formula (III) 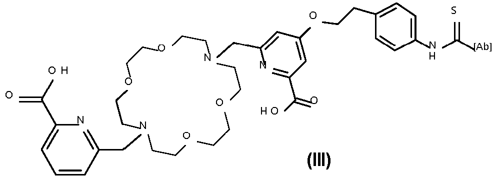 wherein [Ab] is a monoclonal antibody or an antigen-binding fragment thereof having binding affinity for PSMA.

11. The tissue-targeting chelator of formula (III) according to claim 10, wherein [Ab] is the anti-PSMA-monoclonal antibody Pelgifatamab or an antigen-binding fragment thereof, the anti-PSMA-monoclonal antibody J591 or an antigen-binding fragment thereof or the anti-PSMA-monoclonal antibody TLX592 or an antigen-binding fragment thereof.

12. The tissue-targeting chelator of formula (III) according to claim 10, wherein [Ab] is the anti-PSMA-monoclonal antibody Pelgifatamab or an antigen-binding fragment thereof.

13. The tissue-targeting chelator of formula (III) according to any of claims 10 to 12, wherein [Ab] is the anti-PSMA-antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10.

14. The tissue-targeting chelator of formula (III) according to any of claims 10 or 11, wherein [Ab] is the anti-PSMA-antibody J591 or an antigen-binding fragment thereof comprising at least the three CDR heavy chain sequences according to SEQ ID NO. 13, SEQ ID NO. 14 and SEQ ID NO. 15 and the three CDR light chain sequences according to SEQ ID NO. 16, SEQ ID NO. 17 and SEQ ID NO. 18.

15. The tissue-targeting chelator of formula (III) according to any of claims 10 or 11, wherein [Ab] is the anti-PSMA-antibody TLX592 or an antigen-binding fragment thereof comprising at least the three CDR heavy chain sequences according to SEQ ID NO. 21, SEQ ID NO. 22 and SEQ ID NO. 23 and the three CDR light chain sequences according to SEQ ID NO. 24, SEQ ID NO. 25 and SEQ ID NO. 26.

16. Use of a tissue-targeting chelator of formula (III) according to any of claims 10 to 15 for the preparation of a tissue-targeting compound of formula (I) according to any of claims 1 to 6.

17. A combination comprising
(i) a tissue-targeting compound of formula (I) 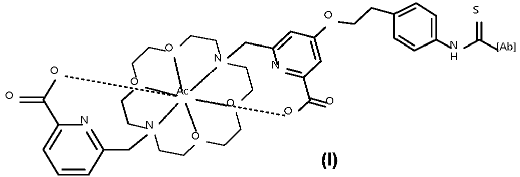 wherein [Ab] is a monoclonal antibody or an antigen-binding fragment thereof having binding affinity for the prostate-specific membrane antigen (PSMA), and
(ii) a pharmaceutical agent selected from a non-steroidal antiandrogen, a steroidal antiandrogen, an androgen synthesis inhibitor, an antigonadotropin, a PARP inhibitor, an ATR inhibitor, an ATM inhibitor, a DNA-PK inhibitor, an AKT inhibitor, a Pi3K inhibitor, a PSMA-targeting beta emitter, an immune checkpoint inhibitor, an alpha emitter, a vaccine and a chemotherapeutic agent.

18. The combination according to claim 17, wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10.

19. The combination according to any of claims 17 or 18, wherein the pharmaceutical agent is selected from a non-steroidal antiandrogen, a steroidal antiandrogen, an androgen synthesis inhibitor, an antigonadotropin, a PARP inhibitor, a PSMA-targeting beta emitter, an alpha emitter, a vaccine and a chemotherapeutic agent.

20. The combination according to any of claims 17 to 19, wherein the pharmaceutical agent is selected from a non-steroidal antiandrogen, a steroidal antiandrogen, an androgen synthesis inhibitor, and an antigonadotropin.

21. The combination according to any of claims 17 to 20, wherein the pharmaceutical agent is selected from a non-steroidal antiandrogen selected from the group consisting of darolutamide, bicalutamide, enzalutamide, apalutamide, flutamide, nilutamide and topilutamide, a steroidal antiandrogen selected from the group consisting of Cyproterone acetate, Allylestrenol, Chlormadinone acetate, Delmadinone acetate, Gestonorone caproate, Hydroxyprogesterone caproate, Medroxyprogesterone acetate, Megestrol acetate, Osaterone acetate, Oxendolone and Spironolactone, an androgen synthesis inhibitor selected from the group consisting of ketoconazole, abiraterone, aminoglutethimide, goserelin and seviteronel, an antigonadotropin selected from the group consisting of Abarelix, Danazol, Gestrinone, Paroxypropione, Cetrorelix, Degarelix, Elagolix, Ganirelix, Linzagolix and Relugolix and a PARP inhibitor selected from the group consisting of Olaparib, Rucaparib, Veliparib, Niraparib, Talazoparib, Pamiparib, CEP 9722, E7016 and Iniparib.

22. The combination according to any of claims 17 to 21, wherein the pharmaceutical agent is selected from a non-steroidal antiandrogen selected from the group consisting of darolutamide, bicalutamide, enzalutamide, apalutamide, flutamide, nilutamide and topilutamide, a steroidal antiandrogen selected from the group consisting of Cyproterone acetate, Allylestrenol, Chlormadinone acetate, Delmadinone acetate, Gestonorone caproate, Hydroxyprogesterone caproate, Medroxyprogesterone acetate, Megestrol acetate, Osaterone acetate, Oxendolone and Spironolactone, an androgen synthesis inhibitor selected from the group consisting of ketoconazole, abiraterone, aminoglutethimide, goserelin and seviteronel and an antigonadotropin selected from the group consisting of Abarelix, Danazol, Gestrinone, Paroxypropione, Cetrorelix, Degarelix, Elagolix, Ganirelix, Linzagolix and Relugolix.

23. The combination according to any of claims 17 to 22, wherein the pharmaceutical agent is selected from the non-steroidal antiandrogen darolutamide, the steroidal antiandrogen Cyproterone acetate, the androgen synthesis inhibitor abiraterone or goserelin and the antigonadotropin Degarelix or Relugolix.

24. The combination according to any of claims 17 to 23, wherein the pharmaceutical agent is the non-steroidal antiandrogen darolutamide.

25. A tissue-targeting compound of formula (I) according to any of claims 1 to 6 or a combination according to any of claims 17 to 24 for use in the treatment of disease.

26. The tissue-targeting compound of formula (I) according to any of claims 1 to 6 or the combination according to any of claims 17 to 24 for use according to claim 25, wherein the disease is **characterized by** an overexpression of the prostate-specific membrane antigen (PSMA).

27. The tissue-targeting compound of formula (I) according to any of claims 1 to 6 or the combination according to any of claims 17 to 24 for use according to any of claims 25 or 26, wherein the disease is cancer, preferably prostate cancer.

28. The tissue-targeting compound of formula (I) according to any of claims 1 to 6 or the combination according to any of claims 17 to 24 for use in the treatment of disease, preferably wherein the disease is a hyperproliferative disease, more preferably wherein the hyperproliferative disease is cancer.

29. A composition containing a combination according to any of claims 17 to 24 together with pharmaceutically acceptable ingredients.

30. A kit comprising a combination of
a tissue-targeting compound of formula (I) according to any of claims 1 to 6 and a pharmaceutical agent;
and, optionally, one or more further pharmaceutical agents;
in which optionally both or either one of the tissue-targeting compound of formula (I) or the pharmaceutical agent are in the form of a pharmaceutical formulation which is ready for use to be administered simultaneously, concurrently, separately or sequentially.

## Patentansprüche

1. Eine gewebezielende Verbindung der Formel (I) 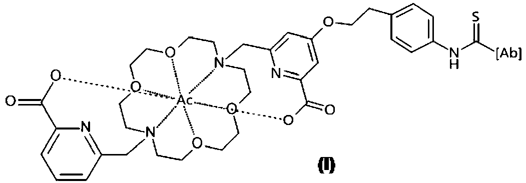 wobei [Ab] ein monoklonaler Antikörper oder ein antigenbindendes Fragment davon ist, das eine Bindungsaffinität für das prostataspezifische Membranantigen (PSMA) aufweist.

2. Die gewebezielende Verbindung der Formel (I) nach Anspruch 1, wobei [Ab] der anti-PSMAmonoklonale Antikörper Pelgifatamab oder ein antigenbindendes Fragment davon, der anti-PSMA-monoklonale Antikörper J591 oder ein antigenbindendes Fragment davon oder der anti-PSMA-monoklonale Antikörper TLX592 oder ein antigenbindendes Fragment davon ist.

3. Die gewebezielende Verbindung der Formel (I) nach Anspruch 1, wobei [Ab] der anti-PSMAmonoklonale Antikörper Pelgifatamab oder ein antigenbindendes Fragment davon ist.

4. Die gewebezielende Verbindung der Formel (I) nach einem der Ansprüche 1 bis 3, wobei [Ab] der anti-PSMA-Antikörper Pelgifatamab oder ein antigenbindendes Fragment davon ist, das mindestens die drei CDR-Schwerkettensequenzen gemäß SEQ ID NO. 5, SEQ ID NO. 6 und SEQ ID NO. 7 und die drei CDR-Leichtkettensequenzen gemäß SEQ ID NO. 8, SEQ ID NO. 9 und SEQ ID NO. 10 umfasst.

5. Die gewebezielende Verbindung der Formel (I) nach einem der Ansprüche 1 oder 2, wobei [Ab] der anti-PSMA-Antikörper J591 oder ein antigenbindendes Fragment davon ist, das mindestens die drei CDR-Schwerkettensequenzen gemäß SEQ ID NO. 13, SEQ ID NO. 14 und SEQ ID NO. 15 und die drei CDR-Leichtkettensequenzen gemäß SEQ ID NO. 16, SEQ ID NO. 17 und SEQ ID NO. 18 umfasst.

6. Die gewebezielende Verbindung der Formel (I) nach einem der Ansprüche 1 oder 2, wobei [Ab] der anti-PSMA-Antikörper TLX592 oder ein antigenbindendes Fragment davon ist, das mindestens die drei CDR-Schwerkettensequenzen gemäß SEQ ID NO. 21, SEQ ID NO. 22 und SEQ ID NO. 23 und die drei CDR-Leichtkettensequenzen gemäß SEQ ID NO. 24, SEQ ID NO. 25 und SEQ ID NO. 26 umfasst.

7. Verfahren zur Herstellung einer gewebezielenden Verbindung der Formel (I) 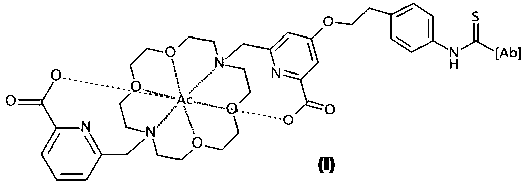
wobei [Ab] ein monoklonaler Antikörper oder ein antigenbindendes Fragment davon ist, das eine Bindungsaffinität für PSMA aufweist,
das Verfahren umfassend
(i) das Koppeln eines Chelat-Rests der Formel (II) 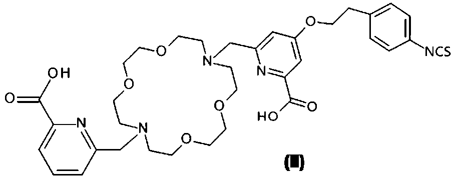 mit einem monoklonalen Antikörper oder einem antigenbindenden Fragment davon, das eine Bindungsaffinität für PSMA aufweist, und
(ii) das resultierende Produkt mit einer wässrigen Lösung in Kontakt bringen, die ein Ion von mindestens einem alpha-emittierenden Actinium-Isotop umfasst.

8. Das Verfahren nach Anspruch 7, wobei [Ab] der anti-PSMA-Antikörper Pelgifatamab oder ein antigenbindendes Fragment davon ist, das Verfahren umfassend
(i) das Koppeln eines Chelat-Rests der Formel (II) 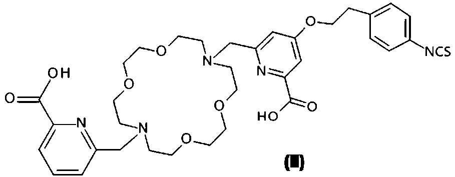 mit dem anti-PSMA-Antikörper Pelgifatamab oder einem antigenbindenden Fragment davon über einen Lysinrest des monoklonalen Antikörpers oder antigenbindenden Fragments davon, und
(ii) das resultierende Produkt mit einer wässrigen Lösung in Kontakt bringen, die ein Ion von mindestens einem alpha-emittierenden Actinium-Isotop umfasst.

9. Verfahren nach Anspruch 7, wobei [Ab] der anti-PSMA-Antikörper Pelgifatamab oder ein antigenbindendes Fragment davon ist,
das Verfahren umfassend
(i) das Koppeln eines Chelat-Rests der Formel (II) 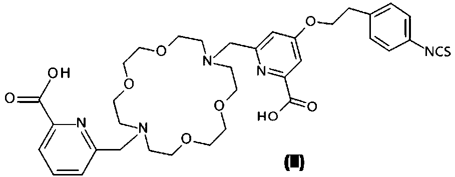 mit dem anti-PSMA-Antikörper Pelgifatamab oder einem antigenbindenden Fragment davon über einen Lysinrest des monoklonalen Antikörpers oder antigenbindenden Fragments davon, und
(ii) das resultierende Produkt mit einer wässrigen Lösung in Kontakt bringen, die ein Ion von mindestens einem alpha-emittierenden Actinium-Isotop umfasst,
wobei der anti-PSMA-Antikörper Pelgifatamab oder ein antigenbindendes Fragment davon mindestens die drei CDR-Schwerkettensequenzen gemäß SEQ ID NO. 5, SEQ ID NO. 6 und SEQ ID NO. 7 und die drei CDR-Leichtkettensequenzen gemäß SEQ ID NO. 8, SEQ ID NO. 9 und SEQ ID NO. 10 umfasst.

10. Ein gewebezielender Chelator der Formel (III) 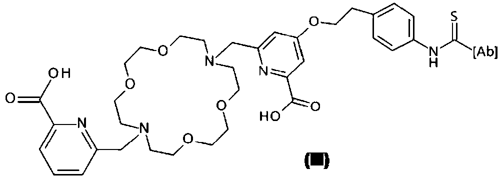 wobei [Ab] ein monoklonaler Antikörper oder ein antigenbindendes Fragment davon ist, das eine Bindungsaffinität für PSMA aufweist.

11. Der gewebezielende Chelator der Formel (III) nach Anspruch 10, wobei [Ab] der anti-PSMAmonoklonale Antikörper Pelgifatamab oder ein antigenbindendes Fragment davon, der anti-PSMA-monoklonale Antikörper J591 oder ein antigenbindendes Fragment davon oder der anti-PSMA-monoklonale Antikörper TLX592 oder ein antigenbindendes Fragment davon ist.

12. Der gewebezielende Chelator der Formel (III) nach Anspruch 10, wobei [Ab] der anti-PSMAmonoklonale Antikörper Pelgifatamab oder ein antigenbindendes Fragment davon ist.

13. Der gewebezielende Chelator der Formel (III) nach einem der Ansprüche 10 bis 12, wobei [Ab] der anti-PSMA-Antikörper Pelgifatamab oder ein antigenbindendes Fragment davon ist, das mindestens die drei CDR-Schwerkettensequenzen gemäß SEQ ID NO. 5, SEQ ID NO. 6 und SEQ ID NO. 7 und die drei CDR-Leichtkettensequenzen gemäß SEQ ID NO. 8, SEQ ID NO. 9 und SEQ ID NO. 10 umfasst.

14. Der gewebezielende Chelator der Formel (III) nach einem der Ansprüche 10 oder 11, wobei [Ab] der anti-PSMA-Antikörper J591 oder ein antigenbindendes Fragment davon ist, das mindestens die drei CDR-Schwerkettensequenzen gemäß SEQ ID NO. 13, SEQ ID NO. 14 und SEQ ID NO. 15 und die drei CDR-Leichtkettensequenzen gemäß SEQ ID NO. 16, SEQ ID NO. 17 und SEQ ID NO. 18 umfasst.

15. Der gewebezielende Chelator der Formel (III) nach einem der Ansprüche 10 oder 11, wobei [Ab] der anti-PSMA-Antikörper TLX592 oder ein antigenbindendes Fragment davon ist, das mindestens die drei CDR-Schwerkettensequenzen gemäß SEQ ID NO. 21, SEQ ID NO. 22 und SEQ ID NO. 23 und die drei CDR-Leichtkettensequenzen gemäß SEQ ID NO. 24, SEQ ID NO. 25 und SEQ ID NO. 26 umfasst.

16. Verwendung eines gewebezielenden Chelators der Formel (III) nach einem der Ansprüche 10 bis 15 zur Herstellung einer gewebezielenden Verbindung der Formel (I) nach einem der Ansprüche 1 bis 6.

17. Eine Kombination umfassend
(i) eine gewebezielende Verbindung der Formel (I) 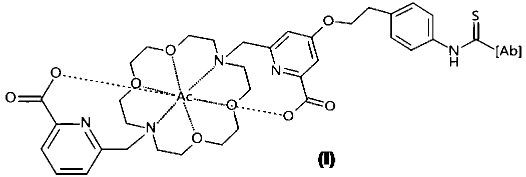 wobei [Ab] ein monoklonaler Antikörper oder ein antigenbindendes Fragment davon ist, das eine Bindungsaffinität für das prostataspezifische Membranantigen (PSMA) aufweist, und
(ii) ein pharmazeutischer Wirkstoff, ausgewählt aus einem nicht-steroidalen Antiandrogen, einem steroidalen Antiandrogen, einem Androgensynthese-Inhibitor, einem Antigonadotropin, einem PARP-Inhibitor, einem ATR-Inhibitor, einem ATM-Inhibitor, einem DNA-PK-Inhibitor, einem AKT-Inhibitor, einem Pi3K-Inhibitor, einem PSMAzielenden Beta-Emitter, einem Immun-Checkpoint-Inhibitor, einem Alpha-Emitter, einem Impfstoff und einem chemotherapeutischen Wirkstoff.

18. Die Kombination gemäß Anspruch 17, wobei [Ab] der anti-PSMA monoklonale Antikörper Pelgifatamab oder ein antigenbindendes Fragment davon ist, umfassend mindestens die drei CDR-Schwerkettensequenzen gemäß SEQ ID NO. 5, SEQ ID NO. 6 und SEQ ID NO. 7 und die drei CDR-Leichtkettensequenzen gemäß SEQ ID NO. 8, SEQ ID NO. 9 und SEQ ID NO. 10.

19. Die Kombination gemäß einem der Ansprüche 17 oder 18, wobei der pharmazeutische Wirkstoff aus einem nicht-steroidalen Antiandrogen, einem steroidalen Antiandrogen, einem Androgensynthesehemmer, einem Antigonadotropin, einem PARP-Inhibitor, einem PSMAzielgerichteten Beta-Emitter, einem Alpha-Emitter, einem Impfstoff und einem chemotherapeutischen Wirkstoff ausgewählt ist.

20. Die Kombination gemäß einem der Ansprüche 17 bis 19, wobei der pharmazeutische Wirkstoff aus einem nicht-steroidalen Antiandrogen, einem steroidalen Antiandrogen, einem Androgensynthesehemmer und einem Antigonadotropin ausgewählt ist.

21. Die Kombination gemäß einem der Ansprüche 17 bis 20, wobei der pharmazeutische Wirkstoff aus einem nicht-steroidalen Antiandrogen aus der Gruppe bestehend aus Darolutamid, Bicalutamid, Enzalutamid, Apalutamid, Flutamid, Nilutamid und Topilutamid, einem steroidalen Antiandrogen aus der Gruppe bestehend aus Cyproteronacetat, Allylestrenol, Chlormadinonacetat, Delmadinonacetat, Gestonoroncaproat, Hydroxyprogesteroncaproat, Medroxyprogesteronacetat, Megestrolacetat, Osateronacetat, Oxendolon und Spironolacton, einem Androgensynthesehemmer, aus der Gruppe bestehend aus Ketoconazol, Abirateron, Aminoglutethimid, Goserelin und Seviteronel, einem Antigonadotropin aus der Gruppe bestehend aus Abarelix, Danazol, Gestrinon, Paroxypropion, Cetrorelix, Degarelix, Elagolix, Ganirelix, Linzagolix und Relugolix und einem PARP-Inhibitor aus der Gruppe bestehend aus Olaparib, Rucaparib, Veliparib, Niraparib, Talazoparib, Pamiparib, CEP 9722, E7016 und Iniparib, ausgewählt is.

22. Die Kombination gemäß einem der Ansprüche 17 bis 21, wobei der pharmazeutische Wirkstoff aus einem nicht-steroidalen Antiandrogen aus der Gruppe bestehend aus Darolutamid, Bicalutamid, Enzalutamid, Apalutamid, Flutamid, Nilutamid und Topilutamid, einem steroidalen Antiandrogen aus der Gruppe bestehend aus Cyproteronacetat, Allylestrenol, Chlormadinonacetat, Delmadinonacetat, Gestonoroncaproat, Hydroxyprogesteroncaproat, Medroxyprogesteronacetat, Megestrolacetat, Osateronacetat, Oxendolon und Spironolacton, einem Androgensynthesehemmer aus der Gruppe bestehend aus Ketoconazol, Abirateron, Aminoglutethimid, Goserelin und Seviteronel und einem Antigonadotropin aus der Gruppe bestehend aus Abarelix, Danazol, Gestrinon, Paroxypropion, Cetrorelix, Degarelix, Elagolix, Ganirelix, Linzagolix und Relugolix ausgewählt ist.

23. Die Kombination gemäß einem der Ansprüche 17 bis 22, wobei der pharmazeutische Wirkstoffaus dem nicht-steroidalen Antiandrogen Darolutamid, dem steroidalen Antiandrogen Cyproteronacetat, dem Androgensynthesehemmer Abirateron oder Goserelin und dem Antigonadotropin Degarelix oder Relugolix ausgewählt ist.

24. Die Kombination gemäß einem der Ansprüche 17 bis 23, wobei der pharmazeutische Wirkstoff das nicht-steroidale Antiandrogen Darolutamid ist.

25. Eine gewebezielende Verbindung der Formel (I) gemäß einem der Ansprüche 1 bis 6 oder eine Kombination gemäß einem der Ansprüche 17 bis 24 zur Verwendung bei der Behandlung von Erkrankungen.

26. Die gewebezielende Verbindung der Formel (I) gemäß einem der Ansprüche 1 bis 6 oder die Kombination gemäß einem der Ansprüche 17 bis 24 zur Verwendung gemäß Anspruch 25, wobei die Erkrankung durch eine Überexpression des prostata-spezifischen Membranantigens (PSMA) gekennzeichnet ist.

27. Die gewebezielende Verbindung der Formel (I) gemäß einem der Ansprüche 1 bis 6 oder die Kombination gemäß einem der Ansprüche 17 bis 24 zur Verwendung gemäß einem der Ansprüche 25 oder 26, wobei die Erkrankung Krebs ist, vorzugsweise Prostatakrebs.

28. Die gewebezielende Verbindung der Formel (I) gemäß einem der Ansprüche 1 bis 6 oder die Kombination gemäß einem der Ansprüche 17 bis 24 zur Verwendung bei der Behandlung von Erkrankungen, vorzugsweise wobei die Erkrankung eine hyperproliferative Erkrankung ist, noch vorzugsweise wobei die hyperproliferative Erkrankung Krebs ist.

29. Eine Zusammensetzung, enthaltend eine Kombination gemäß einem der Ansprüche 17 bis 24 zusammen mit pharmazeutisch akzeptablen Zutaten.

30. Ein Kit umfassend eine Kombination von
einer gewebezielenden Verbindung der Formel (I) gemäß einem der Ansprüche 1 bis 6 und einem pharmazeutischen Wirkstoff;
und, optional, einem oder mehreren weiteren pharmazeutischen Wirkstoffen;
in dem optional sowohl oder entweder eine der gewebezielenden Verbindungen der Formel (I) oder der pharmazeutische Wirkstoff in Form einer pharmazeutischen Formulierung vorliegen, die bereit zur Anwendung ist, um gleichzeitig, getrennt oder nacheinander verabreicht zu werden.

## Revendications

1. Un composé ciblant les tissus de formule (I) 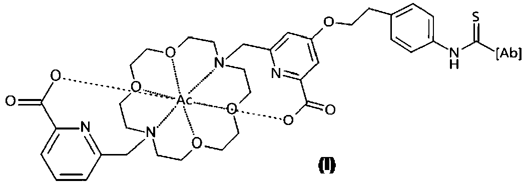 dans lequel [Ab] est un anticorps monoclonal ou un fragment de liaison à l'antigène ayant une affinité de liaison pour l'antigène de membrane spécifique de la prostate (PSMA).

2. Le composé ciblant les tissus de formule (I) selon la revendication 1, dans lequel [Ab] est l'anticorps monoclonal anti-PSMA Pelgifatamab ou un fragment de liaison à l'antigène, l'anticorps monoclonal anti-PSMA J591 ou un fragment de liaison à l'antigène ou l'anticorps monoclonal anti-PSMA TLX592 ou un fragment de liaison à l'antigène.

3. Le composé ciblant les tissus de formule (I) selon la revendication 1, dans lequel [Ab] est l'anticorps monoclonal anti-PSMA Pelgifatamab ou un fragment de liaison à l'antigène.

4. Le composé ciblant les tissus de formule (I) selon l'une quelconque des revendications 1 à 3, dans lequel [Ab] est l'anticorps anti-PSMA Pelgifatamab ou un fragment de liaison à l'antigène comprenant au moins les trois séquences de chaîne lourde CDR selon SEQ ID NO. 5, SEQ ID NO. 6 et SEQ ID NO. 7 et les trois séquences de chaîne légère CDR selon SEQ ID NO. 8, SEQ ID NO. 9 et SEQ ID NO. 10.

5. Le composé ciblant les tissus de formule (I) selon l'une quelconque des revendications 1 ou 2, dans lequel [Ab] est l'anticorps anti-PSMA J591 ou un fragment de liaison à l'antigène comprenant au moins les trois séquences de chaîne lourde CDR selon SEQ ID NO. 13, SEQ ID NO. 14 et SEQ ID NO. 15 et les trois séquences de chaîne légère CDR selon SEQ ID NO. 16, SEQ ID NO. 17 et SEQ ID NO. 18.

6. Le composé ciblant les tissus de formule (I) selon l'une quelconque des revendications 1 ou 2, dans lequel [Ab] est l'anticorps anti-PSMA TLX592 ou un fragment de liaison à l'antigène comprenant au moins les trois séquences de chaîne lourde CDR selon SEQ ID NO. 21, SEQ ID NO. 22 et SEQ ID NO. 23 et les trois séquences de chaîne légère CDR selon SEQ ID NO. 24, SEQ ID NO. 25 et SEQ ID NO. 26.

7. Un procédé pour la préparation d'un composé ciblant les tissus de formule (I) 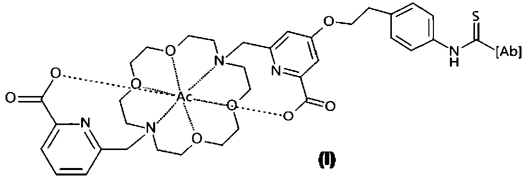
dans lequel [Ab] est un anticorps monoclonal ou un fragment de liaison à l'antigène ayant une affinité de liaison pour le PSMA,
le procédé comprenant
(i) le couplage d'un motif chélateur de formule (II) 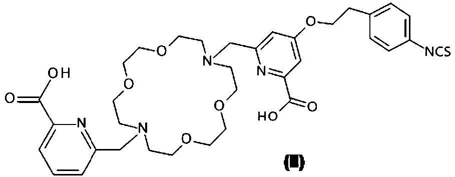 avec un anticorps monoclonal ou un fragment de liaison à l'antigène ayant une affinité de liaison pour le PSMA, et
(ii) le contact du produit résultant avec une solution aqueuse comprenant un ion d'au moins un isotope d'actinium émetteur alpha.

8. Le procédé selon la revendication 7, dans lequel [Ab] est l'anticorps anti-PSMA Pelgifatamab ou un fragment de liaison à l'antigène,
le procédé comprenant
(i) le couplage d'un motif chélateur de formule (II) 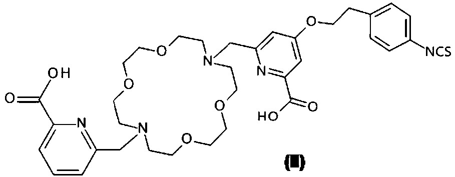 avec l'anticorps anti-PSMA Pelgifatamab ou un fragment de liaison à l'antigène via un résidu de lysine de l'anticorps monoclonal ou du fragment de liaison à l'antigène, et
(ii) le contact du produit résultant avec une solution aqueuse comprenant un ion d'au moins un isotope d'actinium émetteur alpha.

9. Le procédé selon la revendication 7, dans lequel [Ab] est l'anticorps anti-PSMA Pelgifatamab ou un fragment de liaison à l'antigène,
le procédé comprenant
(i) le couplage d'un motif chélateur de formule (II) 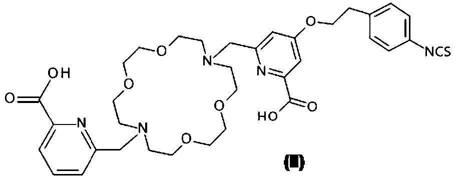 avec l'anticorps anti-PSMA Pelgifatamab ou un fragment de liaison à l'antigène via un résidu de lysine de l'anticorps monoclonal ou du fragment de liaison à l'antigène, et
(ii) le contact du produit résultant avec une solution aqueuse comprenant un ion d'au moins un isotope d'actinium émetteur alpha,
dans lequel l'anticorps anti-PSMA Pelgifatamab ou un fragment de liaison à l'antigène comprend au moins les trois séquences de chaîne lourde CDR selon SEQ ID NO. 5, SEQ ID NO. 6 et SEQ ID NO. 7 et les trois séquences de chaîne légère CDR selon SEQ ID NO. 8, SEQ ID NO. 9 et SEQ ID NO. 10.

10. Un chélateur ciblant les tissus de formule (III) 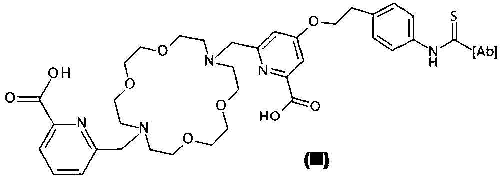 dans lequel [Ab] est un anticorps monoclonal ou un fragment de liaison à l'antigène ayant une affinité de liaison pour le PSMA.

11. Le chélateur ciblant les tissus de formule (III) selon la revendication 10, dans lequel [Ab] est l'anticorps monoclonal anti-PSMA Pelgifatamab ou un fragment de liaison à l'antigène, l'anticorps monoclonal anti-PSMA J591 ou un fragment de liaison à l'antigène ou l'anticorps monoclonal anti-PSMA TLX592 ou un fragment de liaison à l'antigène.

12. Le chélateur ciblant les tissus de formule (III) selon la revendication 10, dans lequel [Ab] est l'anticorps monoclonal anti-PSMA Pelgifatamab ou un fragment de liaison à l'antigène.

13. Le chélateur ciblant les tissus de formule (III) selon l'une quelconque des revendications 10 à 12, dans lequel [Ab] est l'anticorps anti-PSMA Pelgifatamab ou un fragment de liaison à l'antigène comprenant au moins les trois séquences de chaîne lourde CDR selon SEQ ID NO. 5, SEQ ID NO. 6 et SEQ ID NO. 7 et les trois séquences de chaîne légère CDR selon SEQ ID NO. 8, SEQ ID NO. 9 et SEQ ID NO. 10.

14. Le chélateur ciblant les tissus de formule (III) selon l'une quelconque des revendications 10 ou 11, dans lequel [Ab] est l'anticorps anti-PSMA J591 ou un fragment de liaison à l'antigène comprenant au moins les trois séquences de chaîne lourde CDR selon SEQ ID NO. 13, SEQ ID NO. 14 et SEQ ID NO. 15 et les trois séquences de chaîne légère CDR selon SEQ ID NO. 16, SEQ ID NO. 17 et SEQ ID NO. 18.

15. Le chélateur ciblant les tissus de formule (III) selon l'une quelconque des revendications 10 ou 11, dans lequel [Ab] est l'anticorps anti-PSMA TLX592 ou un fragment de liaison à l'antigène comprenant au moins les trois séquences de chaîne lourde CDR selon SEQ ID NO. 21, SEQ ID NO. 22 et SEQ ID NO. 23 et les trois séquences de chaîne légère CDR selon SEQ ID NO. 24, SEQ ID NO. 25 et SEQ ID NO. 26.

16. Utilisation d'un chélateur ciblant les tissus de formule (III) selon l'une quelconque des revendications 10 à 15 pour la préparation d'un composé ciblant les tissus de formule (I) selon l'une quelconque des revendications 1 à 6.

17. Une combinaison comprenant
(i) un composé ciblant les tissus de formule (I) 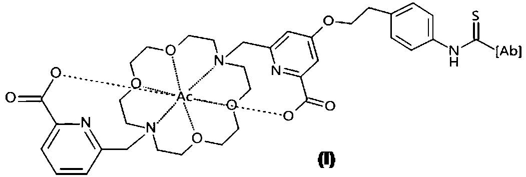 dans lequel [Ab] est un anticorps monoclonal ou un fragment de liaison à l'antigène ayant une affinité de liaison pour l'antigène de membrane spécifique de la prostate (PSMA), et
(ii) un agent pharmaceutique sélectionné parmi un antiandrogène non stéroïdien, un antiandrogène stéroïdien, un inhibiteur de synthèse des androgènes, un antigonadotrope, un inhibiteur de PARP, un inhibiteur d'ATR, un inhibiteur d'ATM, un inhibiteur de DNA-PK, un inhibiteur d'AKT, un inhibiteur de Pi3K, un émetteur bêta ciblant le PSMA, un inhibiteur de point de contrôle immunitaire, un émetteur alpha, un vaccin et un agent chimiothérapeutique.

18. La combinaison selon la revendication 17, dans lequel [Ab] est l'anticorps monoclonal anti-PSMA Pelgifatamab ou un fragment de liaison à l'antigène comprenant au moins les trois séquences de chaîne lourde CDR selon SEQ ID NO. 5, SEQ ID NO. 6 et SEQ ID NO. 7 et les trois séquences de chaîne légère CDR selon SEQ ID NO. 8, SEQ ID NO. 9 et SEQ ID NO. 10.

19. La combinaison selon l'une des revendications 17 ou 18, dans lequel l'agent pharmaceutique est sélectionné parmi un antiandrogène non stéroïdien, un antiandrogène stéroïdien, un inhibiteur de la synthèse des androgènes, un antigonadotrope, un inhibiteur de PARP, un émetteur bêta ciblant le PSMA, un émetteur alpha, un vaccin et un agent chimiothérapeutique.

20. La combinaison selon l'une des revendications 17 à 19, dans lequel l'agent pharmaceutique est sélectionné parmi un antiandrogène non stéroïdien, un antiandrogène stéroïdien, un inhibiteur de la synthèse des androgènes et un antigonadotrope.

21. La combinaison selon l'une des revendications 17 à 20, dans lequel l'agent pharmaceutique est sélectionné parmi un antiandrogène non stéroïdien sélectionné parmi le groupe constitué de darolutamide, bicalutamide, enzalutamide, apalutamide, flutamide, nilutamide et topilutamide, un antiandrogène stéroïdien sélectionné parmi le groupe constitué de l'acétate de cyprotérone, allylestrenol, acétate de chlormadinone, acétate de delmadinone, caproate de gestonorone, caproate d'hydroxyprogestérone, acétate de médroxyprogestérone, acétate de mégestrol, acétate d'osaterone, oxendolone et spironolactone, un inhibiteur de la synthèse des androgènes sélectionné parmi le groupe constitué de kétoconazole, abiratérone, aminoglutéthimide, goséréline et séviteronel, un antigonadotrope sélectionné parmi le groupe constitué de abarelix, danazol, gestrinone, paroxypropione, cétrorelix, degarelix, elagolix, ganirelix, linzagolix et relugolix et un inhibiteur de PARP sélectionné parmi le groupe constitué de olaparib, rucaparib, véliparib, niraparib, talazoparib, pamiparib, CEP 9722, E7016 et iniparib.

22. La combinaison selon l'une des revendications 17 à 21, dans l'agent pharmaceutique est sélectionné parmi un antiandrogène non stéroïdien sélectionné parmi le group constituant en darolutamide, bicalutamide, enzalutamide, apalutamide, flutamide, nilutamide et topilutamide, un antiandrogène stéroïdien sélectionné parmi le group constituant en acétate de cyprotérone, allylestrenol, acétate de chlormadinone, acétate de delmadinone, caproate de gestonorone, caproate d'hydroxyprogestérone, acétate de médroxyprogestérone, acétate de mégestrol, acétate d'osaterone, oxendolone et spironolactone, un inhibiteur de la synthèse des androgènes sélectionné parmi le group constituant en kétoconazole, abiratérone, aminoglutéthimide, goséréline et séviteronel et un antigonadotrophine sélectionné parmi le group constituant en abarelix, danazol, gestrinone, paroxypropione, cétrorelix, degarelix, elagolix, ganirelix, linzagolix et relugolix.

23. La combinaison selon l'une des revendications 17 à 22, dans l'agent pharmaceutique est sélectionné parmi l'antiandrogène non stéroïdien darolutamide, l'antiandrogène stéroïdien acétate de cyprotérone, l'inhibiteur de la synthèse des androgènes abiratérone ou goséréline et l'antigonadotrophine degarelix ou relugolix.

24. La combinaison selon l'une des revendications 17 à 23, dans l'agent pharmaceutique est l'antiandrogène non stéroïdien darolutamide.

25. Un composé ciblant les tissus de formule (I) selon l'une des revendications 1 à 6 ou une combinaison selon l'une des revendications 17 à 24 pour une utilisation dans le traitement des troubles.

26. Le composé ciblant les tissus de formule (I) selon l'une des revendications 1 à 6 ou la combinaison selon l'une des revendications 17 à 24 pour une utilisation selon la revendication 25, dans le trouble est **caractérisé par** une surexpression de l'antigène membranaire spécifique de la prostate (PSMA).

27. Le composé ciblant les tissus de formule (I) selon l'une des revendications 1 à 6 ou la combinaison selon l'une des revendications 17 à 24 pour une utilisation selon l'une des revendications 25 ou 26, dans le trouble est le cancer, de préférence le cancer de la prostate.

28. Le composé ciblant les tissus de formule (I) selon l'une des revendications 1 à 6 ou la combinaison selon l'une des revendications 17 à 24 pour une utilisation dans le traitement des troubles, de préférence dans le trouble est une maladie hyperproliférative, plus de préférence dans le trouble hyperprolifératif est le cancer.

29. Une composition contenant une combinaison selon l'une des revendications 17 à 24 avec des ingrédients pharmaceutiques acceptables.

30. Un kit comprenant une combinaison de
un composé ciblant les tissus de formule (I) selon l'une des revendications 1 à 6 et un agent pharmaceutique;
et, éventuellement, un ou plusieurs autres agents pharmaceutiques;
dans laquelle éventuellement les deux ou l'un des composés ciblant les tissus de formule (I) ou l'agent pharmaceutique sont sous la forme d'une formulation pharmaceutique prête à l'emploi pour être administrée simultanément, de manière concomitante, séparément ou séquentiellement.
